# EUROPEAN PATENT APPLICATION

(11) **EP 2 093 256 A2**
(43) Date of publication of application: **26.08.2009**
(21) Application number: 09004700.2
(22) Date of filing: 28.07.2006
(51) Int. Cl.: C08L 89/00, C08J 5/18, A61L 27/22, A61L 27/50

(54) **Biocompatible polymers and methods of use**

(30) Priority: 28.07.2005 US 703206 P
(62) Divisional of application: 06851119.5
(71) Applicant: Carnegie Mellon University, Pittsburgh, PA 15213 (US)
(72) Inventor: Campbell, Phil, Cranberry Township, PA 16066 (US); Fisher, Gregory W., Pittsburgh, PA 15212 (US); Kumta, Prashant, Pittsburgh, Pennsylvania 15215 (US); Sipe, David M., White Oak, PA 15131 (US); Smith, Jason, Pittsburgh, PA 15213 (US); Weiss, Lee E., Pittsburgh, PA 15217 (US)
(74) Representative: Polypatent

(57) **Abstract**

Compositions and methods for manufacturing polymers are disclosed. Compositions include novel plastics, including films and shaped forms comprising polymer matrices that are biologically compatible and biodegradable. Such plastics may comprise polymers derived from natural sources. Further, such plastics are useful in biological systems for wound repair, implants, stents, drug encapsulation and delivery, and other applications. The disclosed methods comprise mild manufacturing processes such that various additives, such as biologically active proteins, sugars, lipids, and the like may be incorporated into the polymer matrix without subsequent loss of bioactivity during processing. Additionally, methods of manufacture for controlling mechanical properties, such as elasticity, pliancy, and the porosity of such plastics are disclosed.

## Description

### Background

Fibrin elastomers were invented in the 1940's as part of a U.S. defense sponsored research program to develop medical strategies for wounded military personnel. Fibrin elastomers developed out of the human plasma program led by Edwin Cohn at Harvard University. John Ferry, then at Woods Hole, led the group that was involved in developing fibrin elastomers. As a result of this work, elastomeric sheet forms of fibrin were developed and used successfully in neurosurgical applications, burn treatments, and peripheral nerve regeneration, See, for example, Ferry, J.D. et al., Clin. Invest. 23:566-572 (1944); Bailey, O.T. et al., J. Clin. Invest.;23:597-600 (1944); Cronkite, et al., JAMA 124:976-8 (1944); Ferry J.D., et al., Am. Chem Soc. J. 69:400-409 (1947). Hard fibrin plastics were fabricated into implants for bone resurfacing, and were finding clinical success as early as the 1940's. See, for example, U.S. Patent Nos. 1,786,488; 2,385,802; 2,385,803; 2,492,458; 2,533,004; 2,576,006; 3,523,807; 4,548,736; and 6,074,663, which are all incorporated herein by reference. Research sponsored by the Hungarian government lead to the development of similar products in the 1950's through the early 1970's. One form of hard plastic fibrin (BERIPLAST^{™}) was demonstrated to have clinical efficacy in orthopedic applications of bone resurfacing. See, for example, Gerendas, M., Chap. 13 in Fibrinogen, Laki, K., Ed., Marcel Dekker, New York, pp. 277-316 (1968).

Despite the efficacy of fibrin products, concerns about disease transmission from purified human fibrinogen from plasma remained. However, during the late 70's and thereafter, fibrin was developed as a tissue glue and sealant, and although this application required purified human fibrinogen, new techniques had been developed to ensure the safety of these products. Consequently, fibrinogen has been used in clinical practice for over twenty years in Europe (and since 1998 in the US) with no report of disease transmission. Recently, the development of recombinant human fibrinogen and thrombin and purified salmon fibrinogen and thrombin have helped further address both concerns over safety and market availability. See, for example, Butler S.P. et al., Transgenic Res, 13:437-450 (2004); Prunkard D. et al., Nat. Biotechnol. 4:867-871 (1996); Butler S.P. et al., Thromb. Haemost. 78:537-542 (1997); U.S. Patent 5,527,692; U.S. Patent 5,502,034; U.S. Patent 5,476,777; U.S. Patent 6,037,457; U.S. Patent 6,083,902; and U.S. Patent 6,740,736.

Despite such advances in the field, interest in the use of protein biopolymers, such as fibrin elastomers, has significantly declined over time. Silicone rubber sheets, which were introduced in the 1960's and 1970's, have replaced fibrin elastomeric sheets in the clinic, despite inherent problems with silicone, such as biocompatibility and permanence. There are also drawbacks with current synthetic bioresorbable plastics, such as polyurethane, polylactic acid (PLA), polylactic-*co*-glycolic acid (PLGA), polyglycolic acid (PGA), and polycaprolactone. These polymers degrade in the body by hydrolysis, via bulk degradation, or through surface erosion, all of which operate independently of the surrounding biological environment. The inability of these polymers to degrade in response to cellular invasion and to promote the in-growth ofhost tissues remains a profound limitation of bioresorbable implants. In contrast, protein biopolymers degrade in response to cellular proteolytic processes so that degradation occurs in concert with the growth and healing of host tissues. Thus, development of polymeric, biologically compatible materials remains clinically relevant.

To date, the methods and compositions previously developed for biopolymers, including but not limited to, fibrin, elastin, etc., are not sufficiently adaptable for modem clinical use. For example, the original manufacturing methods developed for certain protein-based biopolymers required high temperature (e.g., 100°C -170°C) and pressure, or aggressive solvents. Such processing precludes the use of many drugs and proteins in the manufacturing process because of degradation, dilution, and denaturation as a result of the manufacturing process. In addition, even when high temperature or pressure is used, it is difficult to form complex shapes and control the physical characteristics, such as elasticity and porosity of the manufactured items using reported methods. To date, no one has solved the problem of manufacturing biopolymers while avoiding the disadvantages of known processing techniques, such as increased temperature and pressure and/or difficulty in retaining desirable physical characteristics of the plastics.

Thus, methods of incorporating heat-sensitive materials such as biological response modifiers and drugs into elastomeric and/or pliant materials are needed. In addition, compositions that have the ability to respond to the local cellular milieu are needed. Methods to create spatial patterns of such molecules in materials are also needed. Further, fabrication methods are needed that can be used to control properties of manufactured articles including for example the density, porosity, and mechanical properties, especially with regard to biopolymers. Finally, methods of manufacturing biocompatible materials with anisotropic properties are needed, especially with regard to extrusion or directed strain and/or printing technologies to impart such anisotropic properties.

### Brief Summary

The constructs disclosed herein constitute biocompatible materials which can be degraded in response to the host tissues' proteolytic processes. Processing of native extracellular matrix (ECM) molecules, such as fibrinogen, into biopolymers, such as structural elastomeric and/or pliant films, grafts, and scaffolds for tissue regeneration applications, are readily applicable to orthopedics, neurosurgery, and maxillofacial surgery, prosthetic tissue interface, as well as other clinical disciplines. Disclosed herein are methods of manufacture; including novel compositions comprising biopolymers. In certain embodiments, incorporation of biological response modifiers, antigens, drugs, hormones, tracers, labeled compounds, particulates (e.g., calcium phosphate, and bioglass) and other clinically relevant materials into the materials disclosed herein may be performed. In other embodiments, spatial patterns such of growth factors, hormones, and other constituents may be used to alter biomechanical properties and bioresorption rates.

Further embodiments include compositions and methods comprising processing of polymeric materials, as well as applications and use of such materials in biological systems, for example. Polymeric materials include those that are biocompatible, including, for example, polymeric sugars, such as polysaccharides (e.g., chitosan) and glycosaminoglycans, (e.g., hyaluronan, chondroitin sulphate, dermatan sulphate, keratan sulphate, heparan sulphate, and heparin) and polymeric proteins, such as fibrin, collagen, fibronectin, laminin, and gelatin. In some embodiments, the polymers may further comprise additional molecules of biological relevance that are placed on or in a polymeric matrix. Molecules of interest include, but are not limited to, biological response modifiers, antigens, drugs, hormones, tracers, labeled compounds, and others. In yet other embodiments, the polymeric materials are plastic and, in certain embodiments, capable of deformation. Such plastics may be hard or soft plastic, depending on intended use. These polymers may be shaped, machined, formed, molded, extruded, etc., into desirable shapes depending on the intended uses. The polymers may be used to form matrices for bio-compatible scaffolds capable of being implanted and resorbed. In certain embodiments the surfaces of a structure may be further processed in any manner including milling, maching roughening, porating, etc. to promote attachements and migration of cells for example. In other embodiments, cells may be seeded onto or into a scaffold, for example. Further, the porosity of such materials may be modified by any number of methods including introduction of a porogen which may be intercalated into the polymer matrix until removed by such means as solvation and sublimation, for example. The hydration of polymers, including, for example, hydrogels, may be adjusted in any manner, including, but not limited to, removal of water by evaporation, osmosis, or any other method. Such procedures may be performed for a time, temperature, and/or pressure suitable for the intended application. Thus, in some embodiments, low temperature manufacturing processes are presented.

Compositions and methods are provided relating to protein-based biopolymers and plastics. In certain embodiments, an article of manufacture comprising a biopolymer is provided where the article is dehydrated.

In certain embodiments, an article of manufacture is provided comprising dehydrated biopolymers such as a protein, a fibrin, a fibrinogen, a collagen, a gelatin, an elastin, an extracellular matrix constituent, a polysaccharide, a hylauronic acid, and combinations thereof.

In certain embodiments, an article of manufacture is provided comprising a biopolymer where the article is dehydrated by means of a vacuum; the article forms a film; the article is elastio; the article is pliant; the article comprises disulfide bonds; the article comprises isopeptidic bonds; the article comprises monoaldehyde or polyaldehyde cross-linked amines; the article comprises pyran cross-linked amines; or the article is cross-linked with a cross-linking agent such as an iridoid derivative, genipin, a diimidate, a dione, a carbodiimide, an acrylamide, N,N'methylenebiscrylamide, a sugar, ribose, Factor XIII, fructose, 1-ethyl-[3- (dimethylaminopropyl)] carbodiimide, 2,5-hexanedione, dimethylsuberimite, an aldehyde, glutaraldehyde, formaldehyde, NHS carboxylic acid ester, and combinations thereof.

In certain embodiments, an article of manufacture is provided comprising a dehydrated biopolymer where the article comprises a compound such as a biological response modifier, an antigen, a drug, a hormone, a tracer, RNA, DNA, and a labeled compound, where the tracer is a quantum dot and the biological response modifier is a bone morphogenic protein.

In certain embodiments, an article of manufacture is provided comprising a biopolymer film where a biological response modifier, an antigen, a drug, a hormone, a tracer, RNA, DNA, or a labeled compound is deposited on a surface of the film or a biological response modifier, an antigen, a drug, a hormone, a tracer, RNA, DNA, or a labeled compound is incorporated into the polymer matrix of the film.

In certain embodiments, an article of manufacture is provided comprising a biopolymer film further comprising a particulate such as hydroxyapatite, tricalcium phosphate, calcium phosphate, and calcium sulfate.

In certain embodiments, an article of manufacture is provided comprising a biopolymer film, where the film forms a laminated structure, and where the laminated structure is formed from a stack of sheets, a tubular roll, or combination thereof. In certain embodiments, structures disclosed herein are seeded with cells, such as stem cells.

In certain embodiments, an article of manufacture is provided comprising a biopolymer, where the article is compressed.

In certain embodiments, an article of manufacture is provided comprising a compressed biopolymer, where the biopolymer is, for example, a protein, a fibrin, a fibrinogen, a collagen, a gelatin, an elastin, an extracellular matrix constituent, a polysaccharide, a hylauronic acid, and combinations thereof.

In certain embodiments, an article of manufacture is provided comprising a compressed biopolymer, where the article is compressed at a pressure and a temperature and for a time sufficient to form a polymer matrix, and the article is formed in an extrusion die, a compression mold, or an injection mold.

In certain embodiments, an article of manufacture is provided comprising a compressed biopolymer, where the article comprises a filler or a plasticizer, where the plasticizer is, for example, a phthalate plasticizer, an adipate plasticizer, a trimelliate plasticizer, a maleate plasticizer, a sebacate plasticizer, a benzoate plasticizer, an epoxidized vegetable oil, a sulfonamide plasticizer, a phosphate plasticizer, water, a polyalcohol, a glycol, a glycerin, a glycerol, a polyether, an acetylated monoglyceride, an alkyl citrate, a polymeric plasticizer, and combinations thereof

In certain embodiments, an article of manufacture is provided comprising a compressed biopolymer, where the article is cross-linked with a cross-linking agent such as an iridoid derivative, genipin, a diimidate, a dione, a carbodiimide, an acrylamide, N,N'methylenebisacrylamide, a sugar, ribose, Factor XIII, fructose, 1-ethyl-[3-(dimethylaminopropyl)] carbodiimide, 2,5-hexanedione, dimethylsuberimite, an aldehyde, glutaraldehyde, formaldehyde, NHS carboxylic acid ester, and combinations thereof.

In certain embodiments, an article of manufacture is provided comprising a compressed biopolymer, where the article comprises pores.

In certain embodiments, an article of manufacture is provided comprising a compressed biopolymer, where the article comprises a compound such as a biological response modifier, an antigen, a drug, a hormone, a tracer, and a labeled compound.

In certain embodiments, a method is provided for manufacturing polymer films comprising providing a hydrogel; and vacuum drying the hydrogel at a temperature and a pressure, and for a time, to form a dehydrated film, where the temperatures is less than 80°C, the pressure is less than 20 millibars, and the hydrogel is formed from a polymer such as a protein, a fibrin, a fibrinogen, a collagen, a gelatin, an elastin, an extracellular matrix constituent, a polysaccharide, a hylauronic acid, and combinations thereof.

In certain embodiments, a method is provided for manufacturing polymer films comprising vacuum drying a hydrogel at a temperature and a pressure, and for a time, to form a dehydrated film, where the hydrogel comprises a plasticizer such as a phthalate plasticizer, an adipate plasticizer, a trimellitate plasticizer, a maleate plasticizer, a sebacate plasticizer, a benzoate plasticizer, an epoxidized vegetable oil, a sulfonamide plasticizer, a phosphate plasticizer, water, a polyalcohol, a glycol, a glycerin, a glycerol, a polyether, an acetylated monoglyceride, an alkyl citrate, a polymeric plasticizer, and combinations thereof.

In certain embodiments, a method is provided for manufacturing polymer films comprising vacuum drying a hydrogel at a temperature and a pressure, and for a time, to form a dehydrated film, where the hydrogel is crose-linked with a cross-linking agent such as an iridoid derivative, genipin, a diimidate, a dione, a carbodiimide, an acrylamide, N,N'methylenebisacrylamide, a sugar, ribose, Factor XIII, fructose, 1-ethyl-3-(dimethylaminopropyl) carbodiimide, 2,5-hexanedione, dimethylsuberimiate, an aldehyde, glutaraldehyde, formaldehyde, and combinations thereof, the hydrogel comprises a compound such as biological response modifiers, an antigen, a drug, a hormone, a tracer, RNA, DNA, a labeled compound, and combinations thereof, and/or the hydrogel comprises a filler.

In certain embodiments, a method is provided for manufacturing a plastic comprising admixing a biopolymer with a compound to create an admixture; and compressing the admixture at a pressure and a temperature to form a biopolymer matrix.

In certain embodiments, a method is provided fox manufacturing a plastic comprising admixing a biopolymer with a compound to create an admixture; and compressing the admixture at a pressure and a temperature to form a biopolymer matrix, where the temperature is less than 80°C, the pressure is less than 6000 pounds, and the admixture is formed in an extrusion die, a compression mold, or an injection mold.

In certain embodiments, a method is provided for manufacturing a plastic comprising admixing a biopolymer with a compound and compressing the admixture at a pressure and a temperature to form a biopolymer matrix, where the biopolymer matrix is formed from polymers such as a protein, a polysaccharide, a fibrin, a fibrinogen, a gelatin, a hylauronic acid, a collagen, an extracellular matrix constituent, an elastin, and combinations thereof; and where the biopolymer matrix comprises a plasticizer or a filler or combinations thereof, where the plasticizer is, for example, a phthalate plasticizer, an adipate plasticizer, a trimellitate plasticizer, a maleate plasticizer, a sebacate plasticizer, a benzoate plasticizer, an epoxidized vegetable oil, a sulfonamide plasticizer, a phosphate plasticizer, water, a polyalcohol, a glycol, a glycerin, a glycerol, a polyether, an acetylated monoglyceride, an alkyl citrate, a polymeric plasticizer, and combinations thereof.

In certain embodiments, a method is provided for manufacturing a plastic comprising admixing a biopolymer with a compound to create an admixture; and compressing the admixture at a pressure and a temperature to form a biopolymer matrix, where the biopolymer matrix is cross-linked with a cross-linking agent such as an iridoid derivative, genipin, a diimidate, a dione, a carbodiimide, an acrylamide, N,N'methylenebisacrylamido, a sugar, ribose, Factor XIII, fructose, 1-ethyl-3- (dimethylaminopropyl) carbodiimide, 2,5-hexanedione, dimethylsuberimiate, an aldehyde, glutaraldehyde, formaldehyde, and combinations thereof; and where the compound is, for example, a biological response modifier, an antigen, a drug, a hormone, a tracer, RNA, DNA, a labeled compound, and combinations thereof.

In certain embodiments, a method is provided for manufacturing a porous plastic comprising admixing a biopolymer with a porogen; forming a biopolymer matrix comprising the porogen; and removing the porogen from the biopolymer matrix, where the biopolymer matrix is formed from polymers such as a protein, a polysaccharide, a fibrin, a fibrinogen, a gelatin, a hylauronic acid, a collagen, an extracellular matrix constituent, an elastin, and combinations thereof; the biopolymer matrix comprises a filler; or the biopolymer matrix comprises a plasticizer, where the plasticizer is, for example, a phthalate plasticizer, an adipate plasticizer, a trimellitate plasticizer, a maleate plasticizer, a sebacate plasticizer, a benzoate plasticizer, an epoxidized vegetable oil, a sulfonamide plasticizer, a phosphate plasticizer, water, a polyalcohol, a glycol, a glycerin, a glycerol, a polyether, an acetylated monoglyceride, an alkyl citrate, a polymeric plasticizer, and combinations thereof.

In certain embodiments, a method is provided for manufacturing a porous plastic comprising admixing a biopolymer with a porogen; forming a biopolymer matrix comprising the porogen; and removing the porogen from the biopolymer matrix, where the biopolymer matrix is cross-linked with a cross-linking agent such as an iridoid derivative, genipin, a diimidate, a dione, a carbodiimide, an acrylamide, N,N'methylenebisacrylamide, a sugar, ribose, Factor XIII, fructose, 1-ethyl-3- (dimethylaminopropyl) carbodiimide, 2,5-hexanedione, dimethylsuberimiate, an aldehyde, glutaraldehyde, formaldehyde, and combinations thereof; and the biopolymer matrix comprises a compound such as a biological response modifier, an antigen, a drug, a hormone, a tracer, RNA, DNA, a labeled compound, and combinations thereof.

In certain embodiments, a method is provided for manufacturing a porous plastic comprising admixing a biopolymer with a porogen; forming a biopolymer matrix comprising the porogen; and removing the porogen from the biopolymer matrix, where the porogen is a solvation porogen; the poragen is soluble in an organic phase; the porogen is, for example, polyurethane, polylactic acid, polyglycolic acid, polylactic-*co*-glycolic acid, and polycaprolactone; the porogen is soluble in an aqueous phase; the porogen is sodium chloride; the porogen is a sublimation porogen; or the porogen is, for example, ammonium acetate, ammonium chloride, ammonium sulfate, ammonium bicarbonate, ammonium carbonate, and pyridmium trifluoroacetate.

In certain embodiments, a method is provided for cross-linking a polymer comprising providing a solid polymer powder admixed with a solid croas-linking agent capable of being activated by a solvent to form an admixture, forming a polymer matrix from the admixture comprising the solid cross-linking agent; and contacting the structure with a solvent which activates the cross-linking agent, where the cross-linking agent comprises a pyran moiety, the cross-linking agent is an iridoid derivative, or the cross-linking agent is genipin.

In certain embodiments, a method is provided for croas-linking a polymer comprising providing a solid polymer powder admixed with a solid cross-linking agent capable of being activated by a solvent to form an admixture; and forming a polymer matrix from the admixture comprising the solid cross-linking agent, where the polymer is a biopolymer such as a protein, a fibrin, a fibrinogen, a collagen, a gelatin, an elastin, an extracellular matrix constituent, a polysaccharide, a hylauronic acid, and combinations thereof, and where the admixture comprises a plasticizer such as a phthalate plasticizer, an adipate plasticizer, a trimellitate plasticizer, a maleate plasticizer, a sebacate plasticizer, a benzoate plasticizer, an epoxidized vegetable oil, a sulfonamide plasticizer, a phosphate plasticizer, polyalcohol, glycol, glycerin, glycerol, polyether, acetylated monoglycerides, alkyl citrates, and a polymeric plasticizer.

In certain embodiments, a method is provided for cross-linking a polymer comprising providing a solid polymer powder admixed with a solid cross-linking agent capable of being activated by a solvent to form an admixture; and forming a polymer matrix from the admixture comprising the solid cross-linking agent, where the admixture comprises a porogen, where the porogen is a solvation porogen or a sublimation porogen

In certain embodiments, a method is provided for cross-linking a polymer comprising providing a solid polymer powder admixed with a solid cross-linking agent capable of being activated by a solvent to form an admixture; and forming a polymer matrix from the admixture comprising the solid cross-linking agent, where the admixture further comprises a compound such as a biological response modifier, an antigen, a drug, a hormone, a tracer, RNA, DNA, a labeled compound, and combinations thereof.

In certain embodiments, a method is provided for cross-linking a polymer comprising providing a solid polymer powder admixed with a solid cross-linking agent capable of being activated by water, where the solid polymer contains free hydroxyl groups; and incubating the admixture for a sufficient time for cross-linking to occur.

### Brief Description of the Drawings

**Figure 1.** Figure 1A shows a thin planar structure that has been patterned with bioactive materials, in this case growth factors. Figure 1B shows the same planar structure after it has been rolled into a rod configuration with a gradient of growth factors that increases radially as one approaches the axis of the cylinder.
**Figure 2.** Figure 2 illustrates a tubular mold that can be used to cast tubular structures of materials made in accordance with the present disclosure.

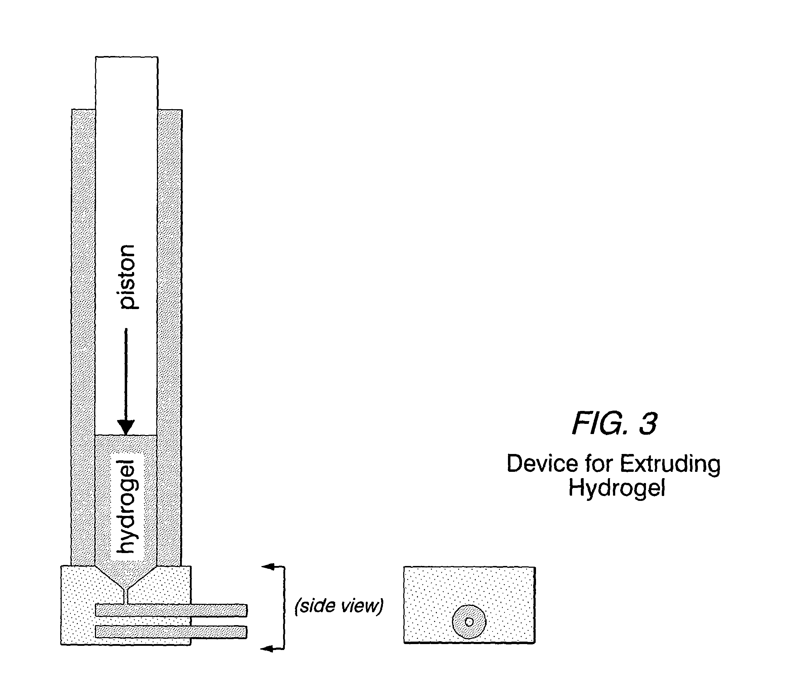

**Figure 3.** Figure 3 shows a hydrogel that has been inserted into or formed within an extruder and is subsequently extruded by forcing the piston downward so that the hydrogel exits the extruder through the die at the bottom.
**Figure 4**. Figure 4 shows a fibrin elastomer on which cy3-labeled bone morphogenetic protein-2 has been imprinted. Four concentrations are shown after seven days in cell culture conditions.
**Figure 5.** Figure 5 shows six SEM images, three of fibrin film surfaces (FIG. 5A-C) and three of fibrin film cross-sections (FIG. 5D-F). The film surfaces show the presence of fibrin fibrils (FIG. 5A-C), which are confirmed in the cross-section images (FIG. 5D). Cross-sections also show the high density of the fibrin (FIG. 5E-F).
**Figure 6.** Figure 6 shows a TEM image of a fibrin film. The lack of distinct patterns in the image demonstrates the random packing of fibrin fibrils within the film.

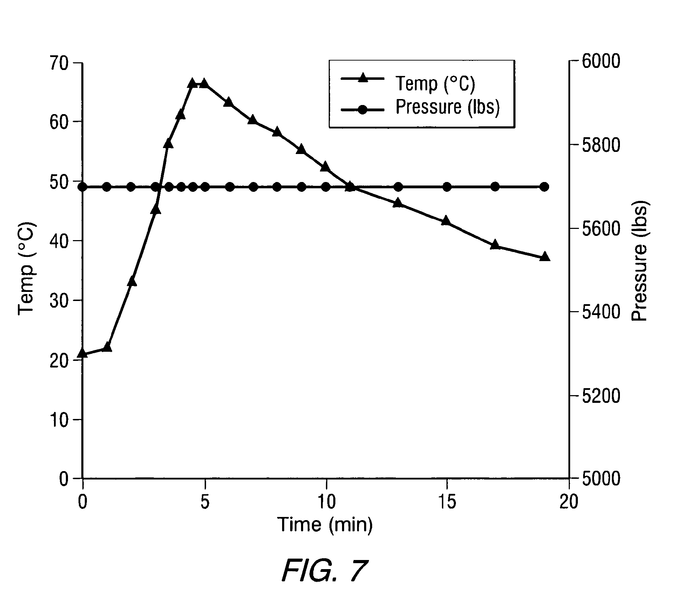

**Figure 7.** Figure 7 shows a time-pressure and a time-temperature profile for a 20 minute operation cycle for gelatin plastic pellet formation in a compaction press. The illustrated pressure and temperature data over time is representative of a 20 minute pellet compaction system.

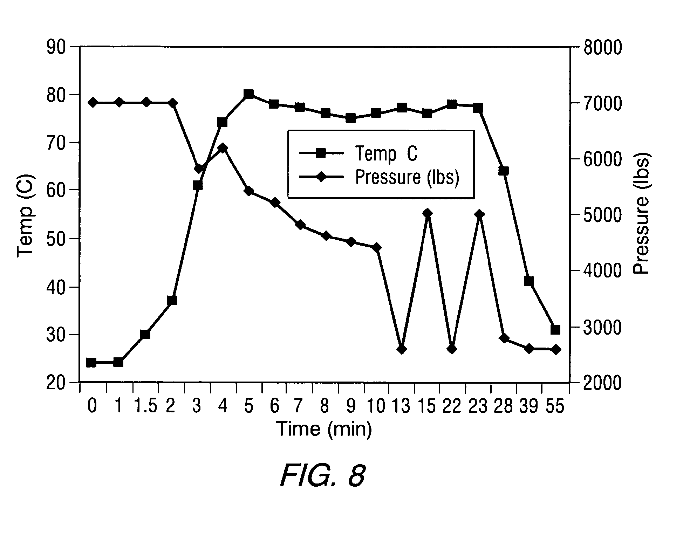

**Figure 8.** Figure 8 shows a time-pressure and a time-temperature profile for a 60 minute operation cycle for gelatin plastic pellet formation in a compaction press. The illustrated pressure and temperature data over time is representative of a 60 minute pellet compaction system.
**Figure 9**. Figure 9 shows an SEM image of the pores formed in the matrix of a gelatin plastic by chloroform leaching of polylactic acid porogen from the gelatin matrix.
**Figure 10**. Figure 10 shows SEM images of the machined surfaces of past-compressed gelatin plastics with glycerin plasticizer at 30X magnification (FIG. 10A) and 60X magnification (FIG. 10B).
**Figure 11.** Figure 11 shows SEM images of the machined surfaces of post-compressed gelatin plastics with no glycerin plasticizer at 33X magnification (FIG. 11A), 50X magnification (FIG. 11B), and 100X magnification (FIG. 11C).
**Figure 12.** Figure 12 shows plastics with incorporated quantum dots and borne morphogenotic protein (BMP-2) viewed under fluorescence. Samples labeled A are control groups containing no quantum dots. Samples labeled B and C contain quantum dots and BMP-2. Samples labeled D are cross-sectional sheets cut from the plastics labeled C. The samples labeled E are the same gelatin samples presented in D, only visualized without fluorescence.
**Figure 13.** Figure 13 shows representative fluorescence images of the vascularization on each of the three types of protein-based plastics with incorporated quantum dots and BMP-2 in a chick chotioallantoic membrane (CAM) assay.
**Figure 14.** Figure 14 is a graph presenting the time-dependent percentage mass remaining during vacuum exposure of gelatin plastics with ammonium salt porogens incorporated into the gelatin matrix and subject to sublimation under vacuum conditions.
**Figure 15**. Figure 15 is an SEM image of the porous matrix formed in a gelatin plastic that had ammonium acetate incorporated into the matrix and was removed by sublimation under vacuum. The resulting interconnected and extensive microporosity of the gelatin plastic post-sublimation is readily identifiable.
**Figure 16.** Figure 16 is a set of graphs presenting the results from *in vitro* degradation experiments on gelatin plastic samples in serum-containing media at 37°C. The degradation was quantised as mean percent area (FIG. 16A) and mean percent mass (FIG. 16B) remaining. Samples were either not cross-linked or cross-linked with 0.6 % gluteraldehyde (GA) or 0.6 % genipin (OP). Symbols represent the mean ± SD for triplicate determination.
**Figure 17**. Figure 17 is a graph presenting the results from *in vitro* degradation experiments on fibrin and urinary bladder ECM (UBBCM) plastic samples in serum containing media at 37°C shown as mean percent area of sample remaining. Samples were either not cross-linked or cross-linked with 0.6 % gluteraldehyde (GA) or 0.6 % genipin (GP). Symbols represent the mean ± SD of triplicate determinations.
**Figure 18.** Figure 18 is a graph presenting the results from *in vitro* degradation experiments on fibrin and urinary bladder ECM (UBECM) plastic samples in serum containing media at 37°C shown as mean percent mass of sample remaining. Samples were either not cross-linked or cross-linked with 0.6 % gluteraldehyde (GA) or 0.6 % genipin (GP). Symbols represent the mean ± SD of triplicate determinations.
**Figure 19**. Figure 19 is a set of graphs presenting the results from *in vitro* degradation experiments on gelatin plastic samples in serum containing media at 37°C, where the gelatin plastic was cross-linked with genipin (2% w/w) during plastic formation. The graph in FIG. 19A shows mean percent area of sample remaining and the graph in FIG. 19B shows mean percent mass of sample remaining. Symbols represent the mean ± SD of triplicate determinations.
**Figure 20.** Figure 20 is a set of graphs presenting the results from *in vitro* degradation experiments on fibrin plastic samples in serum containing media at 37°C, where the fibrin plastic was cross-linked with genipin (2% w/w) during plastic formation. The graph in FIG. 20A shows mean percent area of sample remaining and the graph in FIG. 20B shows mean percent mass of sample remaining. Symbols represent the mean ± SD of triplicate determinations.

### DETAILED DESCRIPTION

It is to be understood that certain descriptions of the present invention have been simplified to illustrate only those elements and limitations that are relevant to a clear understanding of the present invention, while eliminating, for purposes of clarity, other elements. Those of ordinary skill in the art, upon considering the present description of the invention, will recognize that other elements and/or limitations may be desirable in order to implement the present invention. However, because such other elements and/or limitations may be readily ascertained by one of ordinary skill upon considering the present description of the invention, and are not necessary for a complete understanding of the present invention, a discussion of such elements and limitations is not provided herein. As such, it is to be understood that the description set forth herein is merely exemplary to the present invention and is not intended to limit the scope of the claims.

Other than in the examples herein, or unless otherwise expressly specified, all of the numerical ranges, amounts, values, and percentages, such as those for amounts of materials, elemental contents, times and temperatures of reaction, ratios of amounts, and others, in the following portion of the specification and attached claims may be read as if prefaced by the word "about," even though the term "about" may not expressly appear with the value, amount, or range. Accordingly, unless indicated to the contrary, the numerical parameters set forth in the following specification and claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical value, however, inherently contains error necessarily resulting from the deviation found in its underlying respective testing measurements. Furthermore, when numerical ranges are set forth herein, these ranges are inclusive of the recited range end points (i.e., end points may be used). Also, it should be understood that any numerical range recited herein is intended to include all sub-ranges subsumed therein. For example, a range of "1 to 10" is intended to include all sub-range between (and including) the recited minimum value of 1 and the recited maximum value of 10, that is, having a minimum value equal to or greater than 1 and a maximum value of equal to or less than 10.

Certain compositions within the present invention are generally described in the form of biopolymers for use in medical and biological systems. It will be understood, however, that the present invention may be embodied in forms and applied to end uses that are not specifically and expressly described herein. For example, one skilled in the art will appreciate that compositions and methods comprising plastics have application in many industries, as well as the medical arts.

All patents, publications, or other disclosure material referenced herein are incorporated by reference in their entirety. Any patient, publication, or other disclosure materials, in whole or in part, that is incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

The articles "a," "an," and "the" are used herein to refer to one or more than one (i.e., to at least one) of the grammatical objects of the article. By way of example, "a component" means one or more components, and thus, possibly, more than one component is contemplated and may be employed or used.

As disclosed herein, new polymers and methods of manufacture of the same with improved structural properties exhibiting a range of biomechanical properties are presented. The advantages of the compositions and methods of the invention include, but are not limited to, biocompatibility of the materials with the host; the ability of the material to degrade in register to tissue regeneration; the binding of growth factors to the materials disclosed herein, which thereby helps minimize the dosages needed to produce therapeutic results; the ability to easily engineer the mechanical properties (e.g., ranging from elastic to rubbery to hard) of the materials; the ability to easily store the materials for off-the-shelf usage; the ability to easily shape the materials at a time and a place that where the materials will be used (e.g., the operating room, the battlefield) etc.; the ability of the structure to resisting tissue prolapse at the implantation site; and, the ability to modulate the physiological response to the implanted materials by incorporating other materials into the base material. The compositions and methods of the invention include, but are not limited to, the use of proteins to create biopolymers, In fact, other naturally occurring materials, such as the polysaccharides, such as chitosan or glycosaminoglycans, such as hyaluronic acids, as well as extracellular matrix constituents, such as fibrous proteins obtained by processes such as those disclosed in U.S. Patent Numbers 6,653,291; 6,485,723; 6,379,710; 6,375,989; 6,331,319; 6,241,981; 6,187,039; 6,099,567; 5,997,575; 5,955,110; 5,885,619; 5,755,791; 5,753,267; 5,711,969; 5,695,998; 5,645,860; 5,573,784; 5,554,389; 5,445,833; 5,372,821; 5,352,463; 5,281,422; 4,956,178; and 4,902,508, all of which are incorporated herein by reference, can also be used individually or in combination to create elastomeric and/or pliant materials, such as those disclosed here.

The term "polymer," as used herein, refers to natural and synthetic molecules with repeating structural units including, but not limited to, molecules comprising gels and plastics. The term "matrix" refers to a network of linked subunits. The term "polymer matrix" refers to a network of linked polymer subunits and, thus, comprises the interior space as opposed to the surface of a polymer, or structure formed therefrom.

The term "biocompatible" refers to the absence of stimulation of a severe, long-lived or escalating biological response to an implant or coating, and is distinguished from a mild, transient inflammation which typically accompanies surgery or implantation of foreign objects into a living organism.

Examples of biocompatible, non-biodegradable polymers include, but are not limited to, polyethylenes, polyvinyl chlorides, polyamides, such as nylons, polyesters, rayons, polypropylenes, polyacrylonitriles, acrylics, polyisoprenes, polybutadienes and polybutadiene-polyisoprene copolymers, neoprenes and nitrile rubbers, polyisobutylenes, olefinic rubbers, such as ethylene-propylene rubbers, ethylene-propylene-diene monomer rubbers, and polyurethane elastomers, silicone rubbers, fluoroelastomers and fluorosilicone rubbers, homopolymers and copolymers of vinyl acetates, such as ethylene vinyl acetate copolymer, homopolymers and copolymers of acrylates, such as polymethylmethacrylate, polyethylmethacrylate, polymethacrylate, ethylene glycol dimethacrylate, ethylene dimethacrylate and hydroxymethyl methacrylate, polyvinylpyrrolidones, polyacrylonitrile butadienes, polycarbonates, polyamides, fluoropolymers, such as polytetrafluoroethylene and polyvinyl fluoride, polystyrenes, homopolymers and copolymers of styrene acrylonitrile, cellulose aectates, homopolymers and copolymers of acrylonitrile butadiene styrene, polymethylpentenes, polysulfones, polyesters, polyimides, polyisobutylenes, polymethylstyrenes, and other similar compounds known to those skilled in the art. Other biocompatible non-degradable polymers that arc useful in accordance with the present disclosure include polymers comprising biocompatible metal ions or ionic coatings which can interact with DNA. In exemplary embodiments, gold and silver ions may be used, for example, for inhibiting inflammation, binding DNA, and inhibiting infection and thrombosis.

Examples of biocompatible, biodegradable polymers include, but are not limited to, polylactic acid (PLA), polyglycolic acid (PGA), polylactic-*co*-glycolytic acid (PLGA), polycaprolactone, and copolymers thereof, polyesters, such as polyglycolides, polyanhydrides, polyacrylates, polyalkyl cyanoacrylates, such as n-butyl cyanoacrylate and isopropyl cyanoacrylate, polyacrylamides, polyorthoesters, polyphosphazenes, polypeptides, polyurethanes, polystyrenes, polystyrene sulfonic acid, polystyrene carboxylic add, polyalkylene oxides, alginates, agaroses, dextrins, dextrans, and polyanhydrides.

The term "biopolymer" refers to a biocompatible polymers comprising polymers that can be found naturally in organisms, as well as chemical and physical modifications of such polymers, and include, but are not limited to, proteins, fibrins, fibrinogen, collagens gelatins, elastins, lamnin, fibronectin, extracellular matrix constituents, glycosaminoglycans, hylauronic acid, albumin, alginates, chitosans, cellulose, thrombin, heparin, polysaccharides, synthetic polyamino acids, prolamines, combinations thereof, and other such molecules. Biocompatible polymers include, but not limited to, biopolymers that can be, but are not necessarily, biodegradable. As used herein, the term "native" when describing a substance, such as a polymer, refers to a purified form of the substance which is chemically identical to the substance as found in nature, such as, for example, fibrin, elastin, etc.

The term "admixture" refers to a mixture of a base material and one or more additional materials. In exemplary embodiments, the base material of the admixture is a polymer, such as, for example, a biopolymer, and the additional material is a filler, a particulate, a porogen, a biological response modifier, an antigen, a drug, a hormone, a tracer, RNA, DNA, a labeled compound, combinations thereof, and similar materials. IN some embodimented the admixture may be a slurry. The term "slurry" refers to an an admixture suspended in a liquid, which includes, but is not limited to plasticizers such as those disclosed herein and known in the art, and other such agents.

The terms "biodegradable" and "bioerodible" refer to the dissolution of a substance, such as implant or coating, into constituent parts that may be metabolized or excreted, under the conditions normally present in a living tissue. In exemplary embodiments, the rate and/or extent of biodegradation or bioerosion may be controlled in a predictable manner.

The term "biological response modifier" refers to any protein, glycoprotein, sugar, polysaccharide, lipid, DNA, RNA, aptamer, peptide, hormone, vitamin and other such substance, which when introduced into a host organism is capable of eliciting a biological response, and includes, but is not limited to, cytokines, growth factors, protein hormones, genes, or genetically modified organisms, such as viruses and bacteria, and the like. Specific examples of biological response modifiers include, but are not limited to, the interleukins (IL), such as IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, isoforms thereof and others; the interferons such as interferon alpha, beta, gamma and other; the growth factors, such as platelet derived growth factor (PDGF), acidic and basic fibroblast growth factor including FGF-1 and FGF-2, transformation growth factor beta (TGF -beta, e.g. TGF-beta-1 and TGF-beta-2), insulin like growth factor (IGF, e.g., including IGF-I and IGF-II), epidermal growth factor (EGF, e.g., EGF and heparin binding EGF), tumor necrosis factor-alpha (TNF-α), tumor necrosis factor- beta (TNF-β), vascular endothelial growth factor (VEGF), isoforms thereof and others; antibodies; bone morphogenetic proteins(BMPs), including but not limited to BMP-2, BMP-4, and BMP-7, metalloproteases or prometalloproteases and inhibitors thereof, angiotensin converting enzyme inhibitors; plasminogen and tissue plasminogen activator (TPA), including anisoylated plasminogen activator (TPA) and anisoylated plasminogen-streptokinase activator complex (APSAC) and inhibitors therof; RNA and DNA in its various forms to modify gene expression and function; growth factors; cytokins, or other protein-based hormone, steroid-based hormones, engineered hormones, or combinations thereof, and the like.

The term "antigen" refers to any molecule capable of eliciting an immunological response, including, but not limited to, immunological memory responses, T-cell responses, B cell response, allergy, a vaccine response, inflammation, immunological tolerance, and the like. The term "antigenic compound" refers to any organism or substance comprising an antigen, and includes, but is not limited to, whole viruses, bacteria, tissue, and derivatives, modifications, and products thereof, capable of eliciting an immune response. Specific examples of antigens include, but are not limited to, protein antigens, polysaccharide antigens, haptens, tumor antigens, blood antigens, and the like.

The term "drug" refers to a substance used as a medication or in the preparation of medication, including, but not limited to, a substance intended for use in the diagnosis, cure, mitigation, treatment, or prevention of disease. For example, a drug may include, but is not limited to, small organic molecules, complex organic molecules, inorganic elements and molecules, and the like. As used herein, the term "drug" encompasses for examples, fungicides, antibiotics and other molecules.

The term "tracer" refers to any molecule that is introduced into an organism or construct and capable of being detected. For example, tracers include, but are not limited to, radioactive compounds, contrast agents, light-emitting molecules, quantum dots, fluorescent molecules, dyes, biomarkers, molecular tracers for imaging purposes (including fluorescence markers, radioactive markers, contrast agents for CT, microCT, MRI or forms of bio-imaging, and immunospecific markers), and others. As used herein, a "labeled compound" refers to any substance modified such that it (or its metabolites, such as degradation products) is detectable by any means. A labeled compound may be labeled in any manner including attachment (e.g., covalent or non-covalent) of tracers to the molecule of interest.

The term "hormone" refers to any molecule which acts as a biochemical messenger that regulates physiological events in living organisms. Specific examples of hormones, include, but are not limited to, steroid hormones, such as estrogen, pregnenolone, aldosterone, estradiol, cortisol, testosterone, progesterone, etc.; peptide hormones, such as luteinizing hormone (LH), adrenocorticotropic hormone (ACTH), follicle stimulating hormone (FSH), and angiotensin II/III; synthetic steroids including, but not limited to, glucocorticoids, such as prednisone, dexamethasone, triamcinolone, etc., mineralocorticoids, such as fludrocortisone, Vitamin D derivatives, such as dihydrotachysterol, synthetic androgens, such as oxandrolone, decadurabolin, etc., synthetic estrogens such as diethylstilbestrol (DES); synthetic progestins, such as norethindrone and medroxyprogesterone acetate; and others. It is intended herein that the term "hormone" is encompassed by the term "biological response modifier."

The term "filler" refers to any substance incorporated into the polymer in order to provide additional structural or mechanical properties to the compositions disclosed herein, and include, but are not limited to, particulates as disclosed herein including, but not limited to, calcium phosphate, hydroxyapatite, etc., excipients (e.g., inert compounds acting as bulking agents, such as carboxymethylcellulose), synthetic and/or naturally occurring substances, such as polysacharrides and proteins (e.g., fibrous or globular proteins), which can be, for example, inert or biologically active.

As used herein, the term "heat-sensitive" refers to any compound which when heated beyond 80°C, becomes inactive. Thus, the term "heat-sensitive" compound encompasses any compound, such as a biological response modifiers, antigens, drugs, hormones, tracers, labeled compounds, which lose biological activity at a temperature greater than 80°C, by any means including melting, decomposition, denaturation, etc.

The term "bio-ink" is intended to include any material, whether liquid, solid or semisolid, that is suitable for deposition as part of the construction of a scaffold and may comprise, for example, a biological response modifier, antigen, drug, hormone, tracer, RNA, DNA, labeled compound, combinations thereof, and other such substances. Any material that is biocompatible or biodegradable is suitable for use as a bio-ink in accordance with the present disclosure.

The term "scaffold" includes essentially any assembly of materials that is designed to imitate a biological structure, such as, for example, by imitating an aspect of fine structure (e.g., pore size and/or abundance) or by imitating the ability to support adhesion and/or growth of at least one appropriate cell type. The term "biomimetic extracellular matrix" (bECM) refers to a structure, such as a scaffold, comprised of extracellular matrix constituents.

The term "co-depositing" describes the placement of two or more substances, usually bio-inks, at the same position in, for example, a scaffold. Substances may be co-dopoaited simultaneously or non-simultaneously (for example, sequentially).

A "concentration gradient" is one or more dimensions (whether in space or time) along which the concentration and/or accessibility of one or more substances may vary. The term is intended to include gradients in which the concentration is uniform throughout (i.e., a flat line gradient) as well as gradients in which the concentration varies. Concentration gradients include both linear gradients (i.e., gradients which increase or decrease at a continuous rate) and non-linear gradients. A "spatial concentration gradient" is a concentration gradient in which the concentration may vary along one or more spatial dimensions. A "temporal concentration gradient" is a concentration gradient in which the concentration may vary over time. A "3-D concentration gradient" is a set of three orthogonal spatial dimensions in which the concentration of one or more substances may vary independently along each dimension.

"Cross-linking" is the formation of a covalent attachment between two entities, typically polymer subunits. A "cross-linking agent" refers to any agent capable of cross-linking two entities. Cross-linking agents may be physical or chemical. Chemical cross-linking agents include, but are not limited to, iridoid derivatives (such as, for example, genipin), diimidates, diones (e.g., 2,5-hexanedione), carbodiimides, (e.g., 1-ethyl-[3-(dimethylaminopropyl)] carbodiimide) (abbr., EDC), acrylamides (e.g., N,N'methylenebisacrylamide), sugars (e.g., ribose and fructose), proteins (e.g., enzymes, such as transglutaminase Factor XIII), dimethylsuberimidates, aldehydes (e.g., glutaraldehyde, and formaldehyde, formaldehyde sodium bisulfite), dihomo bifuntional NHS esters (e.g., di NHS-esters of dicarboxylic acid comprising 1-20 intervening carbons), carbonyldiimide; glyoxyls; and similar cross-linking agents. Chemical cross-linking agents can be solids (e.g., powders) or liquids. Examples of solid cross-linking agents include, but are not limited to, genipin, dihomo bifuntional NHS esters, and foraldehyde sodium bisulfite. Examples of liquid crosslinking agents include, but are not limited to, formaldehye, glutaraldehyde, etc. Physical cross-linking agents, include, for example, electromagnetic radiation, such as ultraviolet light, heat, microwaves, etc. As used herein "cross-linked amine" refers to any bridging bond between two polymers comprising nitrogen, such as the product of aldehyde cross-linking (i.e., an imine or an eneamine), or product of an ester cross-link, or an amide, or any other similar bond. Cross-linking may occur before or after formation of a structure.

The term "gelation" refers to the phase transition that a polymer undergoes when it increases in viscosity and transforms from a fluid state into a semi-solid material, or gel. At this transition point, the molecular weight (weight average) of the polymer matrix becomes "infinite" due to the formation of an essentially continuous matrix throughout the nascent gel. Polymerization can continue beyond the point of gelation through the incorporation of additional polymer units into the gel matrix. As used herein, "gel" may include both the semi-solid gel state and the high viscosity state that exists above the gelation temperature.

The term "gelation temperature" refers to the temperature at which a polymer undergoes reverse thermal gelation, i.e., the temperature below which the polymer is soluble in water and above which the polymer undergoes phase transition to increase in viscosity or to form a semi-solid gel. Because gelation does not involve any change in the chemical composition of the polymer, the gel may spontaneously reverse to the lower viscosity fluid form when cooled below the gelation temperature. The gelation temperature may also be referred to as the gel-solution (or gel-sol) transition temperature.

A "hydrogel" is defined as a substance formed when a polymer (natural or synthetic) becomes a 3-D open-lattice structure that entraps solution molecules, typically water, to form a gel. A polymer may form a hydrogel by, for example, aggregation, coagulation, hydrophobic interactions, cross-linking, salt bridges, etc. Where a hydrogel is to be used as part of a scaffold onto which cells will be seeded, the hydrogel should be non-toxic to the cells. The term "dehydrated" whether referring to a structure, such as a film, or a hydrogel includes any substance that has had water removed from it by any processes, and, thus, includes partially hydrated hydrogels, such as those described herein.

A "hydrogel solution" is a solute and a solvent comprising a substance that if subjected to the appropriate conditions, such as temperature, salt concentration, pH, the presence of a protease, the presence of a binding partner, etc., becomes a hydrogel or part of a hydrogel. The term "solution" in a hydrogel solution is intended to include true solutions, as well as suspensions, such as colloidal suspensions, and other fluid materials where one component is not truly solubilized

A "mechanical property" refers to essentially any property that provides some description for how a substance responds to the application of an external force. Exemplary mechanical properties include tensile strength, compressional strength, flexural strength, impact strength, elongation, modulus, toughness, having mechanical properties similar to rubber (e.g., rubbery); etc. Mechanical properties include, for example, pliability (i.e., "pliant" is the ability of a polymer to bend or deform without breaking), elasticity (i.e., "elastomeric" is the ability of a polymer to a recover the original shape after deformation) and other such properties. A film can be, for example, both elastic and pliant, elastic without being pliant, or pliant without being elastic. Where a film is neither elastic nor pliant, it is referred to herein as "rigid."

A "film" refers to a thin sheet. Thus, a film can be a sheet up to 1 µm thickness, up to 100 µm thickness, up to 10 µm thickness, up to 1 µm thickness, up to 100 µm thickness, up to 10 nm thickness, up to 1 nm thickness, or any range therebetween. A film will have many mechanical properties, such as, for example, elasticity, non-elasticity, pliancy, rigidity, etc., depending on the formulation and shape.

A "particulate" refers to a solid of sufficiently small size that it can be incorporated into a polymer matrix. Particulates include, but are not limited, to crystals, polymers, powders, ceramics, minerals, metal salts, calcium phosphates, including apatites (e.g., hydroxyapatite) and tricalcium phosphate, calcium sulphate, calcium phosphate, as well as other mineral combinations selected for inclusion to promote osteoconductivity for orthopaedic and other related applications, glasses, bioglasses, porogens, and the like.

The term "porogen" refers to any particulate incorporated into a polymer matrix, wherein the particulate may be removed by any means including dissolution or sublimation of the porogen into a liquid or gas phase. A porogen may be soluble in the aqueous phase, the organic phase, or capable of sublimation into a gas. A porogen may also comprise an encapsulated gas (i.e., CO₂, N, O, etc.) or substance capable of releasing a gas, upon decomposition, such as, for example, sodium bicarbonate releasing CO₂ upon contact with an acid.

The term "powder" or "powdered" refers to small solid particles. Powders, as used herein comprise particles having an average diameter of less than 30 mesh (i.e., 595 microns). In some embodiments of the invention, the average diameter of the particles is less than 35 mesh (i.e., 500 microns), less than 40 mesh (i.e., 400 microns), less than 45 mesh (i.e., 354 microns), less than 50 mesh (i.e., 297 microns), less than 60 mesh (i.e., 250 microns), less than 70 mesh (i.e., 210 microns), less than 80 mesh (i.e., 177 microns), less than 100 mesh (i.e., 149 microns), less than 120 mesh (i.e., 125 microns), less than 140 mesh (i.e., 105 microns), less than 170 mesh (i.e., 88 microns), less than 200 mesh (i.e., 74 microns), less than 230 mesh (i.e., 62 microns), less than 270 mesh (i.e., 53 microns), less than 325 mesh (i.e., 44 microns), less than 400 mesh (i.e., 37 microns), or less. A range of diameters for the particles described herein find use in the invention (e.g., 100-300 microns as used in some embodiments). For example, in one embodiment, the particles have a size range from between 10 and 800 microns. In another embodiment, the particles have a size range from between 30 and 400 microns. In yet other embodiments, the size range of the particles may be between 40 and 390 microns, between 50 and 380 microns, between 60 and 370 microns, between 70 and 360 microns, between 80 and 350 microns, between 90 and 340 microns, between 100 and 330 microns, between 110 and 320 microns, between 120 and 310 microns, between 130 and 300 microns, between 140 and 290 microns, between 150 and 280 microns, between 160 and 270 microns, between 170 and 260 microns, between 180 and 250 microns, between 190 and 240 microns, between 200 and 230 microns, etc.

A powder can be formed by any means know in the art or disclosed herein including milling, grinding, spray-drying, etc.

The term "plastic" refers to any substance, such as organic, synthetic, and/or processed materials that comprise polymer and can be made into structures such as 3-dimensional constructs and 2-dimensional constructs, such as, for example, films, sheets, laminates, filaments, and similar structures. See, for example, U.S. Patent No. 6,143,293. As used herein the term "hard plastic" refers to a plastic that tends to break in response to sufficient deformation and, thus, has small plastic and/or elastic deformation range; whereas the term "soft plastic" refers to a plastic that readily deforms under stress without breaking, and, thus, has a large plastic and/or elastic deformation range. In exemplary embodiments, structures may be stacked upon each other. When such a stack is comprised of films or sheets, the structure is laminated. As used herein, the term "laminated" refers to a structure having layers.

The term "minimal-invasive surgery," or "MIS," refers to surgical procedures for treatment, diagnosis, and/or examination of one or more regions of a patient's body using surgical and diagnostic instruments specially developed to reduce the amount of physical trauma associated with the procedure. Generally, MIS involves instruments that may be parsed through natural or surgically created openings of small diameter into a body to their location of use so that examinations and minor surgical interventions are possible with substantially less stress being imposed on the patient, for example, without general anesthesia. MIS may be accomplished using visualization methods, such as fiberoptic or microscopic means. Examples of MIS include, for example, arthoscopic surgery, laparoscopic surgery, endoscopic surgery, thoracic surgery, neurosurgery, bladder surgery, gastrointestinal tract surgery, etc.

The term "nucleic acid" refers to a polymeric form of nucleotides, either ribonucleotides or deoxyribonucleotides or a modified form of either type of nucleotide. The terms should also be understood to include, as equivalents, analogs of either RNA or DNA made from nucleotide analogs, and, as applicable to the embodiment being described, single-stranded (such as sense or antisense) and double-stranded polynucleotides.

The term "polymerize" means to form an aggregate of multiple subunits, where the exact number of subunits in an aggregate is not precisely controlled by the properties of the aggregate itself. For example, "polymerize" does not refer to the formation of a hexameric enzyme complex that is designed to be consistently hexameric. However, the formation of hexamers of, for example, fibrin or actin, is a polymerization. Polymers are generally elongate, but may be of any shape, including a globular aggregate. As used herein, polymerization may occur by any means including, for example, formation of peptide bonds among polymer similar subunits termed isopeptidic bonds (e.g., amide bond or Schiff base formation with lysine and/or primary amines in proteins), disulfide bond formation, or any other mechanism by which polymeric subunits may be linked.

The term "polypeptide", and the terms "protein" and "peptide" which are used interchangeably herein, refers to a polymer of amino acids.

A "subject" is essentially any organism, although usually a vertebrate, and most typically a mammal, such as a human or a non-human mammal.

The term "therapeutically effective amount" refers to that amount of a modulator, drug or other molecule that is sufficient to effect treatment when administered to a subject in need of such treatment. The therapeutically effective amount will vary depending upon the subject and disease condition being treated, the weight and age of the subject, the severity of the disease condition, the manner of administration and the like, which can readily be determined by one of ordinary skill in the art.

As used herein, the term "tissue" refers to an aggregation of similarly specialized cells united in the performance of a particular function. Tissue is intended to encompass all types of biological tissue including both hard and soft tissue, including connective tissue (e.g., hard forms, such as osseous tissue or bone) as well as other muscular or skeletal tissue.

The term "vector" refers to a nucleic acid capable of transporting another nucleic acid to which it has been linked. One type of vector which may be used herein is an episome, i.e., a nucleic acid capable of extra-chromosomal replication. Other vectors include those capable of autonomous replication and expression of nucleic acids to which they are linked. Vectors capable of directing the expression of genes to which they are operatively linked are referred to herein as "expression vectors". In general, expression vectors of utility in recombinant DNA techniques are often in the form of "plasmids" which refer to circular double stranded DNA molecules that, in their vector form are not bound to the chromosome. In the present specification, "plasmid" and "vector" are used interchangeably as the plasmid is the most commonly used form of vector. However, the present dislcosure is intended to include such other forms of expression vectors which serve equivalent functions and which become known in the art subsequently hereto.

The compositions disclosed herein may comprise natural or synthetic organic polymers that can be gelled, or polymerized, or solidified (e.g., by aggregation, coagulation, hydrophobic interactions, or cross-linking) into a 3-D open-lattice structure that entraps water or other molecules, e.g., to form a hydrogel. Structures may comprise a single polymer or a mixture of two or more polymers. Additionally, two or more polymers may be co-deposited or mixed so as to form a polymeric mixture. Polymers used may be biopolymers which can be biocompatible, biodegradable, and/or bioerodible and may act as adhesive substrates for cells. In exemplary embodiments, the polymers disclosed herein arc easy to process into complex shapes and have a rigidity and mechanical strength suitable to maintain the desired shape under *in vivo* conditions.

In some embodiments, the structures disclosed here are formed from plastics that have had most of the water removed and have subsequently been treated with a plasticizing agent (e.g., glycerol). The plastic precursors to final structures can be created in a variety of manners including solid free-form fabrication, such as by ink-jet printing, molding, extrusion, or casting, such as by the methods disclosed herein and known in the art. It.is also possible to form structures by extruding the plastic precursors through a die. The die may, without limitation, have a number of forms so that the extruded plastic is shaped like a tube, filament, rod, or sheet. Extrusion can be accomplished at relatively low pressures and temperatures; under certain processing conditions the plastic may be partially or completely dehydrated by the extrusion process. After sufficient dehydration, with or without the use of osmotic membranes and/or lyophilization, the extruded material may be plasticized and, optionally, cross-linked. It is expected that the extrusion will, in many instances, create an alignment, i.e., anisotropy, of the constituent molecules within the plastic and so impart certain properties, such as toughness, to the final elastomeric and/or pliant materials. If desired, and if patterning is unimportant, growth factors, drugs, antigens, tracers, or other such molecules may be added into the bulk plastic material, such as the admixture or slurry prior to processing.

In some embodiments, compositions of the invention may be produced either by using pressure to force the liquid from the hydrogel, or by the use of a concentration gradient and a semipermeable osmotic membrane to remove the liquid from the hydrogel. Once the water has been essentially removed from the hydrogel, the water can be replaced by a plasticizer. (In some embodiments, it is possible to simultaneously remove water from and add plasticizer to the hydrogel, such as by the use of one or more osmotic membranes.) Other processes may be used to form structures; for example, in one embodiment, the slurry can be extruded through a thin, optionally heated, slit; extrusion can additionally impart improved biomechanical properties by alignment of the molecules along the direction of the extrusion.

In certain embodiments, the water content of a hydrogel can be controlled vacuum drying, i.e., by controlling vacuum and/or temperature so as to dehydrate the hydrogel. Such vacuum processing techniques are especially useful for large scale processing. In yet other embodiments, the water content of the hydrogel is controlled by evaporating the water under normal atmospheric pressure. Evaporative processes may be performed at any temperature. In certain other embodiments, the temperature used to evaporate the water is less than the temperature at which molecules incorporated into the polymer matrix would denature. This is referred to as the subcritical pressure and/or temperature for the inclusion present in a polymer matrix. Such processing techniques allow the polymer matrix of any type of plastic including those disclosed herein to be loaded with a substance and subsequently formed into a structure, such as a film, without loss of bioactivity of the incorporated substance. For example, a gel can be dehydrated at various temperatures that would prevent the degradation or denaturation of heat-sensitive chemicals and proteins, e.g., at a temperature of 75°C, 70°C, 65°C, 60°C, 55°C, 50°C, 45°C, 40°C, 35°C, 30°C, 25°C, or room temperature, and, thus, within a range of less than 70°C, less than 65°C, less than 60°C, less than 55°C, less than 50°C, less than 45°C, less than 40°C, lose than 35°C, less than 30°C, less than 25°C, or room temperature, or less. Use of temperatures of less than room temperature or even less than 4°C are also possible, such as freeze-drying of the hydrogel. Pressure may also be regulated during the drying process. Pressures may be reduced below a normal atmosphere by any means, including use of a gel dryer connected to a vacuum source. Vacuum pressure can be less than 100 millibars, less than 50 millibars, less than 25 millibars, less than 20 millibars, less than 15 millibars, less than 10 millibars, less than 5 millibars, less than 1 millibar, or even less. Those of skill in the art recognize that by reducing the pressure and/or increasing the temperature the drying time can be decreased. Thus, drying may occur over any time period, such as over 1 hour, 2 hours, 4 hours, 8 hours, 16 hours, 24 hours, or longer. Moreover, the drying time can be varied to allow the gel to remain partially hydrated; i.e., wherein not all of the trapped water in the gel is removed.

It some embodiments, the gels can be dried on a substantially planar surface, thus creating a substantially planar film. To prevent curling and deformation of a substantial planar film during the drying process, gels may be placed in a frame, and/or compressed between sheets of material that preserve the forms, such as plastic sheets. In yet other embodiments, the gel can be dried over a formed shape, thus creating a formed film that can be removed from the shape. In yet other embodiments, the gel can be dried directly onto a structure or surface and not removed, thereby creating a film coating on the structure or surface.

In some embodiments, the processing steps can be performed under tensile load conditions to modify subsequent biomechanical properties of the material by aligning filaments of the component material, e.g., fibrin. For example, a rectangular section of a hydrogel can be clamped on opposite sides and lyophilized, resulting in an orientation of the components of the film on the micro- and nano-scale. When plasticizer is added to the resulting material the orientation of the components of material can exhibit improved mechanical properties for application as a graft substitutes for soft tissue repair including vascular, tendon, and ligament tissues. It will be appreciated that fibrous materials, such as fibrin are particularly well-suited for use as forming biocompatible structures as orientation and/or entanglement of the fibers can provide desirable strength to compositions of the invention while maintaining flexibility of the material.

In certain embodiments, a temporal concentration gradient may be created, for example, by capsules designed for timed release of one or more substances. In other embodiments, a temporal concentration gradient may be created through spatial patterning or structural design of the scaffold. For example, a temporal concentration gradient may be created by immobilizing (e.g., via absorption or chemical cross-linking either directly or via an intermediate) one or more substances on the scaffold in a pattern. In this manner, the timing of interaction with the substances will be controlled based on the time it takes for a cell to come into direct contact with the substances immobilized on the scaffold. In another example, a temporal concentration gradient may be created in a scaffold having a fixed porosity by including one or more substances at a remote location on or within the scaffolds. In this manner, interaction with the substances will be delayed during the period of time that it takes a cell to invade the scaffold and reach the remote location within the scaffold. Alternatively, a temporal gradient may be created in a scaffold using a variable porosity to control the rate of cell invasion into the scaffold. As cells encounter a higher porosity environment, the rate of invasion will be slowed, thus delaying interaction with one or more substances located in an area having a higher porosity. In still another embodiment, a temporal gradient may be created using biodegradable or bioresorbable scaffold. As the scaffold breaks down over time, the porosity of the scaffold may decrease, thus permitting cell invasion at a more rapid rate. Alternatively, breakdown of the scaffold may expose a previously inaccessible area within the scaffold.

In accordance with the disclosure, solid free-form fabrication (SFF) processes and apparatus are used in a layering manufacturing process to build up shapes by incremental materials deposition and fusion of thin cross-sectional layers. In certain embodiments, the structures are created *ex vivo* and then administered to a patient (e.g., surgically implanted or attached to a host organism such as a wound, bone fracture, etc.). In yet other embodiments, the articles disclosed herein further comprise kits for use and may further include packaging and directions for intended use of such kits.

In certain embodiments, the structure may be fabricated out of biocompatible materials which are designed for short-term, long-term or permanent implantation into a host organism. For example, a graft may be used to repair or replace damaged tissue or an artificial organ may be used to replace a diseased or damaged organ (e.g., liver, bone, heart, etc.). Alternatively, structures may be fabricated out of biodegradable materials to form temporary structures. For example, a bone fracture may be temporarily repaired with a biodegradable structure that will undergo controlled biodegradation occurring concomitantly with bioremodeling by the host's cells.

In some embodiments, a 3-D structure of the structure may be fabricated directly using SFF. For example, magnetic resonance imaging (MRI) or computerized axial tomography (CAT) scans may be used to determine the 3-D shape of an *in vivo* structure which is to be repaired or replaced. Computer-aided-design (CAD) or computer-aided-manufacturing (CAM) is then used to facilitate fabrication of the 3-D structure using SFF as described herein. Alternatively, the methods and apparatus disclosed herein may be used to produce a non-specifle 3-D structure (e.g., a block or cube), which is then cut or molded into the desired shape (e.g., using a laser, saw, blade, etc.).

Additionally, the methods and apparatus disclosed herein may be used to create structures with specific microstructural organization such that the structure has the anatomical and biomechanical features of naturally occurring tissues, or engineering designs that are biologically inspired. The microstructural organization includes the spatial concentration of intercalated materials (e.g., biological response modifiers, antigens, drugs, hormones, tracers, or labeled compounds), the degree of porosity of the structure, and/or channels that run through the 3-D structure for improved cell invasion, vascularization, and nutrient diffusion.

In some embodiments, once the water is removed from the hydrogel, plasticizer can be added to the resulting material (and in certain other embodiments addition of water and plasticizer can occur simultaneously). In some embodiments, the addition of plasticizer can be accomplished by seating the dehydrated material in a bath of the plasticizer. Biocompatible plasticizers that can be used for this purpose include, but are not limited to, polyalchohols, such as glycerol and mixtures of water and glycerol, as well as other plasticizers known in the art and disclosed herein.

In other embodiments, the strength and toughness of the material can be increased by cross-linking, either as an intermediate step or after forming the material into its final configuration. Any cross-linking agent known in the art or disclosed hereinmay be used.

In certain other embodiments, even if the material is primarily composed of a single precursor material (e.g., fibrin or chitosan), improved properties can be obtained by including other components, such as fibronectin, collagen, gelatin, hyaluronic acid, hormones, biological response modifiers, fillers, tracers, drugs, and/or particulates, such as calcium phosphate (e.g., hydroxyapatito), etc. to produce distinct and unique final materials. Such formulations can be useful in altering the physical characteristics of the material, the mechanical properties, and biological properties (i.e., degradation rate, cell attachment, application to wound site), depending upon the materials that are added to the elastomeric and/or pliant materials. As noted above, the additional bioactive materials can be added to the polymer material in bulk if patterning is not required. However, if patterning is desired, then complex constructs, including three-dimensional spatial patterns, such as gradients, can be created by the use of the bioprinting technology disclosed in U.S. Patent Application Serial No. 10/391,458 filed March 18, 2003 and U.S. Provisional Application for Patent Serial No. 60/619,192 filed October 15,2004, both of which are incorporated herein by reference in their entirety, in order to create patterns of hormones or other bioactive materials upon prefabricated materials, or by printing the materials directly so that the bioactive agents are incorporated within the materials. Such a printing process can be used alone or further processing steps, such as rolling, folding, or stacking such printed elastomeric and/or pliant materials, may be employed in order to fabricate much more complex constructs

Drugs, tracers, hormones, antigens, biological response modifiers, etc. such as protease inhibitors are among the materials that can be incorporated into or onto the biocompatible polymer in order to provide additional control of biodegradation. It is noted that depending on the source of the biopolymer, endogenous factors may already be present in the material; however, in such instances, additional advantages may be obtained by adding exogenous materials to the biopolymer. Exemplary approaches include addition of protease inhibitors and in certain other embodiments immobilization of the inhibitors within the the biopolymer matrix. Non-immobilized forms of inhibitors, although functional, are susceptible to being extracted during dehydration of the hydrogel and/or once applied to tissue implantation site. Immobilization of inhibitors via basic native binding (i.e., plasminogen activator inhibitors) or engineering solid-phase inhibitors (i.e., aprotinin) is a method that can be used to improve retention of inhibitors. Such methods are disclosed herein and known in the art.

In some embodiments, tracers, and/or labeled compounds may be incorporated into the polymer matrix. In some embodiments, the tracer and/or label is a fluorescent molecule, such as a fluorophore or quantum dot. Where such compounds are used, the movement of the label or tracer can be detected, demonstrating for example, degradation, diffusion, etc, of the tracer or labeled compound from the structure. In one embodiment, the fluorescent compound is a fluorescent dye, including, but not limited to, small molecule fluorophores, such as fluorescein, (e.g., fluorescein isothiocyanate (FITC)), Pacific Blue, Cascade Blue, cyanine dyes (e.g., Cy3, Cy5, Cy5.5, and Cy7), Alexa dyes, etc; large fluorescent proteins, such as phycobiliproteins, R-phycoerythrin (R-PE), green fluorescent protein, and allophycocyanin (APC); tandem dyes (i.e., small molecule fluorophores covalently linked to phycobiliproteins, such as PE-TP, PE-Cy5, PE-Cy5.5, PE-Cy7, APC-Cy7, PerCP, etc.); and substances chemically conjugated to such dyes. In another embodiment, quantum dots are used as a tracer for biodegradation of an implanted structure. Quantum dots are nanocrystal semiconductor materials encapsulated by an inorganic "shell" which increases aqueous solubility of the quantum dot. When excited by an energy source, such as a laser, the quantum dots fluoresce. Quantum dots are fluorophores but differ from traditional fluorophores, such as organic fluorescent dyes and naturally fluorescent proteins. They are nanometer-scale atom clusters, containing from a few hundred to a few thousand atoms of a semiconductor material (often, cadmium mixed with selenium or tellurium), which has been coated with an additional semiconductor shell (e.g., zinc sulfide) to improve the optional properties of the material. Unlike, traditional fluorophores, there is no π->π* electronic transitions, thus allowing the quantum dots to be "tuned" to fluoresce over any spectrum and abrogating the need for multiple lasers for multicolor detection studies. In addition, quantum dots, fluoresce brightly over a long period of time making than useful for time-gated studies. In some embodiments, quantum dots are conjugated to proteins to allow detection as in conventional dye conjugate systems but with improved performance characteristics. Incorporation of fluorophores into or on the composition of the invention allows monitoring of an implanted structure in *vivo* in real time without the need for surgery. Methods of detecting fluorescence and various photodetection devices are known in the art.

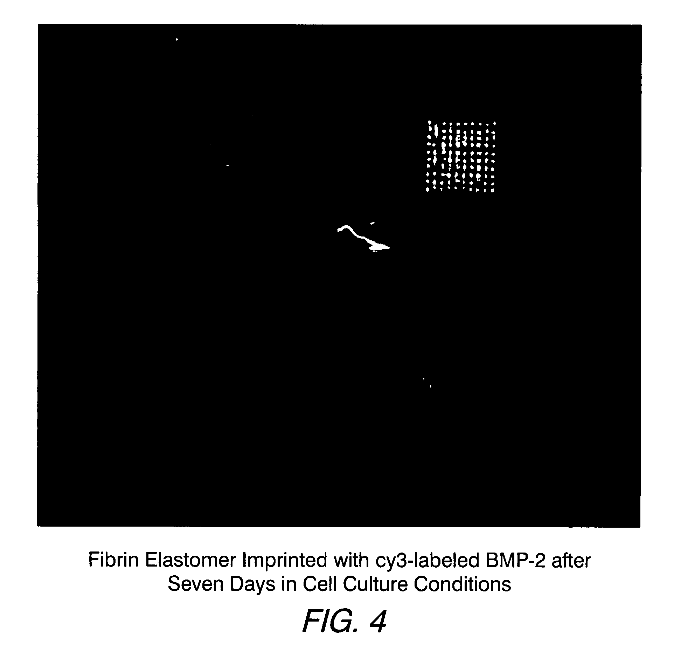

Materials printed upon films as disclosed herein (e.g., bone morphogenetic protein printed on fibrin films) can persist on the printed material for at least one week. See Fig. 4. In certain embodiments, biocompatibility and limited angiogenesis can be demonstrated using the standard chick CAM assay (see, for example, Ribatti, D. et al., Int. J. Dev. Biol., 40, 1189-1197. (1996) Ribatti, D et al., Pathol Res Pract, 192:1068-1076 (1996), and Ribatti, D. et al., Anat Rec, 264:317-324(2001), all of which are incorporated herein by reference).

In some embodiments, multiple layers of polymeric films are stacked atop one another. In such structures, gradients of bioactive materials and/or pores can be created by creating layers of the film each comprising the desired amounts of the bioactive materials within or on the surface of each layer and then stacking the different layers as desired. Such structures can be created in the manner disclosed in U.S. Patent No. 6,165,486, issued December 26, 2000, which is incorporated herein by reference. Such configurations are particularly useful when creating structures to fill cranial voids, for example.

In some embodiments, polymeric films are formed into sheets, tubes, rods, or filaments. Such structures are particularly useful as replacements for tendon, bone, or ligament, for example, and have application in long bone and non-long bone repair. Further incorporating growth factors or anabolic hormones and/or drugs can improve the biological response associated with tissue repair. Tube based structures also find use for vascular grafts and nerve guides, for example. In addition, the films find use as barrier membranes to protect tissues and prevent tissue adhesion. The compositions disclosed herein, e.g., fibrin-based elastomeric films, offer significant advantages in promoting tissue and wound repair.

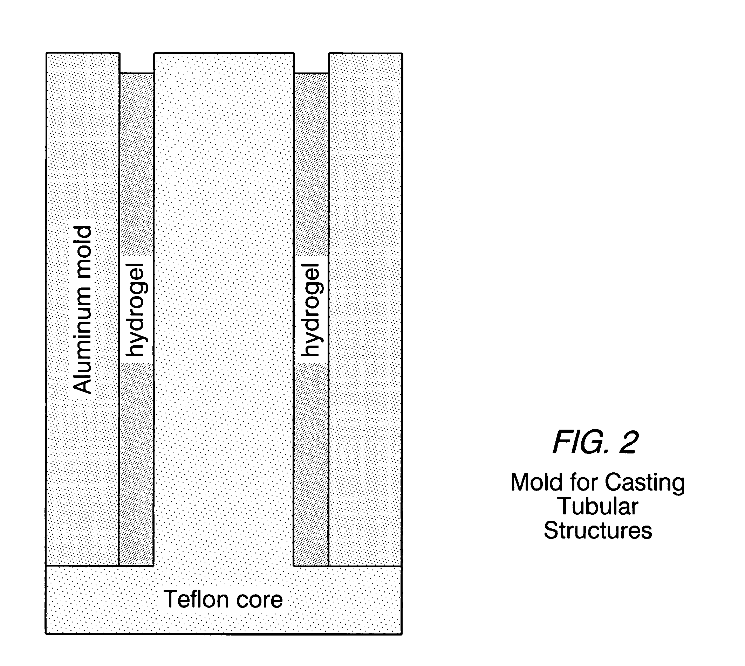

Methods of forming the structure, such as tubular structures, include creating hydrogels which are then cast into molds, as shown in Figure 2. Water can then be removed from the hydrogel (e.g., after removing the tubular structure from the mold or by using osmotic membranes as surfaces of the mold) and replaced by plasticizer. In alternative embodiments, sheats of the compositions disclosed herein are rolled, such as on a mandrel, to create hollow tubular structures, In other embodiments, the hydrogel precursors can be extruded into tubular configurations.

In yet other embodiments, if it is desired to have a cylindrical, non-hollow cross-section a substantially planar composition as disclosed herein may be rolled up on itself, in which case no mandrel would be needed, In certain other embodiments, once the cylindrical form has been attained the elastomer can be cross-linked to retain the tubular shape and/or stapled, heated to a fusing temperature, or otherwise held in the tubular configuration.

In other embodiments, the integrity of a structure, such as those disclosed herein can be increase by including a biocompatible mesh, such as titanium, NYLON^{™}, or DACRON^{™}. The mesh can be added as a layer of the substantially planar material prior to rolling it into a tubular structure or it can form the outer layer of the tubular structure, In alternative embodiments, the materials disclosed herein can be print, cast, or extruded onto the biocompatible mesh materials. Those of skill in the art recognize that the use of mesh materials can also be used to increase the structural integrity of configurations other than tubular or cylindrical shapes.

In certain embodiments, structures may be formed from ionic hydrogels, for example, ionic polysaccharides, such as alginates or chitosan. Ionic hydrogels may be produced by cross-linking the anionic salt of alginic acid, a carbohydrate polymer isolated from seaweed, with ions, such as calcium cations. The strength of the hydrogel increases with either increasing concentrations of calcium ions or alginate. For example, U.S. Pat No. 4,352,883 describes the ionic cross-linking of alginate with divalent cations, in water, at room temperature, to form a hydrogel matrix. In general, these polymers are at least partially soluble in aqueous solutions, e.g., water, or aqueous alcohol solutions that have charged side groups, or a monovalent ionic salt thereof. There are many examples of polymers with acidic side groups that can be reacted with cations, e.g., poly(phosphazenes), poly(acrylic acids), and poly(methacrylic acids). Examples of acidic groups include carboxylic acid groups, sulfonic acid groups, and halogenated (preferably fluorinated) alcohol groups. Examples of polymers with basic side groups that can react with anions are poly(vinyl amines), poly(vinyl pyridine), and poly(vinyl imidazole).

Polyphosphazenes are polymers with backbones consisting of nitrogen and phosphorous atoms separated by alternating single and double bonds. Each phosphorous atom is covalently bonded to two side chains. Polyphosphazenes that can be used have a majority of side chains that are acidic and capable of forming salt bridges with di- or trivalent cations. Examples of acidic side chains are carboxylic acid groups and sulfonic acid groups.

Bioerodible polyphosphazenes have at least two differing types of side chains, acidic side groups capable of terming salt bridges with multivalent cations, and side groups that hydrolyze under *in vivo* conditions, e.g., imidazole groups, amino acid esters, glycerol, and glucosyl groups. Bioerodible or biodegradable polymer i.e., polymers that dissolve or degrade within a period that is acceptable in the desired application (usually *in vivo* therapy), will degrade in less than about five years or in less than about one year, once exposed to a physiological solution of pH 6-8 having a temperature of between about 25°C and 38°C. Hydrolysis of the side chain results in erosion of the polymer. Examples of hydrolyzing side chains arc unsubstituted and substituted imidizoles and amino acid esters in which the side chain is bonded to the phosphorous atom through an amino linkage.

Methods for synthesis and the analysis of various types of polyphosphazenes are described in U.S. Pat. Nos. 4,440,921, 4,495,174, and 4,880,622. Methods for the synthesis of the other polymers described above are known to those skilled in the art. See, for example Concise Encyclopedia of Polymer Science and Polymeric Amines and Ammonium Salts, E. Goethals, editor (Pergamen Press, Elmsford, N.Y. 1980). Many polymers, such as poly(acrylic acid), alginates, and PLURONICS™, are commercially available.

In some embodiments, biopolymer matrices may be manufactured by preparing solid components as an admixture and then subjecting the admixture to pressure to induce formation of a polymer matrix. For example, in one embodiment, biopolymers such as protein, fibrin, fibrinogen, collagen, gelatin, elastin, extracellular matrix constituents, polysaccharide, hylauronic acid, and similar such polymers are powdered (e.g., milled, ground, spray dried, etc.) and then compressed. In other embodiments, the powder is admixed with a substance, such as a plasticizer, a cross-linking agent, a filler, a particulate, a porogen, a biological response modifier, an antigen, a drug, a hormone, a tracer, RNA, DNA, or a labeled compound, combinations thereof, and the like prior to pressing. In certain embodiments, the admixture is compressed at a temperature in which a heat-sensitive compound such as a biological response modifier would not denature or lose bioactivity. For example, in one embodiment, heat-sensitive proteins can be compressed at temperatures below the denaturation temperature or malting point of the protein, thus preserving bioactivity in the polymer matrix after compression. Pressed biopolymers may be made in any shape including 3-dimensional structures and 2-dimensional structures, such as sheets, rods, and filaments. Biopolymer structures can be prepared using different approaches (e.g., casting, cold-pressing, injections molding die extrusion etc.), wherein the amount of pressure applied controlles the thickness and density of the biopolymers structure.

In some embodiments, a structure, such as a bioplastic pellet, is formed from biopolymers by grinding solidified biopolymer into a powder with particle sizes less than or equal to 150 microns (e.g., at least 100 mesh standard sieve). In some other embodiments, a plasticizer is added to the biopolymer, e.g., 12.5%, 19%, 21%, 25%, 35% and 50% plasticizer by weight. In yet other embodiments, the powder (and any additional components in the admixture) are mixed with, for example, glycerin, and mixed until homogenous. In some other embodiments, the biopolymer compositions disclosed herein are then pressed. Those of skill in the art recognize that compression may be accomplished by any means including, for example, using a pellet press. Compression may occur at any suitable pressure such as 1000lbs., 2000lbs., 3000lbs., 4000lbs., 5000lbs., 6000lbs., 70001bs., 8000lbs. of pressure. Those of skill in the art recognize that such pressures can be reported in any manner including interconversion to PSI, torr, bars or any other suitable measurement scale. In certain other embodiments, a mold release agent, such as lecithin is used to facilitate removal of a compressed biopolymer from a press or mold. In some other embodiments, a biopolymer is compressed at a suitable temperature (e.g., 60°, 66°, 70°, 80°C) and at a suitable pressure (e.g., 5000 lbs to 7000 lbs) and for a suitable time (e.g., 1, 5, 10, 20, 30, 40, 50, 60 minutes or longer) to form a biopolymer matrix. In certain other embodiments, the mold pressure is maximized and remains steady (e.g., at approximately 7000 lbs of pressure) for approximately 20 minutes. In yet other embodiments, the mold temperature is decreased from an initial value of approximately 80°C until reaching a final steady value of approximately 27°C (room temperature).

As discussed above, a biopolymer admixture may comprise additional components mixed with the polymer powder, such as a porogen. Such porogens may be distributed homogenously within the biopolymer powder or non-homogenously. Non-homgenous distribution of porogen can be used to introduce a pore gradient and/or distribution within the final formed structure, to modulate the biodegrability, mecanical properties, etc., of the structure. Additionally, other components can be incorporated in such a manner, homogenously or inhomogenously, to introduce for example, biochemical gradients of a substance within the polymer matrix.

In some embodiments, water soluble polymers with charged side groups can be cross-linked by reacting a polymer with an aqueous solution containing multivalent ions of the opposite charge, either multivalent cations if the polymer has acidic side groups, or multivalent anions if the polymer has basic side groups. Cations for cross-linking the polymers with acidic side groups to form a hydrogel include divalent and trivalent cations, such as copper, calcium, aluminum, magnesium, and strontium. Aqueous solutions of the salts of these cations can be added to the polymers to form soft, highly swollen hydrogels and membranes. Anions for cross-linking the polymers to form a hydrogel include divalent and trivalent anions such as low molecular weight dicarboxylate ions, terepthalate ions, sulfate ions, and carbonate ions. Aqueous solutions of the salts of these anions can be added to the polymers to form soft, highly swollen hydrogels and membranes, as described with respect to cations.

Also, a variety of polycations can be used to complex and thereby stabilize the polymer into a semi-permeable surface membrane. Examples of polycations include poly-L-lysine, as well as natural polycations, such as the polysaccharide, chitosan.

In some embodiments, polymers, such as those known in the art and disclosed herein, can be cross-linked using chemical cross-linking agents. In yet other embodiments, the chemical cross-linking agent is a solid. In some other embodiments the solid cross-linking agent becomes active in the presence of water. Thus, in some embodiments, a solid cross-linking agent can be admixed with a dry polymer or otherwise incorporated into a dehydrated polymer matrix (e.g., fibrin, gelatin, etc.). Without being bound by any mechanism of action or theory, it has been surprisingly discovered that solid cross-linking agents can be active, even prior to hydration, for example, where the polymer contains residual water and/or amino groups. Thus, where a solid crosslinking agent is used, it can be active prior to hydration or in the alternative, upon expose to water. For example, in one embodiment, the solid cross-linking agent, genipin, is incorporated into the polymer admixture and subsequently allowed to be activated by water which is either already in the polymer and/or which is absorbed when the subsequently formed polymer matrix is placed in a water bath. Because water is either already in the matrix (e.g., because of compactions) or rapidly diffuses into a polymer matrix (e.g., a biopolymer matrix), the cross-linking occurs rapidly and uniformly thoughout the gel. This method obviates the problem often observed with liquid cross-linking agents, which cross-link as they diffuse into the gel, creating a stiff outer shell, while the internal part of gel swells with water since it has not been exposed to the cross-linking agent due to the slow diffusion of the cross-linking agent. Such inhomogenitey can create pressure within the structure, sometimes leading to cracking and deformation.

Thus in some embodiments, a solid cross-linking agent such as genipin can be used. Genipin (Cyclopenta(c) pyran-4-carboxylic acid, 1,4a-alpha,5,7a-alpha-tetrahydro-1-hydroxy-7-(bydro)(ymethyl)-, methyl ester⁴) is a pyran hydrolytic product of geniposide, and it capable of forming cross-links with amines. Genipin is a non-toxic cross-linking agent, and, thus, better suited for use in numerous biomedical applications, since many other cross-linking agents such as glutaraldehyde have been shown to be toxic to cells. In addition, genipin conjugates turn a blue color and fluoresce, thus allowing visual monitoring of the extent and positions of the cross-linking, in real-time. In certain other embodiments, the solid cross-linking agent is formaldehyde sodium bisulfite, for example. In certain embodiments, NHS-esters of carboxylic acids are used as a solid cross-linking agent.

Those of skill in the art recognize that retention of molecules within a polymer matrix can enhanced if the matrix is selectively permeable, i.e., the matrix allows diffusion of smaller molecules but not larger one. For example, in order to prevent the passage of antibodies and other proteins having a molecular weight greater than 30,000 D through the matrix but allowing passage of nutrients essential for cellular growth and metabolism, a useful permeability of the macromer/polymer is in the range of between 10,000 D and 30,000 D, for example. Smaller macromers result in polymer matrices of a higher density with lower molecular weight cut-offs.

The speed of erosion of a scaffold produced from a bioerodible or biodegradable polymer is also related to the molecular weights of the polymer. Higher molecular weight polymers (e.g., with average molecular weights of 90,000 or higher) produce scaffolds which retain their structural integrity for longer periods of time, while lower molecular weight polymers (e.g., average molecular weights of 30,000 or less) produce scaffolds which erode much more quickly.

In some embodiments, additional features, such as roughened spots, pores, holes, etc, are introduced into the scaffolds by machining milling, grinding, etc. to promote osteoconductive growth. Cells readily migrate and attach upon such roughened surfaces.

Introduction of pores into the compositions of the invention may also be used to regulate permeability, degradation rate, and mechanical properties of the compositions disclosed herein. For example, pores may be introduced mechanically or chemically into the polymer matrix. In certain embodiments, pores are introduced mechanically, such as by machining (e.g., punching) holes in a film that is subsequently stacked or rolled as described herein. In some other embodiments, pores are introduced chemically by incorporating a porogen into the polymer and subsequently removing it once the polymer matrix has formed. In certain embodiments, the porogen is soluble in aqueous or organic solvents. When a polymer matrix comprising the soluble porogen is placed in contact with a solvent, the porogen disuses out of the polymer, leaving pores. In some embodiments, NaCl particles can be used as a porogen with water as the solvent. In other embodiments, where the porogen is soluble in the organic phase, polyurethane, polylactic acid, polylactic-*co*-glycolic acid, or polycaprolactone can be used in conjunction with an organic solvent, such as chloroform for example. Using organic-soluble porogens in a polymer matrix can be advantageous where the polymer matrix comprises additional water-soluble substances, such as biological response modifiers and the like, since these will not diffuse out of the polymer matrix when it is placed in the organic solvent. In certain embodiments, the organic phase system can operate more efficiently than aqueous phase systems, as it has been observed that in certain embodiments residual NaCl may remain in the polymer matrix after leaching.

In yet other embodiments, the porogen can be a sublimation porogen. A sublimation porogen will sublime directly into the gas phase under the appropriate temperature and pressure, thus obviating the need for solvent-leaching altogether. In certain embodiments, the sublimation porogen can be ammonium acetate, ammonium chloride, ammonium bicarbonate, ammonium carbonate, or pyridinium trifluoroacetate, for example. Those of skill in the art recognize that the temperature and pressure at which a substance sublimes is known as the triple point from a phase diagram. Thus, to introduce pores using sublimation, the temperature and pressure must be below this triple point of the porogen. As such, virtually any substance where the triple point is known or can be determined can be used as a sublimation porogen in the methods and compositions of the invention. In additional embodiments, the sublimation porogen is bio-compatible, meaning that should any residual porogen remain in the polymer matrix, it would not be toxic to an organism upon implantation or use. In yet other embodiments, the sublimation porogen is removed under sufficiently mild conditions (such as low temperature and pressure) that any additional substance included in the polymer matrix, such as heat-sensitive proteins or drugs are not denatured or degraded during the sublimation process; e.g. less than 80°C, less than 70 °C, less than 65 °C, less than 60 °C, less than 55 °C, less than 50 °C, less than 45 °C, less than 40 °C, less than 35 °C, less than 30 °C, or room temperature, or less. In certain embodiments, the sublimation porogen is removed by reducing pressure, such as removal using a vacuum. Vacuum pressure can be less than 100 millibars, less than 50 millibars, less than 25 millibars, less than 20 millibars, less than 15 millibars, less than 10 millibars, less than 5 millibars, less man 1 millibar, or less. In yet other embodiments, the sublimation porogen is removed along with water, e.g., drying a gel under a vacuum as discussed *supra*, and, thus, removing both water and the porogen at the drying temperatures and pressures disclosed herein.

Notwithstanding the method of introduction, pores may be closed (i.e., pores not forming a contiguous space with other pores or the surface) or interconnected (i.e., pores form a contiguous space with other pores or the surface). In certain embodiments, the compositions of the invention comprise interconnected pores. Such interconnected pores are advantageous for biological uses because porogen residue is less likely to be trapped within a polymer matrix, and, thus, the construct is more likely to be biocompatible.

In other embodiments, compositions disclosed herein may be temperature-dependent or thermosensitive hydrogels. These hydrogels must have so-called "reverse gelation" properties, i.e., they are liquids at or below room temperature, and gel when warmed to higher temperatures, e.g., body temperature. Thus, these hydrogels can be easily applied at or below room temperature as a liquid and automatically form a semi-solid gel when warmed to body temperature. Examples of such temperature-dependent hydrogels are PLURONICS™ (BASF-Wyandotte), such as polyoxyethylene-polyoxypropylene F-108, F-68, and F-127, poly (N-isopropylacrylamide), and N-isopropylacrylamide copolymers.

Those of skill in the art recognize that polymers can be manipulated to affect their physical properties, such as porosity, rate of degradation, transition temperature, and degree of rigidity. For example, the addition of low molecular weight saccharides in the presence and absence of salts affects the lower critical solution temperature (LCST) of typical thermosensitive polymers. In addition, when these gels are prepared at concentrations ranging between 5 and 25% (W/V) by dispersion at 4°C., the viscosity and the gel-sol (gel-solution) transition temperature are affected, the gel-sol transition temperature being inversely related to the concentration. These gels have diffusion characteristics capable of allowing cells to survive and be nourished. For example, U.S. Pat. No. 4,188,373 describes using PLURONIC™ polyols in aqueous compositions to provide thermal gelling aqueous systems. U.S. Pat. Nos. 4,474,751, 4,474,752, 4,474,753, and 4,478,822 describe drug delivery systems which utilize thermosetting polyoxyalkylene gels; with these systems, both the gel transition temperature and/or the rigidity of the gel can be modified by adjustment of the pH and/or the ionic strength, as well as by the concentration of the polymer.

In yet other embodiments, the structures disclosed herein may be pH-dependent hydrogels. These hydrogels are liquids at, below, or above specific pH values, and gel when exposed to specific pHs, e.g., 7.35 to 7.45, the normal pH range of extracellular fluids within the human body. Thus, these hydrogels can be easily applied in the body as a liquid and automatically form a semi-solid gel when exposed to body pH. Examples of such pH-dependent hydrogels are TETRONICS™. (BASF-Wyandotte) polyoxyethylene-polyoxypropylene polymers of ethylene diamine, poly(diethyl aminoethyl metharylate-g-ethylene glycol), and poly(2-hydroxymethyl methacrylate). Such copolymers can be manipulated by standard techniques to affect their physical properties.

In certain embodiments, structures disclosed herein can comprise light solidified plastics, e.g., solidified by either visible or ultraviolet light. In certain embodiments, hydrogels are made of macromers including a water soluble region, a biodegradable region, and at least two polymerizable regions as described, for example, in U.S. Pat. No. 5,410,016. For example, the hydrogel can begin with a biodegradable, polymerizable macromer including a core, an extension on each end of the core, and an end cap on each extension. The core is a hydrophilic polymer, the extensions are biodegradable polymers, and the end caps are oligomers capable of cross-linking the macromers upon exposure to visible or ultraviolet light, e.g., long wavelength ultraviolet light.

Examples of such light solidified polymers can include polyethylene oxide block copolymers, polyethylene glycol polylactic acid copolymers with acrylate end groups, and 10,000 D polyethylene glycol-glycolide copolymer capped by an acrylate at both ends. As with the PLURONIC™ hydrogels, the copolymers comprising these hydrogels can be manipulated by techniques known to the skilled artisan to modify their physical properties such as rate of degradation, differences in crystallinity, and degree of rigidity.

In other embodiments, structures, such as those disclosed herein, may be a bioerodible or biodegradable synthetic polymers. Suitable polymers include, for example, bioerodible polymers such as polylactic acid (PLA), polyglycolic acid (PGA), polylactide-*co*-glycolide (PLGA), polycaprolactone, polycarbonates, polyamides, polyanhydrides, polyamino acids, polyortho esters, polyacetals, polycyanoacrylates and degradable polyurethanes, and non-erodible polymers, such as polyacrylates, ethylene-vinyl acetate polymers, and other acyl substituted cellulose acetates, and derivatives thereof, non-erodible polyurethanes, polystyrenes, polyvinyl chloride, polyvinyl fluoride, poly(vinyl imidazole), chlorosulphonated polyolefins, polyethylene oxide, polyvinyl alcohol, TEFLON™, and nylon. In an exemplary embodiment, the structure comprises a PLA/PGA copolymer that is biodegradable.

Disclosed herein are systems, compositions, and methods useful for making and using scaffolds, which may be implanted at a desired location. The scaffolds disclosed herein may be used to prepare a scaffold for any mammal in need thereof. Mammals of interest include humans, dogs, cows, pigs, cats, sheep, horses, and the like, preferably humans.

The methods, compositions, and apparatus disclosed herein may be used to prepare a variety of scaffolds that may be utilized as xenografts, allografts, artificial organs, or other cellular transplantation therapeutics. The scaffolds may be used to repair and/or replace any damaged tissue associated with a host. The scaffolds dislcosed herein may also be suitable for other applications, such as for hormone producing or tissue producing implants for deficient individuals who suffer from conditions, such as diabetes, thyroid deficiency, growth hormone deficiency, congenital adrenal hyperplasia, Parkinson's disease, and the like. Likewise, apparatus and methods disclosed herein may be useful for creating scaffolds suitable for therapeutic applications, including, for example, implantable delivery systems providing biologically active and gene therapy products. For example, the scaffolds disclosed herein may be useful for the treatment of the central nervous system, to provide a source of cells secreting insulin for treatment of diabetes, cells secreting human nerve growth factors for preventing the loss of degenerating cholinergic neurons, satellite cells for myocardial regeneration, striatal brain tissue for Huntigton's disease, liver cells, bone marrow cells, dopamine-rich brain tissue, and cells for Parkinson's disease, cholinergic-rich nervous system cell for Alzheimer's disease, adrenal chromaffin cells for delivering analgesics to the central nervous system, cultured epithelium for skin grafts, and cells releasing ciliary neurotrophic factor for amyotrophic lateral sclerosis, and the like. In an exemplary embodiment, the scaffolds disclosed herein may be used to repair bone injuries and induce healing thereof by inducing vascularization to the site of injury.

In other exemplary embodiments, the methods, compositions, and apparatus disclosed herein may be used to create 3-D scaffolds capable of providing a spatial and/or temporally organized therapeutic to a host at a desired location. In such embodiments, the scaffolds contain 3-D patterns of therapeutic and/or diagnostic agents, such as biological response modifiers, antigens, drugs, hormones, tracers, or labeled compounds that operate within the host in a predictable and organized manner. For example, a scaffold may have gradients of one or more growth factors which vary throughout the structure, such as a concentration gradient that diminishes from the center of the structure to the periphery, a gradient from one side of the structure to the other, etc., in an infinite variety of possible configuration. In addition to spatial gradients, temporal gradients may also be engineered using the time release mechanisms described above. Using such spatial and/or temporal gradients, organized doses of one or more therapeutic factors can be provided to an organism in need thereof. For example, such spatial and temporal therapeutics may be used to induce organized neovascularization in a host at a desired location. During wound healing, angiogenic factors are produced at the site of injury producing a concentration gradient which decreases away from the site of injury. However, traditional approaches to inducing angiogenesis involve uniform application of angiogenic factors which typically lead to unorganized vessel formations or angiomas. The scaffolds disclosed herein may be engineered so as to provide a concentration gradient of angiogenic factors in a 3-D spatial and/or temporal configuration that mimics the naturally occurring wound healing response signals resulting in formation of organized and directed neovascularization at a desired location in a host.

In another embodiment, scaffolds may contain a 3-D pattern of adhesion molecules specific for one or more cell types. For example, a 3-D pattern of adhesion molecules may be configured so as to attract and adhere particular cell types to the scaffold in a desired 3-D architecture. These scaffolds can be used to induce a desired configuration of cell attachment/tissue formation at a specified location. The scaffold may be a permanent or long-term implant or may degrade over time as the host's natural cells replace the scaffold. In an exemplary embodiment, two or more adhesion molecules with different cell binding specificities are patterned on the scaffold so as to immobilize two or more desired cell types into a specific 3-D pattern. In practicing this exemplary embodiment, a variety of techniques can be used to foster selective cell adhesion of two or more cell types to the scaffold. For example, adhesion proteins, such as collagen, fibronectin, gelatin, collagen type IV, laminin, entactin, and other basement proteins, including glycosaminoglycans, such as heparan sulfate, RGD peptides, ICAMs, E-cadherins, and antibodies that specifically bind a cell surface protein (for example, an integrin, ICAM, selectin, or E-cadherin). Also envisioned are methods, such as localized protein adsorption, organosilane surface modification, alkane thiol self-assembled monolayer surface modification, wet and dry etching techniques for creating 3-D substrates, radiofrequency modification, and ion-implantation (Lom et al., 1993, J. Neurosci. Methods 50:385-397; Brittland et al., 1992, Biotechnology Progress 8:155-160; Singhvi et al., 1994, Science 264:696-698; Singhvi et al., 1994, Biotechnology and Bioengineering 43:764-771; Ranieri et al., 1994, Intl. J. Devel. Neurosci. 12(8)-725-735; Bellamkonda et al., 1994, Biotechnology and Bioengineering 43:543-554; and Valentini et al., 1993, J. Biomaterials Science Polymer Edition 5(1/2): 13-36).

In still other embodiments, the therapeutic bio-inks disclosed herein may be cells which may be used to directly seed a 3-D cellular architecture of one or more cell types. Combinations of these approaches are also envisioned, e.g., 3-D patterns of cells and growth factors. In other embodiments, cells may be used to coat small or large surface areas of devices, wound dressings or areas of the body. Such coatings may be applied directly to the device and applied to a desired location. In various embodiments, cells may be applied individually or as a population aliquot. Any structure disclosed herein may be seeded with cells of therapeutic use, including stem cells.

In certain embodiments, the apparatus, methods, and compositions described herein may be used to create interpenetrating polymer networks (IPNs). IPNs are blends or alloys of two or more polymer components, each of which is a cross-linked 3-D network. The individual polymer component networks are more or less physically entangled with, but not covalently bonded to, the other polymer network(s) in the IPN. A feature of IPNs is that they permit combining advantageous properties from each of two polymers which are normally incompatible. For example, in a hydrophobic-hydrophilic system, flexibility and structural integrity might be imparted by the hydrophobic polymer and lubriciousness might be imparted by the hydrophilic polymer. An IPN may be a bicontinuous system in which each of the polymers forms a continuous matrix throughout the network.

In another embodiment, the apparatus, methods, compositions, and products disclosed herein may be used in association with minimally invasive surgery techniques. For example, a scaffold may be created *in situ*, or may be pre-fabricated and implanted into a patient, at a desired location using minimally invasive techniques. In certain embodiments, minimally invasive surgical techniques may be used to provide tissue sealants at focused areas and/or to provide short term and/or long term administration of therapeutic agents, including for example, cells, polypeptides, polynucleotides, growth factors, drugs, etc. In one exemplary embodiment, minimally invasive techniques may be used to provide scaffolds for repairing hyaline cartilage and/or fibrocartilage in diarthroidal and amphiarthroidal joints. In another exemplary embodiment, a resorbable vascular wound dressing may be delivered in association with angioplasty procedures to deliver or fabricate a scaffold to selected sites inside or outside a blood vessel. Vascular wound dressings may be tubular, compliant, self-expendable, low profile, biocompatible, hemocompatible, and/or bioresorbable. In certain embodiments, such wound dressings may prevent or substantially reduce the risk of post-angioplasty vessel reclosure. In other embodiments, vascular wound dressings may be fabricated with a therapeutic agent for treatment of vessel wounds, including, for example, anti-platelet agents, such as aspirin and the like, anti-coagulant agents, such as coumadin and the like, antibiotics, anti-thrombus deposition agents, such as polyanionic polysaccharides including heparin, chondroitin sulfates, hyaluronic acid and the like, urokinase, streptokinase, plasminogin activators and the like, wound healing agents, such as transforming growth factor beta (TGF beta) and the like, glycoproteins, such as laminin, fibronectin and the like, various types of collagens.

In another embodiment, the apparatus, methods, compositions, and products disclosed herein may be used to create bioresorbable wound dressings or band-aids. Wound dressings may be used as a wound-healing dressing, a tissue sealant (i.e., sealing a tissue or organ to prevent exposure to a fluid or gas, such as blood, urine, air, etc., from or into a tissue or organ), and/or a cell-growth scaffold. In various embodiments, the wound dressing may protect the injured tissue, maintain a moist environment, be water permeable, be easy to apply, not require frequent changes, be non-toxic, be non-antigenic, maintain microbial control, and/or deliver effective healing agents to the wound site.

Examples of bioresorbable sealants and adhesives that may be used in accordance with the apparatus, methods, and compositions described herein include, for example, FOCALSEAL™ produced by Focal; BERIPLAST™ produced by Adventis-Bering; VIVOSTAT™ produced by ConvaTec (Bristol-Meyers-Squibb); SEALATEN™ produced by Baxter; FIBRX™ produced by CyoLife; TISSEEL™ and TISSUCOL™ produced by Baxter; QUIXIL™ produced by Omrix Biopharm; a PEG-collagen conjugate produced by Cohesion (Collagen); HYSTOACRYL BLUE™ produced by Davis & Geck; NEXACRYL, NEXABOND, NEXABOND S/C™, and TRAUMASEAL™ produced by Closure Medical (TriPoint Medical); OCTYL CNA™ produced by Dermabond (Ethicon); TISSUEGLU™ produced by Medi-West Pharma; and VETBOND™ produced by 3M.

Wound dressings may be used in conjunction with orthopedic applications, such as bone filling/fusion for osteoporosis and other bone diseases, cartilage repair for arthritis and other joint diseases, tendon repair; for soft tissue repair, including nerve repair, organ repair, skin repair, vascular repair, muscle repair, and ophthalmic applications. In exemplary embodiments, wound dressings may be used to treat a surface, such as, for example, a surface of the respiratory tract, the meninges, to synovial spaces of the body, the peritoneum, the pericardium, the synovia of the tendons and joints, the renal capsule and other serosae, the dermis and epidermis, the site of an anastomosis, a suture, a staple, a puncture, an incision, a laceration, or an apposition of tissue, a ureter or urethra, a bowel, the esophagus, the patella, a tendon or ligament, bone or cartilage, the stomach, the bile duct, the bladder, arteries, and veins.

In exemplary embodiments, wound dressings may be used in association with any medical condition that requires coating or sealing of a tissue. For example, lung tissue may be sealed against air leakage after surgery; leakage of blood, serum, urine, cerebrospinal fluid, air, mucus, tears, bowel contents, or other bodily fluids may be stopped or minimized; barriers may be applied to prevent post-surgical adhesions, including those of the pelvis and abdomen, pericardium, spinal cord and dura, tendon, and tendon sheath. Wound dressings may also be useful for treating exposed skin, in the repair or healing of incisions, abrasions, burns, inflammation, and other conditions requiring application of a coating to the outer surfaces of the body. Wound dressings may also be useful for applying coatings to other body surfaces, such as the interior or exterior of hollow organs, including blood vessels. Restenosis of blood vessels or other passages may also be treated.

The range of uses for wound dressings also include cardiovascular surgery applications, prevention of bleeding from a vascular suture line; support of vascular graft attachment; enhancing preclotting of porous vascular grafts; stanching of diffuse non-specific bleeding; anastomoses of cardiac arteries, especially in bypass surgery; support of heart valve replacement; sealing of patches to correct septal defects; bleeding after sternotomy; and arterial plugging; thoracic surgery applications, including sealing of bronchopleural fistulas, reduction of mediastinal bleeding, sealing of esophageal anastomoses, and sealing of pulmonary staple or suture lines; neurosurgery applications, including dural repairs, microvascular surgery, and peripheral nerve repair; general surgery applications, including bowel anastomoses, liver resection, biliary duct repair, pancreatic surgery, lymph node resection, reduction of seroma and hematoma formation, endoscopy-induced bleeding, plugging or sealing of trocar incisions, and repair in general trauma, especially in emergency procedures; plastic surgery applications, including skin grafts, burns, debridement of eschars, and blepharoplasties (eyelid repair); otorhinolaryngology (BNT) applications, including nasal packing, ossicular chain reconstruction, vocal cord reconstruction and nasal repair; opthalmology applications, including corneal laceration or ulceration, and retinal detachment; orthopedic surgery applications, including tendon repair, bone repair, including filling of defects, and meniscus repairs; gynecology/obstetrics applications, including treatment of myotomies, repair following adhesiolysis, and prevention of adhesions; urology applications, including sealing and repair of damaged ducts, and treatment after partial nephrectomy are potential uses; dental surgery applications, including treatment of periodontal disease and repair after tooth extraction; repair of incisions made for laparoscopy or other endoscopic procedures, and of other openings made for surgical purposes, are other uses; treatment of disease conditions, such as stopping diffuse bleeding in hemophiliacs; and separation of tissues to prevent damage by rubbing during healing. In each case, appropriate therapeutic agents may be included in the wound dressing.

In certain embodiments, wound dressings may be fabricated with therapeutic bio-inks to provide delivery of a therapeutic agent at a site of injury, including, for example, antiinfectives, such as antibiotic, anti-fungal or anti-viral agents, anti-inflammatory agents, mitogens to stimulate cell growth and/or differentiation, agents to stimulate cell migration to the site of injury, growth factors, cells, such as osteoblasts, chondrocytes, keratinocytes, and hepatocytes, to restore or replace bone, cartilage, skin, and liver tissue, respectively, etc. Alternatively, therapeutic agents may be added to the wound dressing after fabrication, e.g., by soaking, spraying, painting, or otherwise applying the therapeutic agent to the dressing.

In various embodiments, wound dressings may be fabricated directly at a desired location or may be pre-fabricated and applied to the wound. Wound dressings may be in the form of flat films that may be adhered to tissue to cover the site of an injury or may be in the form of 3-D structures, such as plugs or wedges. In some embodiments, prosthetic tissue interfaces may be engineered which can be used, for example to put inside, around, beside another material such as metal to promote adhesion and ingrowth of tissue. Pre-fabricated wound dressings may be supplied in standard configurations suitable for application to a variety of wounds and may be applied as is or may be cut, molded, or otherwise shaped prior to application to a particular wound. Alternatively, pre-fabricated wound dressings may be produced in a configuration tailored to a specific wound or wound type. In one embodiment, the wound dressing is supplied as a moist material that is ready for application to a wound. In another embodiment, the wound dressing is supplied as a dried material which may be rehydrated upon or prior to application to a wound.

In another embodiment, the apparatus, methods, compositions, and products disclosed herein may be used to fabricate coatings for devices to be used in the body or in contact with bodily fluids, such as medical devices, surgical instruments, diagnostic instruments, drug delivery devices, and prosthetic implants. Coatings may be fabricated directly on such objects or may be pre-fabricated in sheets, films, blocks, plugs, or other structures and applied/adhered to the device. Such coatings may be useful as a tissue-engineering scaffold, as a diffusion membrane, as a method to adhere the implant to a tissue, as a delivery method for a therapeutic agent, and/or as a method to prolong implant stability, e.g., by preventing or suppressing an immune response to the implant from the host. In various embodiment, coatings may be applied to implantable devices, such as pacemakers, defibrillators, stents, orthopedic implants, urological implants, dental implants, breast implants, tissue augmentations, heart valves, artificial corneas, bone reinforcements, and implants for maxillofacial reconstruction; devices such as percutaneous catheters (e.g., central venous catheters), percutaneous cannulae (e.g., for ventricular assist devices), catheters, urinary catheters, percutaneous electrical wires, ostomy appliances, electrodes (surface and implanted), and supporting materials, such as meshes used to seal or reconstruct openings; and other tissue-non-tissue interfaces.

In an exemplary embodiment, a composition of the invention may be placed into a seeping wound to seal off the blood flow. Such wound plug or blood clotting applications may be particularly useful, for example, in battlefield applications.

In certain embodiments, wound dressings may be fabricated to provide delivery of a therapeutic agent at a desired location. Therapeutic agents may be included in a coating as an ancillary to a medical treatment (for example, antibiotics) or as the primary objective of a treatment (for example, a gene to be locally delivered). A variety of therapeutic agents may be used, including passively functioning materials, such as hyaluronic acid, as well as active agents, such as growth hormones. A wide variety of therapeutic agents may be used, including, for example, cells, proteins (including enzymes, growth factors, hormones, and antibodies), peptides, organic synthetic molecules, inorganic compounds, natural extracts, nucleic acids (including genes, antisense nucleotides, ribozymes, and triplex forming agents), lipids and steroids, carbohydrates (including heparin), glycoproteins, and combinations thereof. The agents to be incorporated can have a variety of biological activities, such as vasoactive agents, neuroactive agents, hormones, anticoagulants, immunomodulating agents, cytotoxic agents, antibiotics, antivirals, or may have specific binding properties, such as antisense nucleic acids, antigens, antibodies, antibody fragments, or a receptor.

In other exemplary embodiments, the methods, compositions, and apparatus disclosed herein may be used to create 3-D capsules, tablets, structures, and matrices that deliver therapeutic agents including antibiotics, antivirals, anti-inflammatories, both steroidal and non-steroidal, anti-neoplastics, anti-spasmodics, including channel blockers, modulators of cell-extracellular matrix interactions, including cell growth inhibitors and anti-adhesion molecules, enzymes and enzyme inhibitors, anticoagulants and/or antithrombotic agents, growth factors, DNA, RNA, inhibitors of DNA, RNA or protein synthesis, compounds modulating cell migration, proliferation and/or growth, vasodilating agents, and other drugs commonly used for the treatment of injury to tissue. Specific examples of these compounds include angiotensin converting enzyme inhibitors, prostacyclin, heparin, salicylates, nitrates, calcium channel blocking drugs, streptokinase, urokinase, tissue plasminogen activator (TPA) and anisoylated plasminogen activator (TPA) and anisoylated plasminogen-streptokinase activator complex (APSAC), colchicine and alkylating agents, and aptamers. Specific examples of modulators of cell interactions include interleukins, platelet derived growth factor, acidic and basic fibroblast growth factor (FGF), transformation growth factor beta (TGF-beta), epidermal growth factor (EGF), insulin-like growth factor, and antibodies thereto. Specific examples of nucleic acids include genes and cDNAs encoding proteins, expression vectors, antisense and other oligonucleotides, such as ribozymes which can be used to regulate or prevent gene expression. Specific examples of other bioactive agents include modified extracellular matrix components or their receptors, and lipid and cholesterol sequestrants.

In further embodiments, therapeutic agents which may be used in conjunction the compositions and methods of the invention include proteins, such as biological response modifiers including cytokines, interferons and interleukins, proteins, and colony-stimulating factors. Carbohydrates including sialyl Lewis antigen which has been shown to bind to receptors for selectins to inhibit inflammation. A "deliverable growth factor equivalent" (DGFE), a growth factor for a cell or tissue, may be used, which is broadly construed as including growth factors, cytokines, interferons, interleukins, proteins, colony-stimulating factors, gibberellins, auxins, and vitamins; further including peptide fragments or other active fragments of the above; and further including vectors, i.e., nucleic acid constructs capable of synthesizing such factors in the target cells, whether by transformation or transient expression; and further including effectors which stimulate or depress the synthesis of such factors in the tissue, including natural signal molecules, antisense and triplex nucleic acids, and the like. Exemplary DGFE's are VEGF, ECGF, bFGF, BMP, and PDGF, and DNA's encoding for them Exemplary dot dissolving agents are tissue plasminogen activator, streptokinase, urokinase, and heparin.

In other embodiments, drugs having antioxidant activity (i.e., destroying or preventing formation of active oxygen) may be used, which are useful, for example, in the prevention of adhesions. Examples include superoxide dismutase, or other protein drugs include catalases, peroxidases, and general oxidases or oxidative enzymes, such as cytochrome P450, glutathione peroxidase, and other native or denatured hemoproteins.

In still other embodiments, mammalian stress response proteins or heat shock proteins, such as heat shock protein 70 (hsp 70) and hsp 90, or those stimuli which act to inhibit or reduce stress response proteins or heat shock protein expression, for example, flavonoids, also may be used.

As described above, the pharmaceutical composition or the therapeutic agent for overactive bladder of the present invention may be used, administered or produced in a single preparation or a combination of preparations so far as the preparations are formulated so as to contain the respective active ingredients, Compound (I) or a pharmaceutically acceptable salt thereof and an anticholinergic agent. Preferably, the pharmaceutical composition or the therapeutic agent for overactive bladder has a unit dose form suitable to oral administration, such as tablets or capsule, or has a unit dose form suitable to parenteral administration, such as injections. When preparations are used or administered as a combination of preparations, they may be used or administered together or separately at an interval.

In certain embodiments, the compositions of the invention comprise a polymer matrix that forms a tablet, capsule, or biodegrable implant suitable for oral or parenteral delivery of a therapeutic or diagnostic agent. Such compositions are especially useful where degradation of the capsule or matrix is in response to the local cellular or physiological mileu, e.g., temperature, pH, sites of inflammation and/or necrosis, for example. Thus, in certain embodiments, the compositions of the invention may include a diluent, excipient, disintegrator, lubricant, binder, surfactant, water, physiological saline, vegetable oil solubilizer, isotonizing agent, preservatives, antioxidants etc., in addition to the therapeutic or diagnostic agent. Tablets and capsules, for example, may include excipients, such as lactose, disintegrators, such as starch, lubricants ,such as magnesium stearate, binders, such as hydroxypropyl cellulose, surfactants, such as fatty acid ester, and plasticizer such as glycerin.

The structures disclosed herein may be characterized with respect to mechanical properties, such as tensile strength using an Instron tester, for polymer molecular weight by gel permeation chromatography (GPC), glass transition temperature by differential scanning calorimetry (DSC), and bond structure by infrared (IR) spectroscopy, with respect to toxicology by initial screening tests involving Ames assays and *in vitro* teratogenicity assays, and implantation studies in animals for immunogenicity, inflammation, release, and degradation studies.

In an exemplary embodiment, the processes and compositions disclosed herein may be used *in situ* or *ex vivo* to manufacture a tissue engineered construct to control angiogenesis. The process may be used to fabricate a biomimetic extracellular matrix (bECM) of fibrin incorporating a recombinant human fibroblast growth factor-2 (FGF-2).

In general, bECM with FGF-2 may be used to induce controlled angiogenesis. In particular, a fibrin-based bECM design with or without gradients of FGF-2 targeted for angiogenesis is used in bone tissue engineering. Angiogenesis is a requisite for osteogenesis and successful incorporation of tissue engineered bone grafts. A broad range of biopolymer matrix materials and components targeted for different tissues may be applicable.

There are a several strategies to address angiogenesis in engineered tissue constructs. Most often, a bECM delivery of growth factors (GFs) such as the bone morphogenetic proteins, cells or both, provide structural support, cues, and surfaces for cell attachment. Examples include seeding and culturing bECMs with ECs and other cells, such as stem cells, *in vitro* seeding and culturing structured bECMs, which have networks of channels, with hepatocytes and other cell types *in vitro* seeding ECs and other cells into micromachined branched channels, cultured *in vitro*, and the resulting layers are folded into 3-D structures; seeding bECMs with cells transfected with a recombinant retrovirus encoding VEGF; and incorporation of VEGF-A165 or FGF-2 in bECMs by entrapment, adsorption, microcarriers or immobilized to matrices by covalent bonding. In an exemplary embodiment, a process of forming a scaffold includes incorporating fibrin bECMs with FGF-2. This process may provide a controlled and predictable angiogenic response.

Cells, GFs, and an ECM are fundamental tissue building blocks. Functional roles for each of these building blocks in homeostasis and wound repair guide the tissue engineering designs. Angiogenesis is a reoccurring theme in homeostasis and wound repair. As a consequence of the powerful role angiogenesis has on wound repair, the apparatus, compositions, and methods disclosed herein provide for tissue-engineered therapies. Without angiogenesis, tissues with a volume exceeding a few cubic millimeters cannot survive by diffusion of nutrients and oxygen.

Angiogenesis occurs under specific spatial and temporal control. It has been suggested that temporal release of VEGF and platelet derived growth factor-BB (PDGF-BB) from a bECM effectively enhances neovessel formation. It is believed that VEGF promotes chemoattraction, mitogenesis, and differentiation of endothelial cells and that PDGF enhances smooth muscle cell development for neovessels.

Using the methods and apparatus disclosed herein, a bECM may be constructed that delivers an angiogenic factor, and, thus, fulfills several biological criteria to support wound repair. The angiogenic factor can be spatially localized, protected, and delivered in a controllable and predictable manner by the bECM.

Angiogenic factors, such as VEGF, FGF-2 and PDGF are typically delivered endogenously in soluble forms. Moreover, diffusion and convective flow at the wound implant site which could "wash out" and dilute the factors should be avoided. Increasing the administered doses could mitigate such effects but would be problematic due to potential systemic side-effects. Therefore, factors should be tethered or entrapped within the bECM so that the pharmacokinetics will be sufficiently predictable angiogenesis and subsequent tissue repair.

In addition to the rate and amount of angiogenesis, the quality and topology of the neovascular network are critical. Delivered angiogenic molecules and ECs have been implicated as etiologic agents of vascular pathologies, including hemangiomas and other unusual vascular structures. The bECM/FGF-2 developed in accordance with the methods, compositions, and apparatus disclosed herein provide an organized functional platform for normal vessel formation. Using gradients, pores, and structural forms disclosed herein the FGF-2 can be incorporated throughout the bECM, and the release of FGF-2 can be regulated by, for example, spatial concentration gradients, and/or released during cellular ingrowth and biodegradation. Therefore, neovessel formation is directed and organized.

In an exemplary embodiment, the methods, compositions, and apparatus disclosed herein may be used in bone tissue engineering. Since angiogenesis and osteogenesis are linked, there is a strong correlation between recipient site vascularity and bone graft viability. Recent studies with knockout mice for VEGF underscore the interrelationship between angiogenesis and bone. The initial phase of bone graft healing includes chemotactic and chemokinetic signals (e.g., VEGF, PDGF, FGF-2) directing angiogenesis within the fibrin clot. Moreover, spatial and temporal patterns of GFs required for angiogenesis and osteogenesis also are required to regulate mitogenesis, cell shape, movement differentiation, protein secretion, and apoptosis.

The relatively predictable and organized set of cellular and molecular events during bone regeneration provide a mechanism for creating a controlled spatial gradient of an angiogenic factor in the bECM for bone tissue engineering. For example, when a bone fracture occurs, local blood vessels at the site are disrupted and the wound and immediately surrounding area become vascular, causing localized hypoxia and acidosis. Resident endothelial cells (ECs) respond to the hypoxic and acidotic environment and secrete VEGF and FGF. A localized and spatial concentration gradient of these angiogenic factors is produced throughout the fibrin clot, leading to an organized neovascular response antecedent to osteogenesis. Therefore, a bECM comprising FGF-2 will provide fundamental biologic responses at the wound site.

In various embodiments, a fibrin-based bECM may include two or more angiogenic molecules, including, for example, FGF-2 and PDGF. Such bECMs comprising FGF-2 and PDGF are useful, for example, to regenerate healing of critical-sized defects (CSD). In certain embodiments, a tissue engineered design of a calvarial CSD has a gradient that increases from the bottom to the top of the structure. When such structure is placed into a CSD defect, the gradient encourages migration of cells in an upward direction toward the region having a higher growth factor concentration. The temporal migration of cells could also be controlled using a decreasing porosity gradient from the bottom to the top (e.g., the top is less porous than the bottom). As the cells encounter the higher density/lower porosity area of the scaffold, their migration will be slowed. In certain instances, it may be desirable to have a thick or non-porous layer in one or more areas of the scaffold to prevent cell migration in a certain direction. Such layers act as guides for the formation of tissue along predetermined shapes or axes.

In other embodiments, a tissue engineered design of a calvarial CSD has a gradient of immobilized FGF-2, with concentrations higher in the center of the bECM, gradually decreasing from the center to the periphery to optimize chemoattractant and mitogenic effects that guide controlled neovessel formation. The PDGF at the center of the bECM promotes recruitment of smooth muscle cells to stabilize the neovessels. Thus, temporal control may be achieved through a spatial arrangement of PDGF and FGF-2. Furthermore, spatial variations of fibrin porosity also modulate temporal patterns. The fibrin microstructure determines the torturosity of the 3-D matrix, and manipulation of torturosity affects the bECM mechanical properties, the rate of invading cell migration, proteolysis and growth factor availability. An increase in the fibrin compliance promotes EC differentiation *in vitro*.

The concentration range, direction, and shape for the gradient design may be determined by the biological properties of the wound. CSD studies have reported a significant quantitative difference in osteogenic cell sources for peripheral bone, dural, and subcutaneous cell sources.

Prototypic proangiogenic agents are the VEGF and FGF families. VEGF is a powerful regulator for angiogenesis, and regulating vasodilation, vessel permeabilization, and vascularogeneis. Transforming growth factor-beta (TGF-β), tumor necrosis factor-alpha (TNF-α), PDGFs, and insulin-like growth are additional proangiogenic clans. In an exemplary embodiment, FGF-2 may be used because it is angiogenic and osteogenic.

FGFs, a growing family of over nine members, are mitogenic polypeptides implicated in embryonic development, angiogenesis, regeneration, and wound healing. In various embodiments, acidic and basic FGFs, FGF-1, and FGF-2 are used for therapeutic applications for angiogenesis and bone formation. Moreover, these isoforms instigate a vasodilatory effect, mediated perhaps by an intracellular calcium-nitric oxide loop. This beneficial hemodynamic effect, as well, as the angiogenic capacity of FGFs merit enthusiasm as an angiogenic factor for a tissue engineered therapy. In certain embodiments, FGF-2 may be used for the positive affects of FGF-2 on bone formation and fracture healing.

Microencapsulation of biological factors by degradable polymer microspheres is a popular approach in tissue engineering. Accordingly, in certain exemplary embodiments, microencapsulation may be used to control the release of diffusible molecules over time, producing a transient diffusion gradient to regulate cell response. In other embodiments, FGF-2 may be immobilized with tissue transglutaminase (tTG). Specific binding of the FGF-2 to the bECM (i.e., FGF-2 in the solid-phase) provides maintenance of spatial patterns. Many GPs sustain residence in native ECMs through specific binding patterns. The methods disclosed herein provide bulk fabrication techniques, as well as, methods of spatial patterning. The binding interactions determine GF availability and influence receptor binding, and, therefore, significantly impact cell responses.

In an exemplary embodiment, hydrogels may be used as a precursor to form the structural scaffold. Suitable hydrogels include, for example, fibrin, chitosan, collagen, alginate, poly(N-isopropylacrylamide), and hyaluronate, which can be deposited and gelled with the aid of a second component that modulates cross-linking, pH, ionic concentration, or by photopolymerization, or temperature increase with body contact. In an exemplary embodiment, fibrin may be used. During wound healing, fibrin provides a foundational substratum for wound healing and angiogenesis. Fibrin results when circulating plasma fibrinogen becomes localized in a wound and following a cascade of coagulation events is finally proteolytically cleaved by thrombin and self-assembles into an insoluble fibrin network. Following this gelation event, the interconnecting fibrin fibers become stabilized by interfibri cross-linking catalyzed by transglutaminase Factor XIII (FXIII). From the plasma and platelet degranulation, a range of GFs, cell attachment molecules, proteases, and blood cell components become immobilized and entrapped within the fibrin matrix. Fibrin properties can be controlled for degradation rate and porosity. In addition, a fibrin bECM can be modified with GFs, osteoconductive bioceramics, and plasmids, so as to expand clinical versatility. Fibrin is known to bind with high affinity to FGF-2. Fibrin has demonstrated excellent biocompatibility in clinical applications. In other embodiments, other hydrogels or composites of these hydrogels may be used.

Gelation rate, structure, and material properties of fibrin polymers are determined by relative concentrations of fibrinogen and thrombin, pH, ionic strength, and other biophysical parameters present during fibrin polymerization. For example, fibrinogen concentration directly affects fibrin gel strength, as does cross-linking of the fibrin gel with FXIII, which also protects fibrin from plasmin proteolysis, thus, modulating bECM degradation. The resulting 3-D microstructural properties of the fibrin gel play a decisive role in EC migration, proliferation, and angiomorphogenesis. Typically, FGF-2 and VEGF stimulation of migration is enhanced by more rigid or less porous fibrin gels, whereas capillary morphogenesis is enhanced by less rigid or more porous gels.

In an exemplary embodiment, fibrinogen, thrombin, FGF-2, tissue transglutaminase (tTG), and dilutant buffers are combined to form the biopolymer matrix. In certain embodiments, pH and ionic strength are held constant in 100 mM Tris bluffer, pH 7.0, containing 150 mM NaCl, and 5 mM CaCl. In other embodiments, biopolymer matrices in their simplest form consist of fibrinogen and thrombin. These two components form the base for both a native thrombus formation and commercial fibrin glue. The addition of TGs cross-links fibrin fibrils can be used to stabilize the fibrin polymer, thereby improving mechanical properties. TGs are Ca²⁺-dependent enzymes that catalyze post-translational modification of proteins through the formation of γ-glutamyl-ε-lysine cross-links between polypeptide chains. Plasma FXIII is activated by thrombin and is primarily associated with the covalent cross-linking of fibrin fibrils. A stronger clot is produced with FXIII. tTG is widely distributed in cells and tissues and does not require proteolytic activation. TGs impart fibrolytic resistance by cross-linking α2-antiplasmin to fibrin fibrils and by cross-linking the fibrin α -and/or γ-chains. TGs have a broad range of substrate proteins including fibrinogen/fibrin, fibronectin, plasminogen activator inhibitor-2, α 2-antiplasmin, IGF binding protein-1, osteonectin, β-casein, collagen, laminin, and vitronectin. There is differential substrate specificity between TGs. tTG is preferred because it does not require thrombin activation, is readily available, and because it is a factor in osteogenesis.

Human plasminogen-free fibrinogen and human thrombin may be purchased from Enzyme Systems Research Laboratories (South Bend, Ind.), tTG from Sigma (St Louis, Mo.), and human recombinant FGF-2 from ReproTech, Inc. (Rocky Hill, N.J.). Such materials are also available from GMP facilities and FDA approved sources. In some embodiments fibrinogen is prepared at concentrations in the range of 4-75 mg/ml. Four mg/ml is the concentration of native fibrin clots, and up to 130 mg/ml is used in commercially available fibrin glue formulations, Such as TISSEEL™. Thrombin concentrations between 1 to 50 NIH units/ml can be tested to modify gelation time, fibrin fibrillar diameter and porosity. In some embodiments, biopolymer may comprise of FGF-2 concentrations between 1-12 ng/ml.

Temperature plays an important role in stability of biopolymer components and the rate of fibrin gelation. In some embodiments, biopolymer formation can occur at 23°C. Protein-based compositions may be stored at -70°C or freeze-dried prior to use to maintain viability.

To validate biopolymers incorporating growth factors. Biopolymers can be formed using the methods as known in the art and described herein. For example, for a fibrin matrix incorporating FGF-2, the system can be validated using immunohistochemical staining and SEM and/or fluorescent microscopy. The persistence of FGF-2 can be validated using fluorescence-labeld FGF-2 and ¹²⁵I labled FGF-2 labeling incorporated into the biopolymer matrix.

For fluorescence confocal laser microscopy, fibrinogen biopolymer can be conjugated to Cy5 and mixed with unlabeled fibrinogen (5% vol:vol to unlabelled fibrinogen). Similarly, FGF-2 can be conjugated to Cy3 and mixed with unlabeled FGF-2 (5% vol:vol to unlabelled FGF-2). Subsequent confocal microscopy can be performed using a Zeiss confocal LSM10 microscope equipped with 5 mW Ar 488/514 nm and a 5 mW He/Ne 633 nm lasers. A Zeiss Plan-Neofluar 20x0.5 NA water immersion objective can be used to image sections in 1µm, or better, increments. Images can be processed using Zeiss LSM software.

Persistence of FGF-2, can be measured by immediately fixing or placing the construct in excess phosphate buffered saline, pH 7.4 (PBS), containing 0.02% sodium azide for various times (0, 0.5, 1, 4, 8, 24, 72 hrs) at 23°C using time 0 as the control. For other experiments, radiolabeled ¹²⁵I-FGF-2 can be used.

For determination of FGF-2 biological activity, biopolymers comprising FGF-2 can be placed in 24 well tissue culture plates for ³H-thymidine assay. Human umbilical ECs (HUVECs) can be purchased from Clonetics (BioWhittaker, Inc., Walkersville, Md.) and maintained according to supplier's instructions. Cells can then be grown 70% confluence and seeded onto the biopolymer at 20,000 cells/well in serum-free media. After 48 hr culture, 0.5 µCi ³H -thymidine can be added to the wells. After overnight culture, biopolymers can be trypsinized to dissolve the biopolymer matrix and cells can be washed with PBS. Subsequent ³H-thymidine incorporation into the growing cells can be determined by standard protocols known in the art.

Statistical analyses can be performed using multiple analysis of variance (ANOVA) and Tukey's post-hoc test for multiple comparison analysis, with a significance level of p<0.05.

Since the stiffness of the fibrin matrix decreases with fibrinogen concentration, slumping may occur at lower fibrinogen concentrations. Varying the pH and ionic concentrations are one method of altering mechanical properties while maintaining fibrinogen concentration. Alternatively, lateral support for biopolymers can be provided using plastic rings glued to the surface. Ring dimensions should be equivalent to the biopolymer construct.

The microstructure (porosity, fibril diameter) of structures may be characterized using scanning electron microscopy (SEM) and fluorescence confocal microscopy. Patterns and concentrations of therapeutic factors may be determined by fluorescence microscopy using direct fluorescent labeling and immunofluorescence.

In embodiments where FGF-2 is cross-linked to the biopolymer matrix, tTG cross-linking of FGF-2 can be used. A broad range of substrate proteins for FXIII and tTG have been identified, including fibrinogen/fibrin, fibronectin, plasminogen activator inhibitor-2, α2-antiplasmin, IGF binding protain-1, osteonectin, P-casein, collagen, laminin, and vitronectin. To account for differences in substrate specificity, different TGs or FXIII may be used. Alternatively, FGF-2 may be cross-linked to a dilute solution of fibrinogen prior to formulation

FGF-2 specifically binds fibrinogen via standard reaction using BS³ (a water soluble bis(sulfosuccinimidyl) suberate) from Pierce (Rockford, III.). This cross-linker may be used to immobilize IGF-I to metal surfaces and it is biocompatible. Should bECMs require higher FGF-2 concentrations, an oligoglutamine moiety maybe coupled onto FGF-2 via BS³. Furthermore, the exact nature of the binding region can be tailored to maximize its reactivity; for example, chain length and composition can be altered. Various oligopeptides can be synthesized which are rich in both glutamine and the facilitating amino acids. Cross-linking heparin to fibrinogen or fusion peptides using TG substrate sequences may be utilized. Engineered peptides, fusion proteins, and other such molecules may also be used to promote attachment of therapeutic agents, such as drugs, growth factors, etc., to matrix components either directly as a fusion protein (i.e., a growth factor with a TG substrate component without a protease cleavage site) or an engineered peptide (i.e., such as a heparin binding domain sequence with a TG substrate sequence that may be used to immobilize heparin to serve as a generic binder for proteins containing heparin binding domains).

Following the fabrication, structures can be assayed *in vitro* or via the CAM model by immediately placing the construct in serum-free media containing 50 µg/ml BSA (Insulin RIA grade, Sigma, St. Louis, Mo.) and 1 µg/ml aprotinin at 23°C. These samples can be incubated with media changes so as to remove unbound growth factors such as FGF-2. Holding the temperature at 23°C and the addition of a protease inhibitor, such as aprotinin, helps stabilize the biopolymer structure.

The effectiveness of tissue-engineered constructs is often evaluated in the art *in vitro* prior to assessment *in vivo*. *In vitro* results may not directly translate to *in vivo* results. However, compared to *in vivo* experimentation, *in vitro* experimentation is associated with reduced expense, increased experimental turnover rates, and more selective control of associated variables. These considerations support *in vitro* experimentation in the tissue engineering design process.

A scientifically accepted alternative to animal models is the chorioallantoic membrane (CAM) model. The CAM is a vascular extraembryonic membrane located between the embryo and the eggshell of developing chicken egg. Angiogenesis and the CAM have become an important *in vivo* biological assay to screen therapies for wound repair and blood vessel development. CAM will be used to assess angiogenesis in response to the fibrin /FGF-2 designs. To ensure fixation of the construct to the CAM, a cutting device is used to make a 17 mm diameter hole in the horizontal center of eggs. An optically clear plastic insert (15 mm OD x10 mm ID) can be used to create windows for focused treatment application and subsequent *in situ* assessment. Placing sample constructs of smaller size than the insert provides a border region surrounding the construct within the viewing window allows *in situ* observation of the directed vascular ingrowth.

The CAM assay consists of incubating fertilized White Leghorn eggs at 37.8°C in 60% relative humidity. On day three, eggs are opened using a mid-horizontal orientation in the cutting device. Removal of 0.5 ml of albumin from the large end of the egg prior to cutting drops the embryo from the cutting site, protecting it from vibration and surgical trauma. Porous medical tape placed over the hole minimizes evaporative loss and prevents contamination. On day 8, window inserts are placed through the hole and rest directly on the CAM.
A construct can be placed in the CAM on day 10. *In situ* imagining allows a digitally recording for image processing to be made from days one through eight. The construct placed into the CAM inserts can be recovered at this time and prepared for histological analyses of angiogenesis. Embryos, membranes, and the construct can be fixed *in ovo* in Bouin's fluid. The window/CAM area can then be removed, dehydrated, and embedded in paraffin. Serial sections can be made in a plane parallel to the CAM surface. Sections can be stained using 0.5% toluidene blue. Angiogenesis can be evaluated with a Zeiss Axiophot microscope interfaced with an image analysis system using Zeiss imaging software. Quantitative data can be analyzed by multiple analysis of variance (ANOVA) and Tukey's post-hoc test for multiple comparison analysis at a level of significance of p<0.05.

The following examples are intended to further illustrate the invention, without any intent for the invention to be limited to the specific embodiments described therein.

### Examples

### Example 1: Fibrin processing.

A fibrinogen formation for a 1 mL batch was prepared by mixing a solution comprising 250 µL of 40 mg/ml Aventis or diaPharma fibrinogen (commercially available from ZBL Behring, King of Prussia, PA; or diaPharma, West Chester, OH respectively), 230 µL of sterile water, 300 µL of 1M NaCl (Sigma, St. Louis, MO), 200 µL of 200 mM bicine, pH 8.0 (Sigma), 10 µL of 100 U/mL Factor XIII (ZBL Behring, King of Prussia, PA), and 10 µL of 10 mg/mL bone morphogenetic protein 2 (BMP-2) (R&D Systems, Minneapolis, MN). The fibrinogen formulation was subsequently incubated for 30 minutes at 37°C to allow for the growth factors to associate with the fibrinogen in solution.

A thrombin formulation for the 1 mL batch was prepared by mixing a separate solution comprising 100 µL of 100 U/mL thrombin (Enzyme Research Labs, South Bend, IN) and 10 µL of 500 mM CaCl₂ (Sigma).

The fibrin gels were prepared by adding 15 µL of the thrombin solution to a 1.5 mL microfuge tube, adding 350 µL of the fibrinogen solution to the microfuge tube, and immediately transferring the mixed solution to a gel mold. The gel solution was then allowed to set for 20-30 minutes in the gel mold to allow for complete gelation. After 20-30 minutes when the gel solution has completely set, the molds were disassembled and the resulting fibrin films removed.

The gel molds were constructed of four components: bottom pieces, 0.75mm top pieces, 1.5mm top pieces, and standard binder clips. "Bottom pieces" comprise 2.5cm x 2.5cm x 0.63 em TEFLON™ segments; "0.75mm top pieces" comprise 2.5cm x 2.5cm x 0.075cm TEFLON™ square segments with 12.5mm x 12.5mm square segments cut out and removed from the canter; and "1.5mm top pieces comprise 2.5cm x 2.5cm x 0.15cm TEFLON™ square segments with 12.5mm x 12.5mm square segments cut out and removed from the center. The gel molds were assembled by placing one 1.5 mm top piece on top of a bottom piece, and placing one 0.75 mm top piece on top of the one 1.5 mm top piece with the edges of the segments aligned. The segments were clamped together with the binder clips. The gel mold assemblies were placed inside a 100 mm Petri dish with a wet tissue (commercially available from Kimberly-Clark, Roswell, GA) and with a closed lid to create a humidified chamber.

It was found that fibrin gel structure (i.e., fibrin fibril formation) is highly dependent on the pH, NaCl concentration, CaCl₂ concentration, and fibrinogen concentration. Therefore, modification of these concentrations and volumes in the initial gel formulation imparted different properties to the final film. Accordingly, on skilled in the art could modify the initial gel formulation to prepare films with structures and properties that are suitable for their particular applications while still being within the scope and spirit of the present invention.

### Example 2: Vacuum Dehydration of Fibrin Gel.

Upon removal from the gel molds, the fully-hydrated fibrin gels were lyophilized in order to remove water and reduce the thickness of the fibrin gel films from 2.25 mm to approximately 100 µm. Lyophilization was performed in a gel dryer (commercially available from BioRad, Hercules, CA). The lid of the gel dryer was opened and the silicon rubber gasket was peeled back. Spectrapor 1 dialysis tubing (6-8k MWCO, Spectrum Laboratories, Rancho Dominguez, CA) was soaked in PBS and cut along one side to allow the tubing to be opened up into a sheet. The opened tubing had 3cm x 3cm pieces cut into it. The resulting dialysis "sheets" were placed onto the gel dryer. The fibrin gel was placed in the center of the dialysis sheet. A 2.5cm x 2.5cm x 3cm TEFLON™ segment with a 20mm x 20mm square cut out of the center was placed around the gel. A 2.5cm X 2.5cm x 0.075cm Teflon segment was placed on top of the 2.5cm x 2.5cm x 3cm TEFLON™segment. The silicon rubber gasket was carefully repositioned and the gel dryer lid closed. A lyophilizer unit (Labconco, Kansas City, MO) was activated and allowed to reach reduced pressure and temperature. The lyophilizer unit was then connected to the gel dryer. The gel was dried into a film using one of two alternative methods: (1) the gel dryer was run at 50°C for 2-2.5 hours, or (2) the gel dryer was run at room temperature for 8-10 hours.

The dialysis sheet with the attached fibrin film was carefully removed and immediately placed in a 6 wall microwell plate containing 3 mL PBS with 10 units/mL penicillin G sodium, 10 µg/mL streptomycin sulfate solution (Invitrogen, Carlsbad, CA), and 10 µM D-Phe-Pro-Arg-chloromethylketone (PPACK) thrombin inhibitor (BIOMOL International, Plymouth Meeting, MA). Lyophilized film was allowed to sit for 24 hours at 4°C. Every 24 hours the buffer was replaced with fresh, sterile PBS, but only for a total of 2 buffer exchanges maximum. The films were stored at 4°C until further processing or analysis was performed.

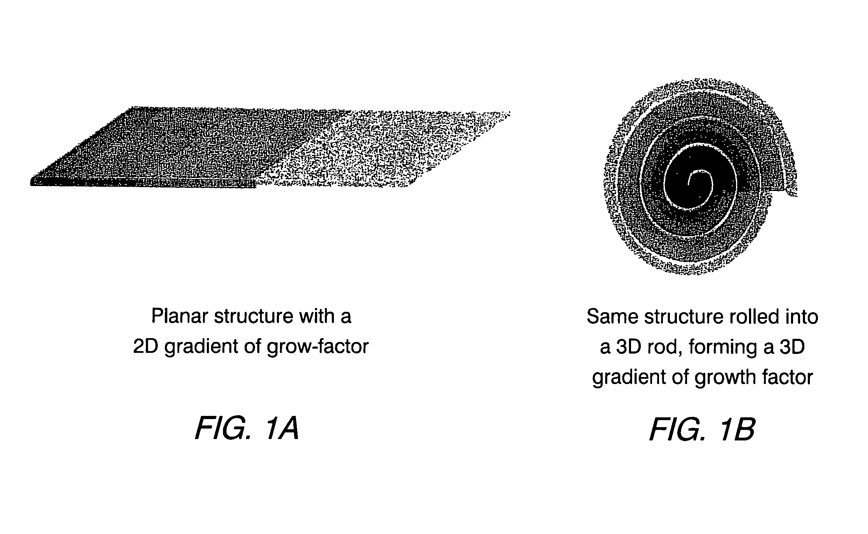

The lyophilization process produced films that were transparent to translucent in appearance and possessed elastomeric properties as demonstrated in FIG. 1B.

### Example 3: Osmotic Dehydration of Fibrin Gel.

An alternative method to that disclosed in Example 2 was also used to process the fibrin gels upon gelation and removal from the gel molds. In this method, an osmotic dehydration process was used (based upon the method of Müller and Ferry, US Patent No. 4,548,736). Fibrin gels were prepared the same as in the method of Example 1. The fibrin gels were placed on a coverslip inside a 60 mm Petri dish. Approximately 500 µL of a 35% high molecular weight polyvinyl alcohol solution was added to the inside of an approximately 3inch segment of SPECTRAPOR-1™ (6-8,000 MWCO) dialysis membrane tubing soaked in PBS. The ends of the tubing were clamped off. The tubing was placed on top of the gel making sure the polyvinyl alcohol solution rested directly on top of, and in complete contact with, the entire gel to ensure that water evenly diffused from the gel and entered into the tubing along the osmotic gradient. A lid was placed on the Petri dish and it was incubated at room temperature for 24 hours. The resulting fibrin film was removed from the dish and soaked in a 50% glycerol solution for 24 hours at room temperature followed by storage at 4°C in a PBS solution.

This method involved longer processing time and occasional inhomogeneities in film thickness due to occasional lack of even osmosis. However, this method was advantageous with respect to the relatively mild treatment of the film and because the film never completely dried out.

The fibrin films fabricated according to the method described in Example 1 and either of Examples 2 or 3 were transparent to translucent in appearance and possessed elastomeric properties.

### Example 4: Fibrin film biocompatibility assay.

Fibrin films fabricated according to the method described in Examples 1 and 2, and Examples 1 and 3, were placed on 12 day old chick embryos and biocompatibility was tested using the chick chorioallantoic membrane (CAM) assay. The tested films did not exhibit any objective signs of incompatibility. CAM blood vessels underneath the fibrin film were visualized by intravital injection of fluorescent quantum dots (QDs). Two days post-placement of the film, the embryo was injected with 655 nm emitting QDs and blood vessel fluorescence was imaged on a M2BIO stereoscope using a 1.6X objective (1X zoom), Retiga Exi CCD camera, and a (Ex:Em) 450spuv:655/20 filter set. The films induced no observable ill effects on the underlying blood vessels.

### Example 5: Fibrin film biocompatibility assay.

Fibrin gels were prepared with 10 mg/mL diaPharma fibrinogen (commercially available from diaPharma, West Chester, OH), 300 mM NaCl, 40 mM bicine pH 8.0, and further comprising 10 ng/mL fibroblast growth factor 2 (FGF-2) and processed into films as described in Example 1, and either of Examples 2 or 3. These films were placed on 10-day old chick CAMs. Twenty-four hours later bleeding could be seen around blood vessels under the films. Without being bound by theory, the observed bleeding was most likely due to the very high concentration of FGF-2 as a result of the approximately 20-fold decrease in gel thickness observed upon lyophilization. Final FGF-2 concentrations in the film were estimated to be approximately 200 ng/mL. This is the same amount of vascular endothelial growth factor (VEGF₁₆₅) within fibrin disks that was reported to cause CAM vessel bleeding under the fibrin disk (Woog C. et al., Thromb. Haemost. 89: 573-582 (2003)). Lower concentrations of FGF-2 should not result in bleeding.

### Example 6:2-D inkjet tissue printing system and BMP-2 layering on fibrin films.

Fibrin films were prepared with 11 mg/mL diaPharma fibrinogen, 1 U/mL Factor XIII, 300 mM NaCl, and 40 mM bicine, pH 8.0. Using a 2D inkjet tissue printing system, 4 square patterns of Cy3-labeled BMP-2 was printed on top of the film in a grid pattern as shown in FIG. 4. The film was submerged in PBS and stored at 4°C. The media was changed daily and the film imaged for Cy3 fluorescence over the course of one week using a M2BIO stereomicroscope with a 1.6X objective (1X zoom), a Retiga Exi CCD camera, and a standard Cy3 filter set. The film exhibited little loss of fluorescence over the 7 days of the evaluation indicating stable BMP-2 incorporation into the film.

### Example 7: Scanning electron microscopy (SEM) of fibrin films.

Fibrin gels were prepared with 11 mg/mL diaPharma fibrinogen, 1 U/mL Factor XIII, 300 mM NaCl, and 40 mM bicine, pH 8.0) and processed into films. These films were cut into quarters and processed for scanning electron microscopy (SEM). Specimens were fixed in I % glutaraldehyde and 3% paraformaldehyde in PBS overnight. Subsequently, after 3 washes of PBS, the specimens were fixed for one hour in 1% OsO₄ buffered with PBS. The OsO₄ was removed with three five-minute washes of ddH₂O, followed by dehydration in an ascending series of ethanol (50%, 70%, 80%, 94%, and 3 changes of 100%) The samples were held in each ethanol wash for 10 minutes and then held in 100% ethanol overnight. The specimens were dried in a Pelco CPD2 critical point dryer (Clovis, CA) using CO₂ at 1200 psi and 42°C. Dried specimens were attached to SEM stubs using double stick tape and coated with gold using a Pelco SC-6 sputter coater. Specimens were examined using a Hitachi 2460N Scanning Electron Microscope (Pleasanton, CA). Digital images were obtained using Quartz PCI Image software (Vancouver, BC, Canada).

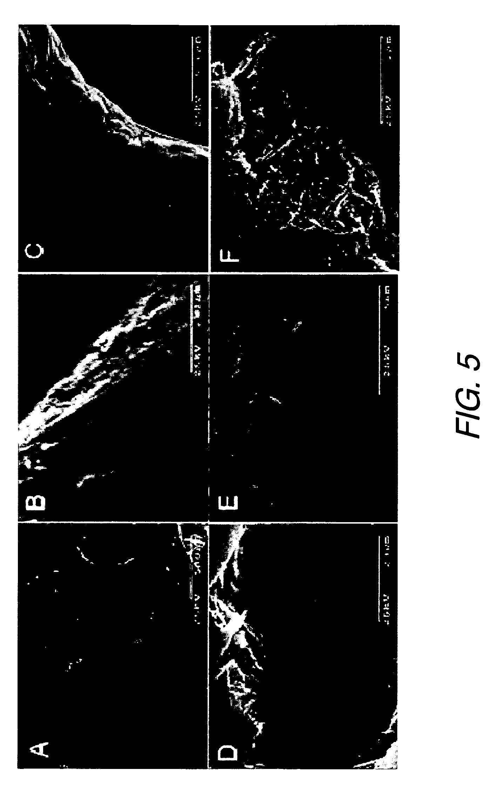

FIG. 5 shows SEM images of the film surface and cross-section. The surface views show a somewhat smooth surface with a dense fiber-like packing (FIG. 5B). Some surface cracks and wrinkles are induced by the fixation and critical point drying steps (FIG. 5A, 5C). The cross-sectional views confirm this. Fibrin fibrils were visible, indicating that film processing does not destroy native fibrin structure (FIG. 5D-F). The high density of fibrils was a result of the 20-fold reduction of fibrin hydrogel thickness as a result of the lyophilization or osmotic dehydration processing of Examples 2 or 3 respectively.

### Example 8: Transmission electron microscopy (TEM) of fibrin films.

Fibrin gels were prepared with 11 mg/mL diaPharma fibrinogen, 1 U/mL Factor XIII, 300 mM NaCl, and 40 mM bicine, pH 8.0) and processed into films. These films were cut into quarters and processed for transmission electron microscopy (TEM). Specimens were fixed in 1% glutaraldehyde and 3% paraformaldehyde in PBS overnight. After 3 washes of PBS, the specimens were fixed for one hour in 1% OsO₄ buffered with PBS. After osmium fixation, sections were washed with three changes of ddH₂O and dehydrated with an ascending series of ethanol (50%, 70%, 80%, 90% and 100%). Propylene oxide was used as a transitional solvent, and the specimens infiltrated with LR White (London Resin Company, Reading, Berkshire, England). The LR White was polymerized at 60°C for 48 hours. Thin (100nm) sections were cut using a diamond knife on a Reichert-Jung Ultracut E ultramicrotome (Leica, Wetzlar, Germany), placed on copper grids, and stained with uranyl acetate, and lead citrate, The grids were viewed on a Hitachi H-7100 transmission electron microscope (Pleasanton, CA) operating at 50 kV. Digital images were obtained using an AMT Advantage 10 CCD Camera System (Advanced Microscopy Techniques Corporation, Danvers, MA) and NIH Image software (Bethesda, MD).

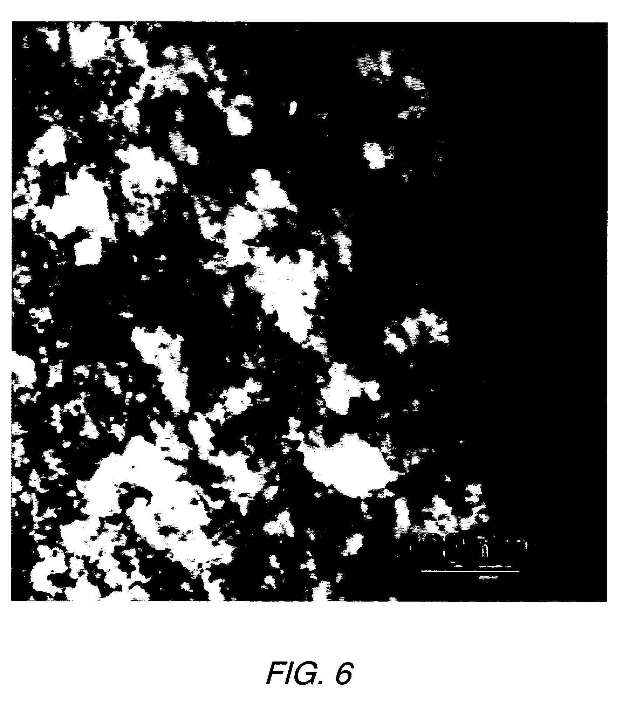

FIG. 6 shows a TEM micrograph of the fibrin film. The fibrin appears random and homogeneous, Pore size is very small and in the nanometer range, which is expected as a result of the high fibrin density within the film.

### Example 9: Fibrin film formulation further comprising tricalcium phosphate.

Fibrin gel films were prepared by the method described in Examples 1 and 2, further comprising the addition of tricalcium phosphate (0.4 mg of TCP powder per 400 uL of fibrinogen solution). The films were qualitatively evaluated for handling properties and found to handle well. [Inventors: please describe the properties that were observed or tested] Thin films comprising TCP have potential for bone tissue engineering applications.

### Example 10:MD-63 Osteosarcoma cell seeding on fibrin films.

Fibrin gel films were prepared with 11 mg/mL diaPharma fibrinogen, 1 U/mL Factor XIII, 300 mM NaCl, and 40 mM bicine, pH 8.0 and processed to films by the method described in Example 2. MG-63 osteosarcoma cells in modified Eagle's medium F-12 (MFM F-12) supplemented with serum did not adhere or spread out on these films. Cells were bound to the films but stayed rounded. Without being bound by theory, it was believed that thrombin remaining in the gel prior to dehydration was concentrated and actually inhibited cell attachment through proteolytic activity once films were placed under *in vitro* conditions.

### Example 11: Extracellular matrix incorporation into fibrin films.

Gels were prepared with 11 mg/mL diaPharma fibrinogen, I U/mL Factor XIII, 300 mM NaCl, and 40 mM bicino, pH 8.0 according to the method described in Examples 1 and 2, and further comprising lyophilized and powdered extracellular matrix (ECM) preparations. See, e.g., Gilbert TW, et al, Biomaterials 26:1431.5 (2005). Gels containing liver-derived ECM powder or urinary bladder ECM (15-20 µL of powder per 400 µL of fibrinogen solution) were successfully processed into films. MG-63 osteosarcoma cells did not attach to these films.

### Example 12: Film treatments and cell seeding.

Gels were prepared with 11 mg/mL diaPharma fibrinogen, 1 U/mL Factor XIII, 300 mM NaCl, and 40 mM bicine, pH 8.0 and processed into films according to the method described in Examples 1 and 2. Films were subsequently treated in one of the following ways: (1) 26 µM PPACK thrombin inhibitor or (2) 20% formamide solution containing 26 µM PPACK. These films were then submerged in PBS or submerged in 1 mg/mL fibrinogen followed by 1 U/mL thrombin to form an additional fibrin layer on the film surface. MG-63 osteosarcoma cells were placed on top of these films in 6 well microplates containing MEM-F12 media supplemented with serum. The results were as follows:
1. PPACK only - Very few cells spread out on the film. Most were attached but still rounded.
2. Farmemide/PPACK - Majority of the seeded cells attached and spread out on the films.
3. PPACK and fbrinogen/thrombin - Majority of the seeded cells attached and spread out on the films.
4. Formamide/PPACK and fibrinogen/thrombin - Majority of the seeded cells attached and spread out on the films.

### Example 13: Fibrinogen source testing.

Fibrin gels were prepared with 10 mg/mL Aventis fibrinogen, 1 U/mL Factor XIII, 300 mM NaCl, 40 mM bicine, pH 8.0 and processed into films according to the method described in Examples 1 and 2. Films were then post-treated with either PBS alone or PBS containing 23µM PPACK, 10 units/mL penicillin G sodium, and 10 µg/mL streptomycin. Films were further post-treated in either PBS or PBS containing 1 mg/mL fibrinogen followed by 1 U/mL thrombin. MG-63 cells were placed on films in 6 well microplates containing MEM-F12 supplemented with sarum and antibiotic. Cells attached to, and spread on, both sets of films. Due to these results, combined with those of Examples 10 and 12, Aventis fibrinogen was determined to be the better source of fibrinogen and formamide was determined to be an optional post-treatment for these experiments.

### Example 14: Cell differentiation on seeded fibrin films.

Fibrin gels were prepared with 10 mg/mL Aventis fibrinogen 1 U/mL Factor XIII, 300 mM NaCl, 40 mM bicine, pH 8.0, and 2.5 ng/mL BMP-2. Gels were processed into films according to the method described in Examples 1 and 2. The final BMP-2 concentration was estimated at approximately 50 ng/mL. Films were also made without BMP-2. The films were placed in 6 well microplates. All wells contained 3 mL Dulbecco's modified Eagle's medium (DMBM) supplemented with serum and antibiotic and aprotinin at 1 µg/mL. The wells were occupied as follows:
Well 1 (negative control): C2C12 mouse myoblast cells only
Well 2 (positive control): C2C12 mouse myoblast cells with 50 ng/mL BMP-2 added to the media
Well 3: C2C12 mouse myoblast cells on the fibrin film not containing BMP-2
Well 4: C2C12 mouse myoblast cells on the fibrin film containing BMP-2

Three days later, alkaline phosphatase (ALP) activity was assessed by a colorimetric staining kit (Sigma) following the manufacturer's protocol. Positive ALP activity resulted in cells stained blue. Cells in wells 2 and 4 were stained blue whereas cells in wells 1 and 3 exhibited very little to no staining. These results indicate that BMP-2 retained activity after film processing and that films comprising BMP-2 support differentiation of C2C12 mouse myoblast cells toward an osteoblast lineage as measured by alkaline phosphatase activity.

### Example 15: Protein based rubbery to hard plastic formulations.

Gelatin, fibrin, fibrinogen and extracellular matrix plastics were fabricated in the form of approximately 0.5cm height x 1.3 cm diameter disks (hereinafter "pellets") using a commercially available press apparatus. In some experiments, the pellets were subsequently lyophilized under vacuum. The gelatin plastics comprised powdered gelatin, glycerin plasticizer, and in some experiments further comprised FGF-2, BMP-2, quantum dots, NaCl, polylactic acid, hydroxyapatite, ammonium acetate, ammonium bicarbonate, ammonium carbonate, and/or pyridinium TFA. The fibrin plastics comprised polymerized fibrin (ground to form a powder), glycerin plasticizer, and in some experiments further comprised FGF-2, BMP-2, andquantum dots. The fibrinogen plastics comprised powdered fibrinogen, glycerin plasticizer, and in some experiments further comprised water, FGF-2, BMP-2, quantum dots, NaCl, polylactic acid, hydroxyapatite, water, or ammonium acetate. The ECM plastics comprised powdered urinary bladder (UB) derived ECM, and in some experiments further comprised glycerin plasticizer, FGF-2, BMP-2, and quantum dots, as well as water in further experiments. The specific Simulations were formed into homogeneous pellets that held together well and were used for additional post-processing and testing.

### Example 16: Gelatin plastic with glycerin as plasticizer - low heat compression pellet processing.

Gelatin plastic pellets were prepared by compaction with glycerin as a plasticizer additive in concentrations of 0%, 12.5%, 19%, 21 %, 2S%, 36%, 40% and 50%. Percentages at about 40% provided particularly useful mechanical properties. The plastic pellets were formed by grinding solidified gelatin into a powder with particle sizes less than or equal to 150 microns (powders passing a 100 mesh standard sieve). A sample of the ground gelatin powder was mixed with glycerin plasticizer, and optionally with the substances described in Example 15 in quantities appropriate to achieve desired concentrations in the resulting plastic. In all preparations, the gelatin powder and any additional components were mixed with glycerin in 50 mL conical plastic centrifuge tubes and extensively manually mixed for 5-10 minutes three times to ensure proper homogonization of the mixture, and left to incubate for at least 2 hours. The homogeneous mixture was then loaded into a standard pellet press. The press mold was twice coated with lecithin mold release agent prior to mixture loading in order to lubricate the inner surface to ensure easy removal of the pressed pellet from the mold without undesirable sticking. The press mold was loaded approximately ¾ full with the glycerin-wetted gelatin powder mixture. The press was then set to a temperature between 50°C and 125°C and a pressure ranging from 5000 lbs to 7000 lbs and the press operated for 20 to 60 minutes, where the specific operating parameters were set based on the desired properties of the resulting plastic. The press operating parameter profiles were over a 20 and a 60 minute operation, respectively, for a typical pellet formation. The mold pressure maximized and held steady at approximately 7000 lbs of pressure for approximately 20 minutes, and the mold temperature generally decreased from an initial value of approximately 80°C until reaching a final steady value of approximately 27°C (room temperature) at approximately 40 minutes into the 60 minute process. After the 60 minute pellet formation process, the resulting plastic pellet was removed from the mold, trimmed of excess plastic, and stored dry at 4 °C. The gelatin plastic formulations were formed into homogeneous pellets that held together well and were used for additional post-processing and testing. Hardness of the compacted plastic was a function of the amount of plasticizer added to milled powders prior to compaction. A more rubbery plastic resulted with increasing percent plasticizer.

### Example 17: Sodium chloride as a porogen in gelatin plastics.

Solid sodium chloride (NaCl) was evaluated as a porogen in gelatin plastics prepared as described in Example 16. The solid NaCl crystals were added to the gelatin powders prior to compaction processing or introduced into the press molds either on either the top or the bottom of the gelatin pellet and physically pressed into the plastic. The embedded NaCl was removed by placing the pressed pellets in water and allowing the solid NaCl to dissolve into solution and leach out of the plastic leaving open pores. Neither the top pressed nor bottom pressed NaCl embedded porogen yielded even pores. Plastics formed with pro-mixed NaCl did not exhibit open pored structure.

### Example 18: NaCl as a porogen with gelatin plastic cross-linking.

Solid NaCl was introduced into gelatin plastic as described in Example 17. The pressed disks were simultaneously cross-linked and NaCl-leachad with either a 2.5% or a 5% glutaraldehyde solution. The gelatin disks exhibited pronounced swelling in the center of the pellets, cracking across the circular faces of the pellets, and pronounced brown discoloration from the GA reaction. SEM imaging continued that not all of the solid NaCl leached out of the cross-linked matrix.

### Example 19: Polylactic acid (PLA) as a porogen in gelatin plastics.

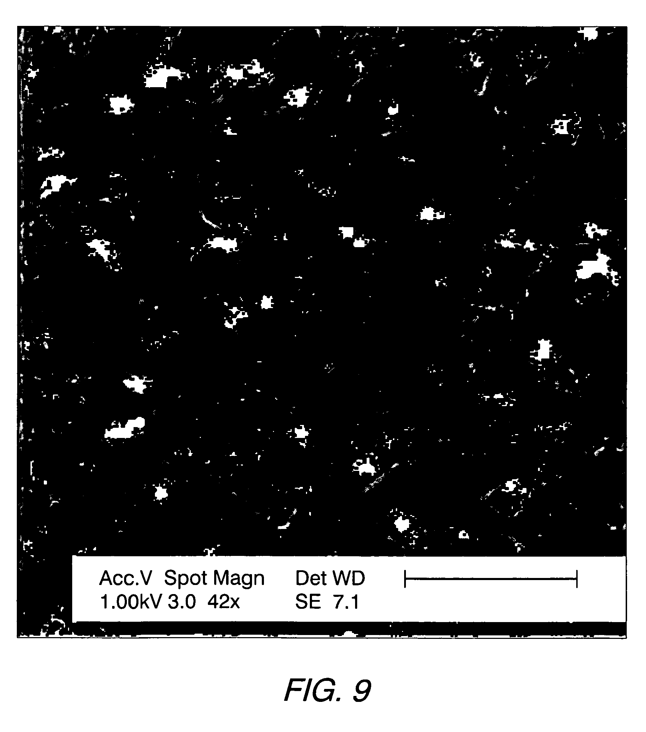

Milled polylactic acid (PLA) (180-150 micron diameter particles) was introduced into the gelatin plastic formulation and the material processed as described in Example 16. The plastic pellets were submerged in chloroform (CRCl₃) and the solid PLA trapped within the gelatin matrix allowed to dissolve into solution and leach from the material leaving open pores. FIG. 9 is a micrograph illustrating the formation of the pores in the gelatin matrix due to the leaching of the physically trapped PLA.

### Example 20: Gelatin plastic extrusion processing.

Gelatin (19%vol:vol) plastic was prepared and processed as described in Example 16. The clear plastic pellets were further processed by extrusion. The gelatin plastic was extruded at a series of temperatures, ranging from 60°C to 117°C (i.e., 60°C, 70°C, 78°C, 80 °C, 90°C, 100°C, 110°C, and 117°C), Notwithstanding the prior plastic pressing steps, the extruded gelatin plastic formed bubbles and expanded after extrusion. Cold rolling of the extruded gelatin plastic resulted in cracking.

### Example 21: Hydroxyapatite incorporation into gelatin plastics.

Hydroxyapatite (HA) was introduced into the gelatin plastic formulation and the material processed as described in Example 16. The resulting gelatin disks comprised 5% HA homogenously dispersed throughout the matrix.

### Example 22: Machinability of gelatin plastics.

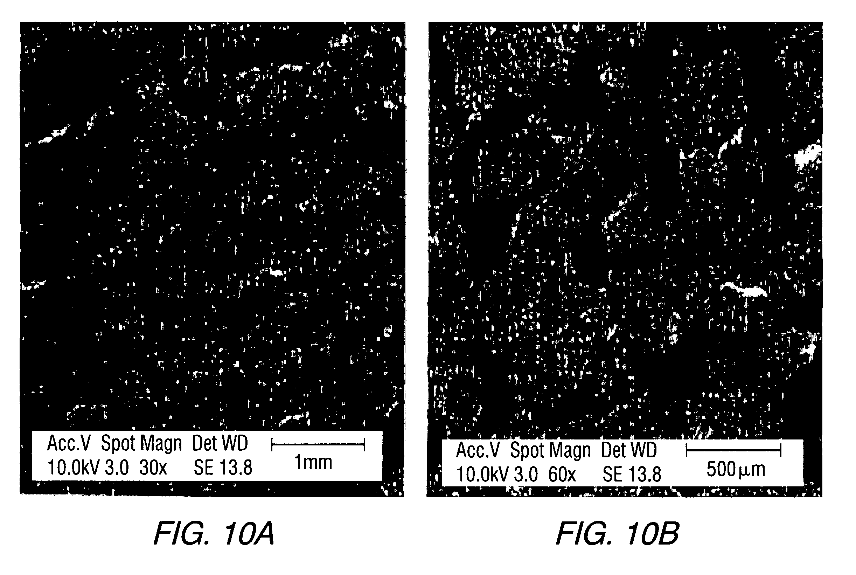

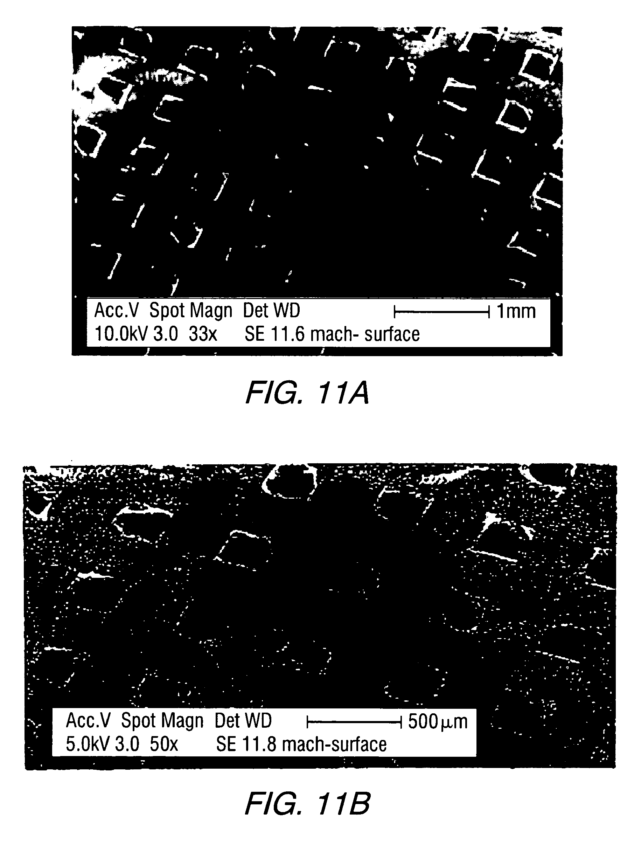

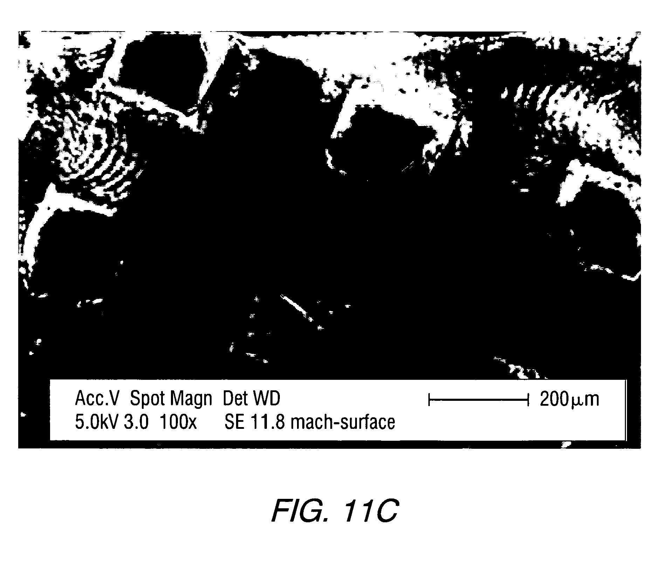

The gelatin plastics prepared according to the methods described in Example 16 possess demonstrated machinability with varying concentrations of glycerin plasticizer. The relative hardness of the plastic determined its compatibility to machining techniques. The plastics generally exhibit excellent machinability as indicated by the precise formation of approximately 215 micron squares after machining, FIG. 10 and FIG. 11. The softer plastics that resulted from higher concentrations of glycerin plasticizer formed qualitatively rougher machined surfaces, FIG. 10, whereas the harder plastics that resulted from lower concentrations of glycerin plasticizer formed more clean machined surfaces, FIG.11. Example 23: Pressing of gelatin plastic into sheets.

The gelatin plastics prepared according to the method described in Example 16 possess excellent formability in terms of pressing thin sheets. The sheets formed from the gelatin plastic are thin, partially transparent, flexible, and tough. Generally, the resulting plastic shape was a function of the specific mold in which it was formed. The sheet comprised gelatin powder and 23% (vol:vol) glycerin grassed with a specially constructed rectangular shape mold.

### Example 24: Fibrin and fibrinogen pressed-plastics with glycerin plasticizers.

The fibrin formulations described in Example 1 but using porcine fibrin were powdered and further modified by addition of glycerin and/or water plasticizers and processed into plastic disks as described in Example 16 (i.e, fibrinogen compacted at temperatures of 80°C, 81°C, and 121°C; and fibrin compacted at temperatures of 79°C and 101°C). The non polymerized fibrinogen was mixed with glycerin and water and processed into plastic disks as described in Example 16. The use of 12.5% (vol:vol) glycerin plasticizer with water and non-polymerized fibrinogen yielded soft pliable plastics at all tested temperatures; however, fibrinogen-based materials pyrolyzed when heated to 121°C, in contrast to gelatin-based materials, which tended to melt when approaching that temperature. Fibrin plastics made using glycerin alone at 21 weight percent were hard and translucent

### Example 25: ECM-based pressed-plastic fabrication.

Powdered urinary bladder extracellular matrix (UB ECM) was pressed into plastic pellets as described in Example 16. Specifically, a first pellet was made without glycerin and processed at 102°C. A second pellet was made with 21% glycerin and processed at 77°C. A third pellet was made with 38% glycerin and processed at 100°C. Example 26: Growth factor inclusion and distribution visualization with quantum dots.

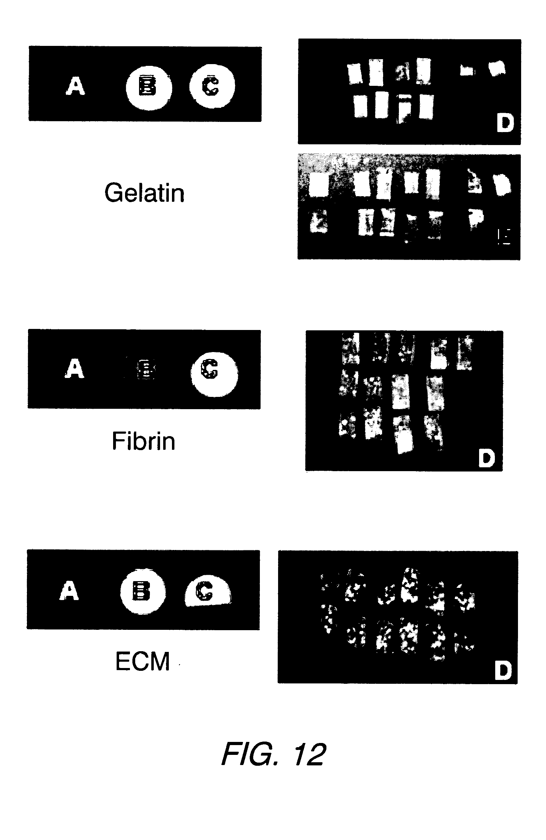

The use of the lower temperature (60°C) for compaction pressing of the protein powders into plastics made possible the inclusion of growth factors and similar biologies into the plastic during the initial compaction process. Experiments were run with inclusion of BMP-2 and PGF-2. Both BMP-2 and quantum dots (800 nm emission, 8 µM) were premixed with glycerin prior to its addition to the protein powder. As a control, plastics were formed with only protein powder and glycerin plasticizer. Additional plastics were formed with quantum dots and BMP-2 (i.e., gelatin with 25% glycerol processed at 77°C, or fibrin with 36% glycerol processed at 78°C, or B-ECM with 36% glycerol processed at 78°C). Gelatin comprising 25% glycerol was pressed at 66°C, fibrin, comprising 36% glycerol was pressed at 66°C, and ECM comprising 43% glycerol and was pressed at 76°C.
The quantum dot addition allowed for visualization of the distribution of the quantum dots in the plastic and was used to indicate the distribution of growth factor within the plastic. FIG. 12 presents representative samples of plastics comprising quantum dots viewed under fluorescence. Samples labeled A were control groups not containing quantum dots. Samples labeled B and C contained quantum dots and BMP-2, and samples labeled D were cross-sectioned pellet sheets from samples C from indicated plastic. Samples labeled E is visual micrographs of cut films of gelatin plastic. Fluorescence was observed throughout the entire plastic sample for all formulations and processing parameters investigated, which indicated homogenous distribution of the quantum dots and BMP-2.

### Example 27: Quantum dot incorporation into ECM plastic to monitor degradation in vivo.

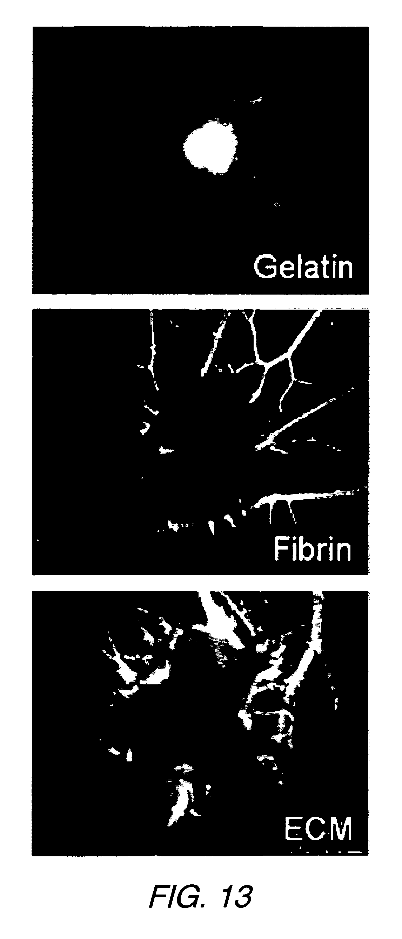

In order to monitor pellet degradation via non-invasive fluorescence, fibrin-based plastics were prepared according to the method described in Example 16 and further comprised carboxyl coated (8.0 µM) 800nm quantum dots. The addition of the quantum dots permit the noninvasive monitoring of the *in vivo* degradation of the plastic, for example through the skin when implanted in a patient. The degradation of the plastic would be evaluated through the loss of fluorescence at the implant site. The *in vitro* results indicted retention of biological activity of BMP-2 incorporated into the plastics as demonstrated by the induction of extensive vascularization in a chick CAM model performed analogously to the method described in Example 4. FIG. 13 presents representative fluorescence images of the vascularization on each of the three types of protein-based plastics.

### Example 28: Ammonium compounds as porogens.

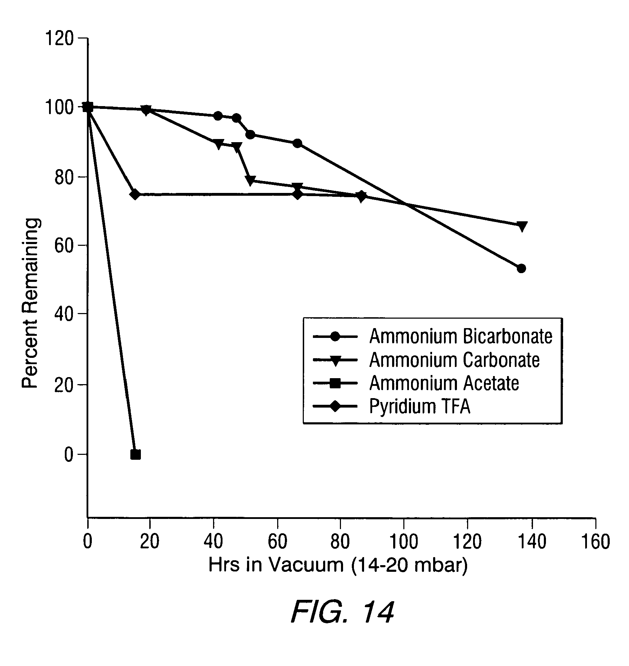

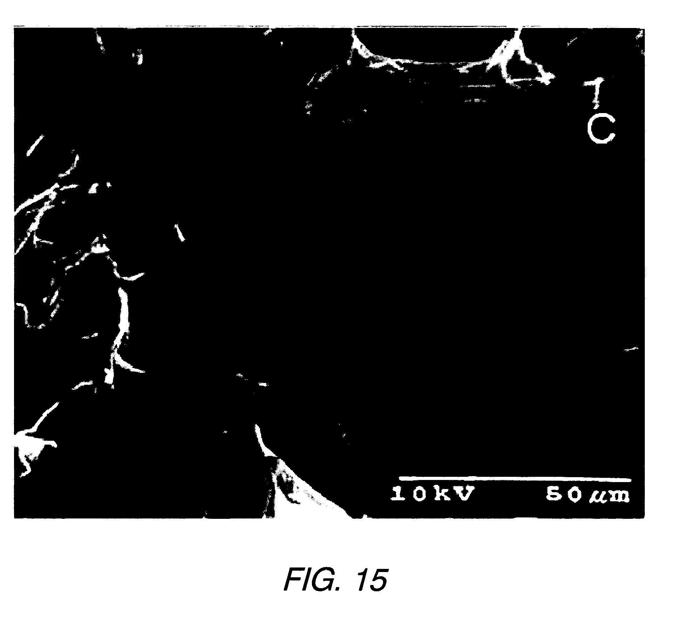

Various ammonium compounds are solids at room temperature and atmospheric pressure, and are know to undergo sublimation to the gaseous phase at reduced pressures. Moreover, these ammonium salts are known in the art to be biocompatible. Commercially available compunds were, thus, tested for their ability to sublimation. Based on these data, gelatin plastics were processed according to the method described in Example 16, and comprised ammonium acetate. The ammonium acetate was physically incorporated into the plastic matrix and formed into pellets as described in Example 16. The pellets were then exposed to vacuum to sublime the ammonium acetate, thereby leaving open pores in the plastic matrix. Pore formation was quantified as percent remaining mass over the course of the vacuum treatment, FIG. 14. Lyophilization of the ammonium salt particulates resulted in the creation of extensive networks of interconnected porosity in the gelatin plastic. FIG. 15 presents an SEM image of the resulting microporosity of the gelatin plastic post-sublimation. The interconnected pores are readily identifiable and extend throughout the entire plastic pellet. It was further discovered that ammonium acetate provided for the most rapid sublimation and most efficient formation of pores.

### Example 29: Degradation Testing of plastics

Plastic degradation testing was performed on non-cross-linked materials processed according to the method described in Example 16 and on identical plastics further processed by cross-linking with glutaraldehyde and genipin. The degradation testing was performed on gelatin, fibrin, and ECM-based plastic pellets in three solutions: PBS only (⌀), 0.6% genipin in PBS (OP), and 0.6% gluaraldehyde in PBS (GA). The solutions were prepared and transferred into three 15 ml tubes per solution for a total of nine tubes. The samples were arranged into sets of three, i.e. Gelatin {⌀, GP, GA}, Fibrin{ ⌀, GP, GA}, ECM{⌀, GP, GA}. The appropriate plastic pellet was placed into each dish and allowed to cross-link at 23°C with rocking for 21.5 hours. It was observed that all three of the genipin-containing samples turned blue after only 50 minutes. After 21.5 hours, the solutions were vacuum aspirated from the wells and the samples rinsed three times with PBS. Each plastic pellet was measured for initial area and initial mass. The plastic samples were then transferred to new wells, submerged in DMEM-F12 solution containg 0.1% Na azide, and 1% streptomycin/penicillin, sealed with laboratory film and incubated at 37°C and 5% CO₂.

The area of the plastic pellet was measured by arranging two rulers perpendicular to each other with the millimeter scales on the inside edges so that measurements were readily taken in mm by mm, and securing the rulers with tape. The scale was placed underneath a microscope lens so that the focus included as much of both axes as possible. The scale was fastened to the microscope stage. When measuring area, the pellet was left in solution as much as possible, preferably submerged in the well. This ensured that diffraction due to the solution was consistent for all measurements. The pellet was aligned so that two of its edges were flush with each axis of the rulers. If necessary, the pellet was moved uni-axially to measure a side, then the other edge was measured separately. During testing pipette tips were used as the tools for manipulating the pellets. The pellet and scale was viewed through the microscope objective and the size of the pellets read from the visual scales.

The mass of the plastic pellets was measured using a standard laboratory scale of appropriate precision (at least accurate to 0.02 mg). A pellet was taken out of solution and blotted twice to remove excess solution. The mass was measured on the scale and the chip immediately returned to the well. Fresh media solution was added to each well during the area and mass measurements while the pellet was out of the well.

Measurements were taken every 24 hours for up to 45 days and the pellets were incubated at 37°C and 5% CO₂ in the sealed multiwell dishes.

### Example 30: Plastic degradation testing with external cross-linking.

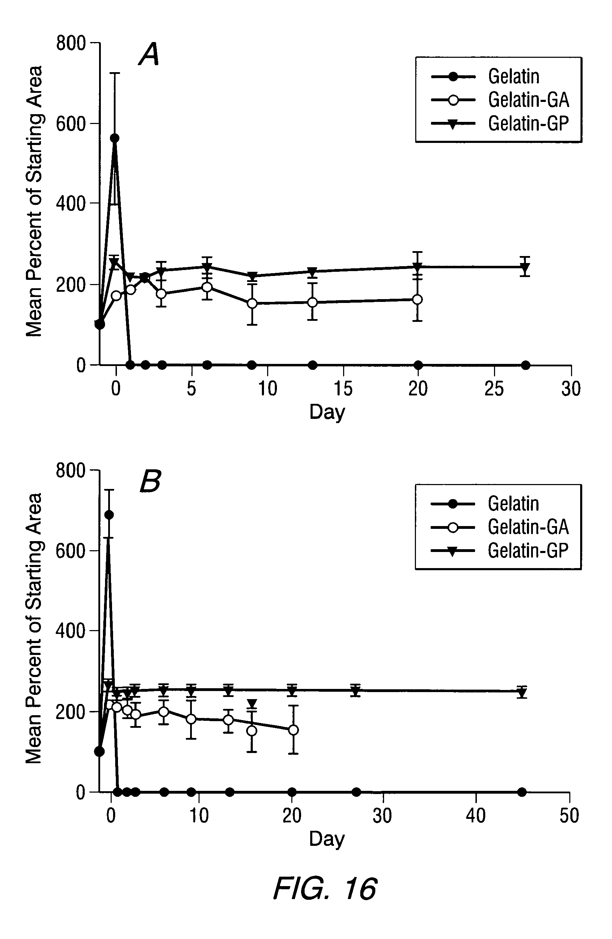

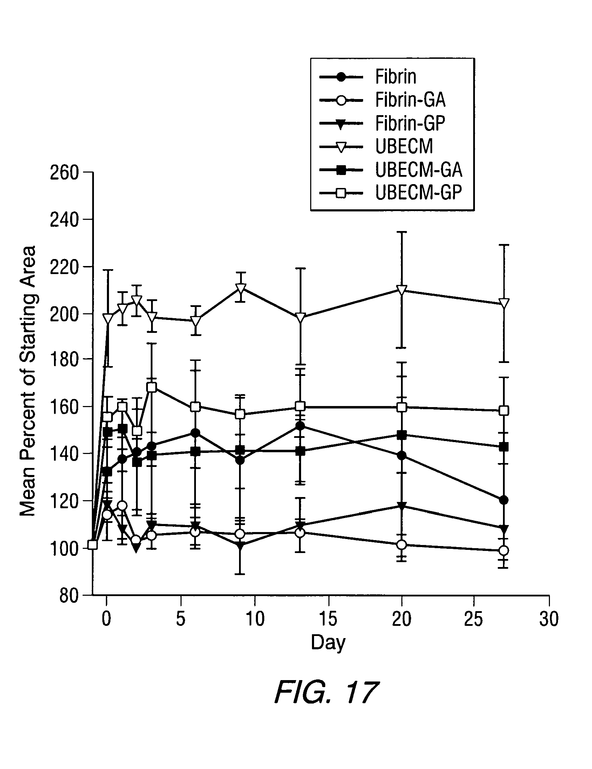

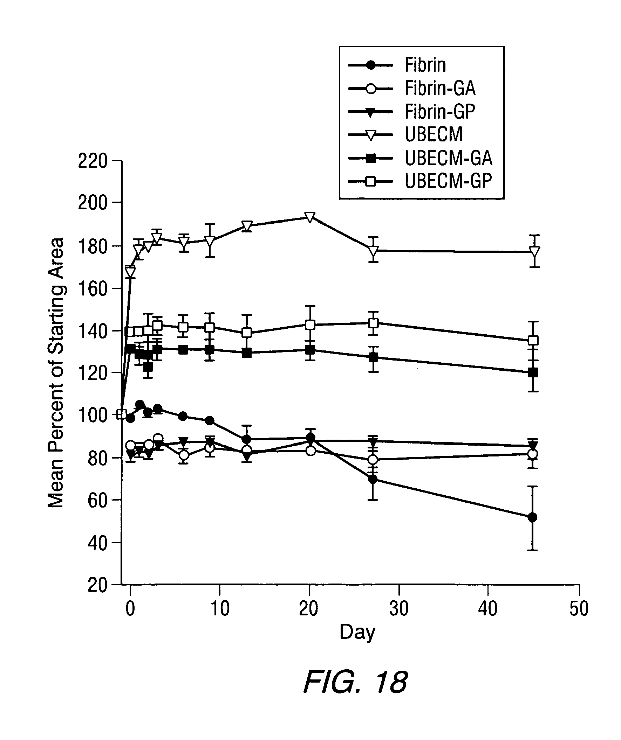

The plastic degradation testing method described in Example 28 was performed. FIG. 16, FIG. 17, and FIG. 18 illustrate these data graphically. Non-cross-linked gelatin plastic swelled approximately 6-fold as measured by both area (FIG. 16A) and mass (FIG. 16B), after which samples rapidly dissolved and were essential gone by 24 hrs post initiation of incubation. Cross-linked samples, either glutaraldehyde (GA) cross-linked or genipin (GP) cross-linked, swelled approximately 2-fold, and then maintained both area and mass through the 45 day test period. Gluteraldehyde and genipin have similar cross-linking chemistries, but genipin is a plant-based molecule reported to have much less biotoxicity.
Similar degradation experiments were also performed for both fibrin and urinary bladder ECM (UBECM) using both non-cross-linked and cross-linked samples. Cross-linking was performed as described above for gelatin-based plastic, Non-cross-linked fibrin swelled to approximately 140%, by either area (FIG. 17) or mass (FIG. 18) measures, whereas either GA or GP cross-linked fibrin did not exhibit significant swelling. An approximately 20% loss in mass was observed for cross-linked fibrin samples. The non-cross-linked fibrin samples did not degrade based on area but did lose approximately 50% mass by day 45. There was no significant change in either area or mass for cross-linked fibrin samples. Non-cross-linked UBECM swelled to approximately 200% by area determination (FIG. 17) and approximately 180 % by mass determination (FIG. 18). Swelling of cross-linked UBECM, either GA or GP, was reduced to approximately 150% and 130% by area and mass determinations, respectively. Once swelling occurred, there was no change in either area or mass through the 45 day period for either non-cross-linked or cross-linked UBECM. Non-cross-linked fibrin and UBECM are much more stable than non-cross-linked gelatin.
Inducing cross-linking during plastic formation controlled swelling while maintaining control of degradation. As observed in FIG. 16, significant swelling of gelatin occurred during cross-linking of pro-formed gelatin plastic. When a whole pellet of gelatin plastic treated with a cross-linker, swelling resulted which resulted in cracking of the pellet. Without being bound by theory, this was most likely due to the diffusion rate of cross-linking agent into the pellet. Because the cross-linking reaction occurs from outside of the pellet toward its center, diffusion of the cross-linking agent into the pellet becomes a limiting variable, resulting in a cross-linked "rigid shell" of gelatin on the surface of the pellet. Swelling continued inside the disk which eventually applied sufficient force to crack the pellet.

### Example 31: Plastic degradation testing with internal cross-linking.

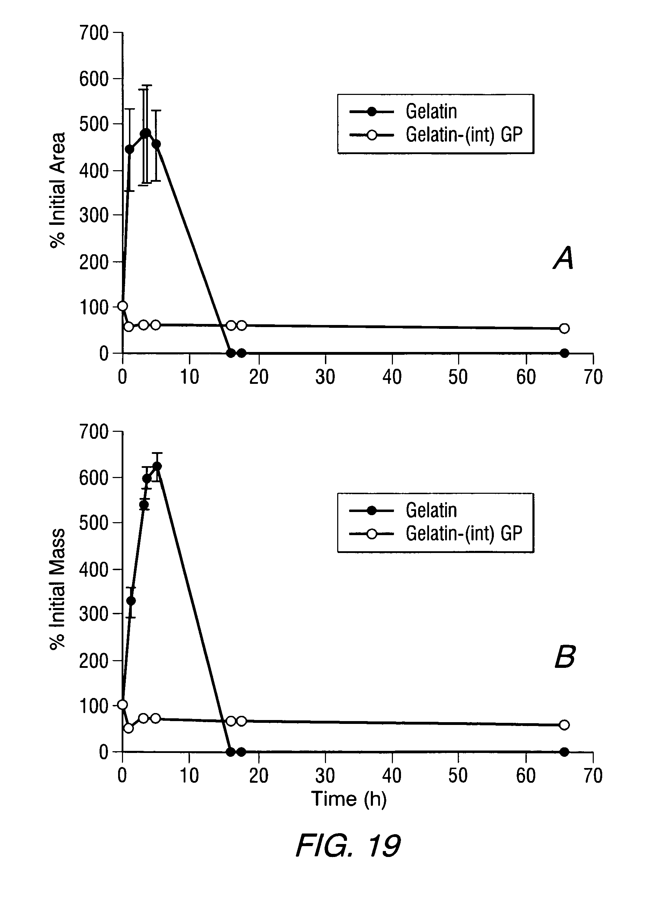

It was proposed that mixing GP with the protein powder prior to compression would result in a homogenous distribution of GP throughout the compressed plastic. Without being bound by theory, it was proposed that once placed in an aqueous solution, GP would initiate cross-linking as water diffused into the plastic rapidly. To evaluate this proposal, GP was mixed at 2% (weight:weight) with the milled gelatin powders prior to compression. GP-gelatin powders were mixed with 40% glycerin as plasticizer and pressed at 5000 lbs pressure. The trace water in the powders and/or glycerol, under the temperature and pressure conditions of compression, apparently resulted in cross-linking during compression. Samples were placed under in vitro serum degradation conditions as described in Example 29. Area and mass measurements were determined over time. The change is mass and area are shown in FIG. 19.

Non-oross-linked gelatin samples swelled similarly to experiments presented in Example 30. However, no swelling occurred with the internally cross-linked GP gelatin samples compared to the approximately 200% swelling for externally cross-linked gelatin samples. Similarly to the externally cross-linked GP gelatin samples, internally cross-linked GP gelatin did not degrade. This represents a significant advance permitting not only control of degradation but also shape retention using internally GP cross-linked proteins.

### Example 32: Plastic degradation testing with internal cross-linking.

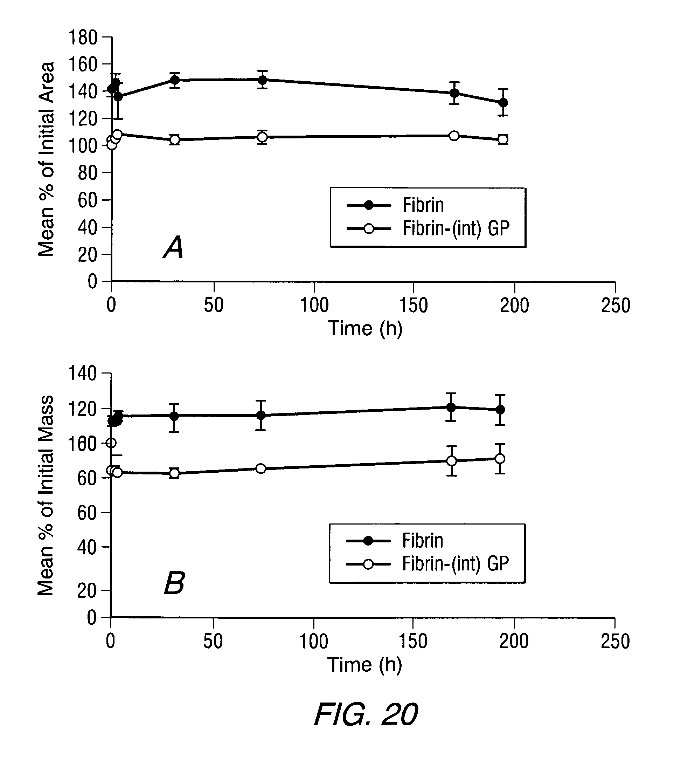

Internal GP cross-linking was applied to fibrin-bsaed plastics in the manner described in Example 31 for gelatin. FIG. 20 shows the change in mass and demonstrated that that internal GP cross-linked fibrin does not swell and does not degrade over time, making it particularly useful for biomedical applications such as for implants.

The aspects of the invention are:
1. An article of manufacture comprising a biopolymer, wherein the article is dehydrated.
2. The article of manufacture of aspect 1, wherein the biopolymer is selected from the group consisting of a protein, a fibrin, a fibrinogen, a collagen, a gelatin, an elastin, an extracellular matrix constituent, a polysaccharide, a hyaluronic acid, and combinations thereof.
3. The article of manufacture of aspect 1, wherein the article is dehydrated by means of a vacuum.
4. The article of manufacture of aspect 3, wherein the article forms a film.
5. The article of manufacture of aspect 4, wherein the article is elastic.
6. The article of manufacture of aspect 4, wherein the article is pliant.
7. The article of manufacture of aspect 3, wherein the article comprises disulfide bonds.
8. The article of manufacture of aspect 3, wherein the article comprises isopeptidic bonds.
9. The article of manufacture of aspect 3, wherein the article comprises monoaldehyde or polyaldehyde cross-linked amines.
10. The article of manufacture of aspect 3, wherein the article comprises pyran cross-linked amines.
11. The article of manufacture of aspect 3, wherein the article is cross-linked with a cross-linking agent selected from the group consisting of an iridoid derivative, genipin, a diimidate, a dione, an NHS-ester of dicarboxylic acid, a carbodiimide, an acrylamide, N,N'-methylenebisacrylamide, a sugar, ribose, Factor XIII, fructose, 1-ethyl-3-(dimethylaminopropyl) carbodiimide, 2,5-hexanedione, dimethylsuberimidate, an aldehyde, glutaraldehyde, formaldehyde, and combinations thereof.
12. The article of manufacture of aspect 3, wherein the article comprises a compound selected from the group consisting of a biological response modifier, an antigen, a drug, a hormone, a tracer, RNA, DNA, and a labeled compound.
13. The article of manufacture of aspect 12, wherein the tracer is a quantum dot.
14. The article of manufacture of aspect 12, wherein the biological response modifier is a bone morphogenetic protein.
15. The article of manufacture of aspect 4, wherein a heat-sensitive compound selected from the group consisting of a biological response modifier, an antigen, a drug, a hormone, a tracer, RNA, DNA, or a labeled compound is deposited on a surface of the film.
16. The article of manufacture of aspect 4, wherein a biological response modifier, an antigen, a drug, a hormone, a tracer, RNA, DNA, or a labeled compound is incorporated into the polymer matrix of the film.
17. The article of manufacture of aspect 4, wherein the article comprises a particulate.
18. The article of manufacture of aspect 17, wherein the particulate is selected from the group consisting of hydroxyapatite, tricalcium phosphate, calcium phosphate, and calcium sulfate.
19. The article of manufacture of aspect 3, wherein the article comprises a filler.
20. The article of manufacture of aspect 4, wherein the film forms a laminated structure.
21. The article of manufacture of aspect 20, wherein the laminated structure is formed from a stack of sheets, a tubular roll, or combination thereof.
22. An article of manufacture comprising a biopolymer, wherein the article is compressed.
23. The article of manufacture of aspect 22, wherein the biopolymer is selected from the group consisting of a protein, a fibrin, a fibrinogen, a collagen, a gelatin, an elastin, an extracellular matrix constituent, a polysaccharide, a hyaluronic acid, and combinations thereof.
24. The article of manufacture of aspect 22, wherein the article is compressed at a pressure and a temperature and for a time sufficient to form a polymer matrix.
25. The article of manufacture of aspect 24, wherein the article is formed in an extrusion die, a compression mold, or an injection mold.
26. The article of manufacture of aspect 22, wherein the article comprises a filler.
27. The article of manufacture of aspect 22, wherein the article comprises a plasticizer.
28. The article of manufacture of aspect 27, wherein the plasticizer is selected from the group consisting of a phthalate plasticizer, an adipate plasticizer, a trimellitate plasticizer, a maleate plasticizer, a sebacate plasticizer, a benzoate plasticizer, an epoxidized vegetable oil, a sulfonamide plasticizer, a phosphate plasticizer, water, a polyalcohol, a glycol, a glycerin, a glycerol, a polyether, an acetylated monoglyceride, an alkyl citrate, a polymeric plasticizer, and combinations thereof
29. The article of manufacture of aspect 22, wherein the article is cross-linked with a cross-linking agent selected from the group consisting of an iridoid derivative, genipin, a diimidate, a dione, an NHS-ester of dicarboxylic acid, a carbodiimide, an acrylamide, N,N'-methylenebisacrylamide, a sugar, ribose, Factor XIII, fructose, 1-ethyl-3-(dimethylaminopropyl) carbodiimide, 2,5-hexanedione, dimethylsuberimidate, an aldehyde, glutaraldehyde, formaldehyde, and combinations thereof.
30. The article of manufacture of aspect 22, wherein the article comprises pores.
31. The article of manufacture of aspect 22, wherein the article comprises a compound selected from the group consisting of a biological response modifier, an antigen, a drug, a hormone, a tracer, and a labeled compound.
32. A method of manufacturing polymer films comprising:
   providing a hydrogel; and
   vacuum drying the hydrogel at a temperature, and for a time, to form a dehydrated film.
33. The method of aspect 32, wherein the temperature is less than 80°C.
34. The method of aspect 32, wherein the pressure is less than 20 millibars.
35. The method of aspect 32, wherein the hydrogel is formed from a polymer selected from the group consisting of a protein, a fibrin, a fibrinogen, a collagen, a gelatin, an elastin, an extracellular matrix constituent, a polysaccharide, a hyaluronic acid, and combinations thereof.
36. The method of aspect 32, wherein the hydrogel comprises a plasticizer.
37. The method of aspect 36, wherein the plasticizer is selected from the group consisting of a phthalate plasticizer, an adipate plasticizer, a trimellitate plasticizer, a maleate plasticizer, a sebacate plasticizer, a benzoate plasticizer, an epoxidized vegetable oil, a sulfonamide plasticizer, a phosphate plasticizer, water, a polyalcohol, a glycol, a glycerin, a glycerol, a polyether, an acetylated monoglyceride, an alkyl citrate, a polymeric plasticizer, and combinations thereof.
38. The method of aspect 32, wherein the hydrogel is cross-linked with a cross-linking agent selected from the group consisting of an iridoid derivative, genipin, a diimidate, a dione, an NHS-ester of dicarboxylic acid, a carbodiimide, an acrylamide, N,N'-methylenebisacrylamide, a sugar, ribose, Factor XIII, fructose, 1-ethyl-3-(dimethylaminopropyl) carbodiimide, 2,5-hexanedione, dimethylsuberimidate, an aldehyde, glutaraldehyde, formaldehyde, and combinations thereof.
39. The method of aspect 32, wherein the hydrogel comprises a compound selected from the group consisting of biological response modifiers, an antigen, a drug, a hormone, a tracer, RNA, DNA, a labeled compound, and combinations thereof.
40. The method of aspect 32, wherein the hydrogel comprises a filler.
41. A method of manufacturing a plastic comprising:
   admixing a biopolymer with a compound to create an admixture; and
   compressing the admixture at a pressure and a temperature to form a biopolymer matrix.
42. The method of aspect 41, wherein the temperature is less than 80°C.
43. The method of aspect 41, wherein the pressure is less than 6000 pounds.
44. The method of aspect 41, wherein the admixture is formed in an extrusion die, a compression mold, or an injection mold.
45. The method of aspect 41, wherein the biopolymer matrix is formed from polymers selected from the group consisting of a protein, a polysaccharide, a fibrin, a fibrinogen, a gelatin, a hyaluronic acid, a collagen, an extracellular matrix constituent, an elastin, and combinations thereof.
46. The method of aspect 45, wherein the biopolymer is a powder.
47. The method of aspect 41, further comprising adding a filler to the admixture before compressing the admixture.
48. The method of aspect 41, wherein the biopolymer matrix comprises a plasticizer.
49. The method of aspect 47, wherein the plasticizer is selected from the group consisting of a phthalate plasticizer, an adipate plasticizer, a trimellitate plasticizer, a maleate plasticizer, a sebacate plasticizer, a benzoate plasticizer, an epoxidized vegetable oil, a sulfonamide plasticizer, a phosphate plasticizer, water, a polyalcohol, a glycol, a glycerin, a glycerol, a polyether, an acetylated monoglyceride, an alkyl citrate, a polymeric plasticizer, and combinations thereof.
50. The method of aspect 41, wherein the biopolymer matrix is cross-linked with a cross-linking agent selected from the group consisting of an iridoid derivative, genipin, a diimidate, a dione, an NHS-ester of dicarboxylic acid, a carbodiimide, an acrylamide, N,N'-methylenebisacrylamide, a sugar, ribose, Factor XIII, fructose, 1-ethyl-3-(dimethylaminopropyl) carbodiimide, 2,5-hexanedione, dimethylsuberimidate, an aldehyde, glutaraldehyde, formaldehyde, and combinations thereof.
51. The method of aspect 41, wherein the compound is selected from the group consisting of a biological response modifier, an antigen, a drug, a hormone, a tracer, RNA, DNA, a labeled compound, and combinations thereof.
52. A method of manufacturing a porous plastic comprising:
   admixing a biopolymer with a porogen;
   forming a biopolymer matrix comprising the porogen; and
   removing the porogen from the biopolymer matrix.
53. The method of aspect 52, wherein the biopolymer matrix is formed from polymers selected from the group consisting of a protein, a polysaccharide, a fibrin, a fibrinogen, a gelatin, a hyaluronic acid, a collagen, an extracellular matrix constituent, an elastin, and combinations thereof.
54. The method of aspect 52, wherein the biopolymer matrix comprises a filler.
55. The method of aspect 52, wherein the biopolymer matrix comprises a plasticizer.
56. The method of aspect 55, wherein the plasticizer is selected from the group consisting of a phthalate plasticizer, an adipate plasticizer, a trimellitate plasticizer, a maleate plasticizer, a sebacate plasticizer, a benzoate plasticizer, an epoxidized vegetable oil, a sulfonamide plasticizer, a phosphate plasticizer, water, a polyalcohol, a glycol, a glycerin, a glycerol, a polyether, an acetylated monoglyceride, an alkyl citrate, a polymeric plasticizer, and combinations thereof.
57. The method of aspect 52, wherein the biopolymer matrix is cross-linked with a cross-linking agent selected from the group consisting of an iridoid derivative, genipin, a diimidate, a dione, an NHS-ester of dicarboxylic acid, a carbodiimide, an acrylamide, N,N'-methylenebisacrylamide, a sugar, ribose, Factor XIII, fructose, 1-ethyl-3-(dimethylaminopropyl) carbodiimide, 2,5-hexanedione, dimethylsuberimidate, an aldehyde, glutaraldehyde, formaldehyde, and combinations thereof.
58. The method of aspect 52, wherein the biopolymer matrix comprises a compound selected from the group consisting of a biological response modifier, an antigen, a drug, a hormone, a tracer, RNA, DNA, a labeled compound, and combinations thereof.
59. The method of aspect 52, wherein the porogen is a solvation porogen.
60. The method of aspect 59, wherein the porogen is soluble in an organic phase.
61. The method of aspect 60, wherein the porogen is selected from the group consisting of polyurethane, polylactic acid, polyglycolic acid, polylactic-co-glycolic acid, and polycaprolactone.
62. The method of aspect 52, wherein the porogen is soluble in an aqueous phase.
63. The method of aspect 62, wherein the porogen is sodium chloride.
64. The method of aspect 52, wherein the porogen is a sublimation porogen.
65. The method of aspect 64, wherein the porogen is selected from the group consisting of ammonium acetate, ammonium chloride, ammonium bicarbonate, ammonium carbonate, and pyridinium trifluoroacetate.
66. A method of cross-linking a polymer comprising:
   providing a solid polymer powder admixed with a solid cross-linking agent capable of being activated by a solvent to form an admixture; and
   forming a polymer matrix from the admixture comprising the solid cross-linking agent.
67. The method of aspect 66, further comprising contacting the structure with a solvent which activates the cross-linking agent.
68. The method of aspect 66, wherein the cross-linking agent comprises a pyran moiety.
69. The method of aspect 66, wherein the cross-linking agent is an iridoid derivative.
70. The method of aspect 69, wherein the cross-linking agent is genipin.
71. The method of aspect 66, wherein the polymer is a biopolymer.
72. The method of aspect 71, wherein the biopolymer is selected from the group consisting of a protein, a fibrin, a fibrinogen, a collagen, a gelatin, an elastin, an extracellular matrix constituent, a polysaccharide, a hyaluronic acid, and combinations thereof.
73. The method of aspect 66, wherein the admixture comprises a plasticizer.
74. The method of aspect 73, wherein the plasticizer is selected from the group consisting of a phthalate plasticizer, an adipate plasticizer, a trimellitate plasticizer, a maleate plasticizer, a sebacate plasticizer, a benzoate plasticizer, an epoxidized vegetable oil, a sulfonamide plasticizer, a phosphate plasticizer, polyalcohol, glycol, glycerin, glycerol, polyether, acetylated monoglycerides, alkyl citrates, and a polymeric plasticizer.
75. The method of aspect 66, wherein admixture comprises a porogen.
76. The method of aspect 75, wherein the porogen is a solvation porogen.
77. The method of aspect 76, wherein the porogen is a sublimation porogen.
78. The method of aspect 66, wherein the admixture further comprises a compound selected from the group consisting of a biological response modifier, an antigen, a drug, a hormone, a tracer, RNA, DNA, a labeled compound, and combinations thereof.
79. The method of aspect 66, further wherein the admixture comprises a filler.
80. A method of cross-linking a polymer comprising:
   providing a solid polymer powder admixed with a solid cross-linking agent capable of being activated by a water, wherein the solid polymer contains amino groups;
   and
   incubating the admixture for a sufficient time for cross-linking to occur.

## Claims

1. A biocompatible article of manufacture prepared from an admixture comprising protein admixed with a plasticizer, wherein the admixture is compressed at a temperature of less than about 80°C and the article retains at least one bioactivity after compression.

2. The biocompatible article of manufacture of claim 1, wherein the protein is a powder.

3. The biocompatible article of manufacture of claim 1 or 2, wherein the plasticizer is selected from the group consisting of a phthalate plasticizer, an adipate plasticizer, a trimellitate plasticizer, a maleate plasticizer, a sebacate plasticizer, a benzoate plasticizer, an epoxidized vegetable oil, a sulfonamide plasticizer, a phosphate plasticizer, water, a polyalcohol, a glycol, a glycerin, a glycerol, a polyether, an acetylated monoglyceride, an alkyl citrate, a polymeric plasticizer, and combinations thereof.

4. The biocompatible article of manufacture of claim 1, wherein the article is prepared from an admixture comprising protein admixed with a plasticizer and a crosslinking agent.

5. The article of manufacture according to any of claims 1 to 4, wherein the protein is selected from the group consisting of fibrin, fibrinogen, albumin, immunoglobulins, fibronectin, vitronectin and mixtures thereof.

6. The article of manufacture according to any of claims 1 to 5, wherein the protein comprises fibrin.

7. The article of manufacture according to any of claims 1 to 6, wherein the plasticizer comprises glycerol.

8. The article of manufacture according to any of claims 1 to 7, wherein the article is cross-linked with a crosslinking agent selected from the group consisting of an iridoid derivative, genipin, a diimidate, a dione, an NHS-ester of dicarboxylic acid, a carbodiimide, an acrylamide, N,N'-methylenebisacrylamide, sugar, ribose, fructose, Factor XIII, 1-ethyl-3-(dimethylaminopropyl) carbodiimide, 2,5-hexanedione, dimethylsuberimidate, glutaraldehyde, formaldehyde, formaldehyde sodium bisulfite, and combinations thereof.

9. The article of manufacture according to any of claims 1 to 8, wherein the article is a wound dressing, implant, tissue replacement or tissue repair article.

10. The article of manufacture according to any of claims 1 to 9, wherein the article is in the form of a film, multiple layers of film, sheet, tube, rod, filament, scaffold, block, cube, capsule or tablet.

11. An article of manufacture according to any of claims 1 to 10, wherein the article is a laminated structure.

12. The article of manufacture according to any of claims 4 to 11, wherein the crosslinking agent comprises genipin.

13. The article of manufacture according to any of claims 1 to 12, wherein the article further comprises a compound selected from the group consisting of a biological response modifier, an antigen, a drug, a hormone, a tracer, RNA and DNA.

14. The article of manufacture according to any of claims 1 to 13, wherein the article further comprises particulates selected from the group consisting of polymers, ceramics, minerals, metal salts, apatites and mixtures thereof.

15. The article of manufacture according to any of claims 1 to 14, wherein the article comprises pores.

16. A method of manufacturing a plastic article of manufacture according to any of claims 1 to 15, comprising:
admixing protein with a plasticizer to create an admixture; and
compressing the admixture at a pressure and a time to form a matrix.

17. A method of manufacturing a porous plastic article of manufacture according to claim 15, comprising:
admixing protein with plasticizer and a solvation porogen or a sublimation porogen to form an admixture;
forming a matrix comprising the porogen; and
removing the porogen from the matrix.

18. The method according to claim 17, wherein the porogen is a solvation porogen selected from the group consisting of polyurethane, polylactic acid, polyglycolic acid, polylactic-co-glycolic acid, and polycaprolactone.

19. The method according to claim 17, wherein the porogen is a sublimation porogen selected from the group consisting of ammonium acetate, ammonium chloride, ammonium bicarbonate, ammonium carbonate, and pyridinium trifluoroacetate.

20. A method of cross-linking a polymer article according to any of claims 4 to 15, comprising:
providing a solid protein admixed with plasticizer and a solid cross-linking agent capable of being activated by a solvent to form an admixture; and
forming a polymer matrix from the admixture comprising the solid crosslinking agent.
